# EUROPEAN PATENT SPECIFICATION

(11) **EP 2 929 352 B1**
(45) Date of publication and mention of the grant of the patent: **21.11.2018**
(21) Application number: 13861116.5
(22) Date of filing: 06.12.2013
(51) Int. Cl.: G01N 33/574, C12Q 1/68, G01N 33/50, G01N 33/92

(54) **METABOLOMIC PROFILING DEFINES ONCOGENES DRIVING PROSTATE TUMORS**
METABOLOMISCHE PROFILIERUNG ZUR DEFINITION VON PROSTATATUMOR-FÖRDERNDEN ONKOGENEN
LE PROFILAGE MÉTABOLOMIQUE DÉFINIT DES ONCOGÈNES ENTRAÎNANT DES TUMEURS DE LA PROSTATE

(30) Priority: 06.12.2012 US 201261734040 P; 13.03.2013 US 201361779446 P
(43) Date of publication of application: 14.10.2015
(60) Divisional application: 18199278.5
(73) Proprietor: Dana-Farber Cancer Institute, Inc., Boston, MA 02215 (US)
(72) Inventor: LODA, Massimo, Belmont, MA 02478 (US); PRIOLO, Carmen, Brookline, MA 02446 (US); PYNE, Saumyadipta, Gachibowli Hyderabad-500046 (IN)
(74) Representative: Gill Jennings & Every LLP
(86) International application number: PCT/US2013/073569
(87) International publication number: WO 2014/089431

(56) References cited:
- WO-A1-2011/084108
- WO-A2-2012/064967
- US-A1- 2006 088 894
- US-A1- 2009 047 269
- US-A1- 2012 021 983
- NICOLA J. CLEGG ET AL: "MYC Cooperates with AKT in Prostate Tumorigenesis and Alters Sensitivity to mTOR Inhibitors", PLOS ONE, vol. 6, no. 3, E17449, 4 March 2011 (2011-03-04), pages 1-14, XP055256565, DOI: 10.1371/journal.pone.0017449
- BRUCE J TROCK: "Application of metabolomics to prostate cancer", UROLOGIC ONCOLOGY, vol. 29, no. 5, 2011, pages 572-581, XP028294288, ISSN: 1078-1439, DOI: 10.1016/J.UROLONC.2011.08.002 [retrieved on 2011-08-22]
- D. M. MILLER ET AL: "c-Myc and Cancer Metabolism", CLINICAL CANCER RESEARCH, vol. 18, no. 20, 15 October 2012 (2012-10-15), pages 5546-5553, XP055269633, US ISSN: 1078-0432, DOI: 10.1158/1078-0432.CCR-12-0977
- R. L. ELSTROM ET AL: "Akt Stimulates Aerobic Glycolysis in Cancer Cells", CANCER RESEARCH, vol. 64, no. 11, 1 June 2004 (2004-06-01), pages 3892-3899, XP055269638, US ISSN: 0008-5472, DOI: 10.1158/0008-5472.CAN-03-2904
- SREEKUMAR ARUN ET AL: "Metabolomic profiles delineate potential role for sarcosine in prostate cancer progression (includes Methods)", NATURE, NATURE PUBLISHING GROUP, UNITED KINGDOM, vol. 457, no. 7231, 12 February 2009 (2009-02-12), pages 910-914+1PP, XP002590536, ISSN: 0028-0836, DOI: 10.1038/NATURE07762
- C. PRIOLO ET AL: "AKT1 and MYC Induce Distinctive Metabolic Fingerprints in Human Prostate Cancer", CANCER RESEARCH, vol. 74, no. 24, 16 October 2014 (2014-10-16), pages 7198-7204, XP055269641, US ISSN: 0008-5472, DOI: 10.1158/0008-5472.CAN-14-1490
- CLEGG ET AL.: 'MYC cooperates with AKT in prostate tumorigenesis and alters sensitivity to mTOR inhibitors.' PLOS ONE vol. 6, no. 3 E174, 04 March 2011, pages 1 - 14, XP055256565
- HU ET AL.: 'Proteomics revisits the cancer metabolome.' EXPERT REV PROTEOMICS vol. 8, no. 4, August 2011, pages 505 - 533, XP008179353
- FLAVIN ET AL.: 'Metabolic alterations and targeted therapies in prostate cancer.' J PATHOL vol. 223, no. 2, January 2011, pages 283 - 294, XP055256569

## Description

### BACKGROUND OF THE INVENTION

Prostate cancer is the most common cause of death from cancer in men over age 75. Many factors, including genetics and diet, have been implicated in the development of prostate cancer. Proliferation in normal cells occurs when nutrients are taken up from the environment as a result of stimulation by growth factors. Cancer cells overcome this growth factor dependence either by acquiring genetic mutations that result in altered metabolic pathways or by affecting metabolic pathways *de novo* with targeted mutations in critical metabolic enzymes. Altered metabolic pathways, in turn, stimulate cell growth by either providing fuel for energy or by efficiently incorporating nutrients into biomass.

Metabolic alterations may occur as a result of altered pathways, in turn a consequence of genetic events. Alternatively, metabolic alterations may be primary events in cancer but require genetic alterations in critical pathways for oncogenesis. A fundamental unanswered question is whether all oncogenic drivers (such as Myc or Akt) harness a similar metabolic response or whether each oncogenic event results in its own specific metabolic program. This is important because if the latter is true, targeting selected metabolic enzymes/pathways together with the putative driving oncogenes could become a powerful and targeted approach in cancer therapeutics.

Clegg et al (2011) PLoS ONE 6(3): e17449 observed statistically significant co-occurrence of *MYC* amplification and PI3K-pathway alteration in human prostate tumours, suggesting these two lesions cooperate in prostate cancer progression.

### SUMMARY OF THE INVENTION

It has been discovered, surprisingly, that metabolic profiles are specific to oncogenes driving human tumors, specifically prostate tumor.

Accordingly, in a first aspect, the invention provides a method to identify Aktl and Myc status in a prostate tumor comprising: analyzing, with at least one computer processor, a profile of at least 5 metabolites in a prostate tumor sample obtained from a subject to assign an Aktl and Myc status to the sample, wherein: the at least 5 metabolites are differentially produced in prostate tumors with high Aktl expression versus prostate tumors with high Myc expression, wherein the metabolites are selected from Table 1; wherein the expression profile of metabolites is compared to an appropriate reference profile of the metabolites, wherein the appropriate reference profile of the metabolites comprises profiles of the metabolites in prostate tumor with high Aktl expression, in prostate tumor with low Akt1 expression, in prostate tumor with high Myc expression, and in prostate tumor with low Myc expression; and wherein the processor assigns a status of high Akt1/high Myc, high Akt1/low Myc, low Aktl /high Myc, or low Akt1/low Myc to the sample.

In some embodiments, the method further comprises performing an assay to measure the profile of the at least 5 metabolites in the prostate tumor sample obtained from the subject. In some embodiments, the metabolic profile comprises at least 10, at least 25, at least 50, at least 75, at least 100, at least 125, at least 150, at least 175, at least 200, at least 225, at least 250, at least 275, at least 300, at least 350, at least 375, at least 400 metabolites, at least 450 metabolites, at least 500 metabolites, at least 1000 metabolites, or at least 1500 metabolites. In some embodiments, the metabolic profile of the tumor sample is measured using one or more of mass spectroscopy, nuclear magnetic resonance or chromatography. In some embodiments, the metabolites are selected from Table 1. In some embodiments, the profile of metabolites of the tumor sample is compared using cluster analysis. In some embodiments, the cluster analysis is selected from the group consisting of: hierarchical clustering, k-mean clustering, distribution-based clustering, and density-based clustering. In some embodiments, the differentially produced metabolites are selected using a threshold of p value < 0.05. In some embodiments, the methods described herein further comprise determining a confidence value for the Akt1 and Myc status assigned to the sample and providing an indication of the confidence value and the Akt1 and Myc status assigned to the sample to a user. In some embodiments the method further comprises determining whether the confidence value is below a threshold value and providing an indication that the confidence value is below the threshold value.

According to a second aspect of the invention, a computer-readable storage medium is provided. The storage medium is encoded with a plurality of instructions that, when executed by at least one processor, performs a method according to the first aspect and related embodiments.

In some embodiments of the second aspect, the method further comprises determining a confidence value for the Akt1 and Myc status assigned to the sample, and providing an indication of the confidence value and the Akt1 and Myc status assigned to the sample to a user.

In some embodiments of the second aspect, the method further comprises determining whether the confidence value is below a threshold value, and providing an indication that the confidence value is below the threshold value.

### BRIEF DESCRIPTION OF THE DRAWINGS

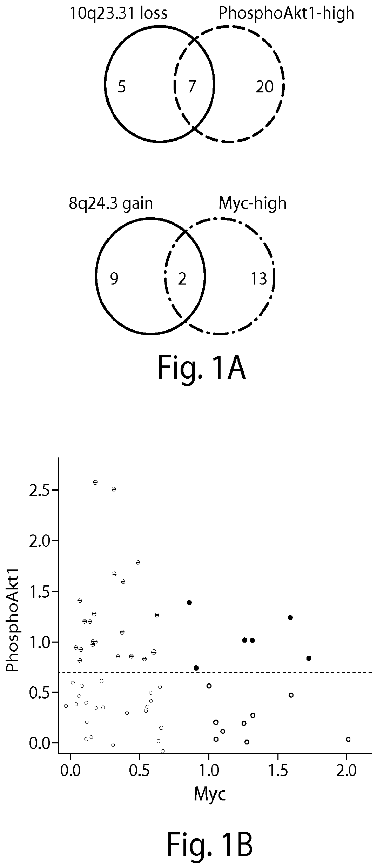

FIG. 1. Classification of prostate tumors by genomics and protein expression levels. The Venn diagram in (A) shows the number of tumors characterized by both copy number change at the PTEN or MYC locus and high phosphoAKT1 or MYC expression levels, and the number of those with either one alteration. Twelve and eleven tumors harbor 10q23.31 (PTEN locus) loss and 8q24.3 (MYC locus) gain, respectively, representing only 26% (7/27) of phosphoAKT1-high and 13% (2/15) of MYC-high tumors. K-means clustering was used to segregate 4 prostate tumor subgroups, i.e. phosphoAKT1-high/MYC-high (black dots), phosphoAKT1-high/MYC-low (red dots), phosphoAKT1-low/MYC-high (green dots) and phosphoAKT1-low/MYC-low (grey dots) (B).

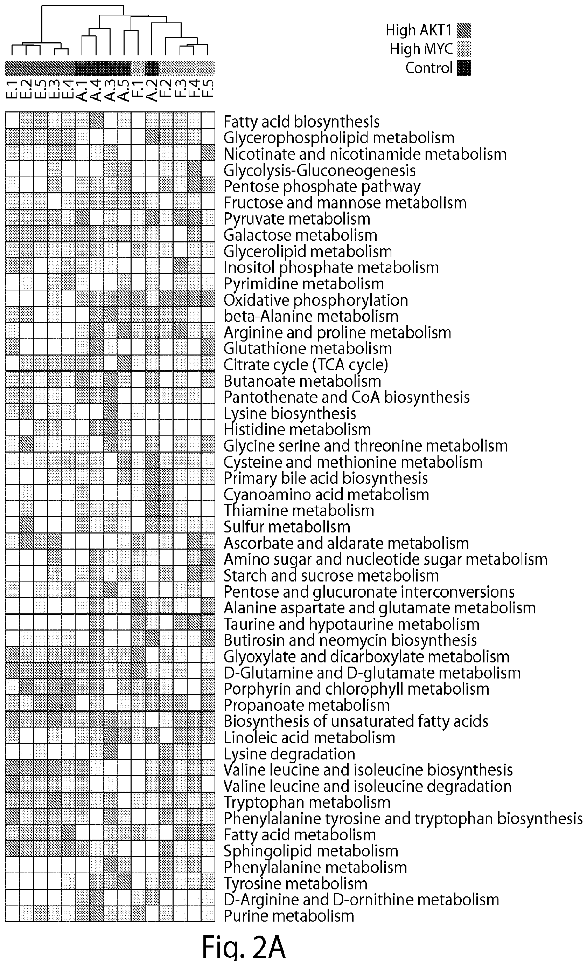

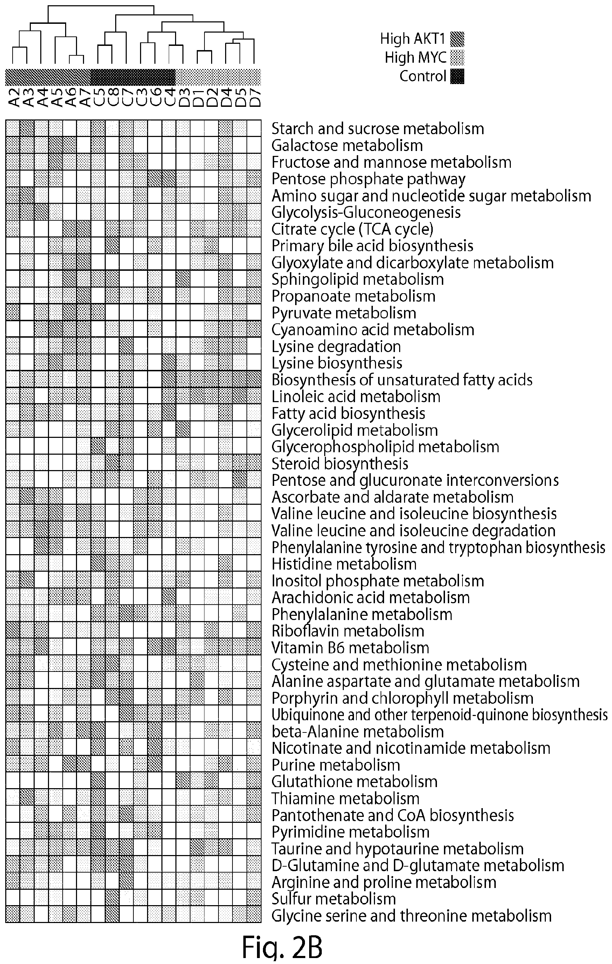

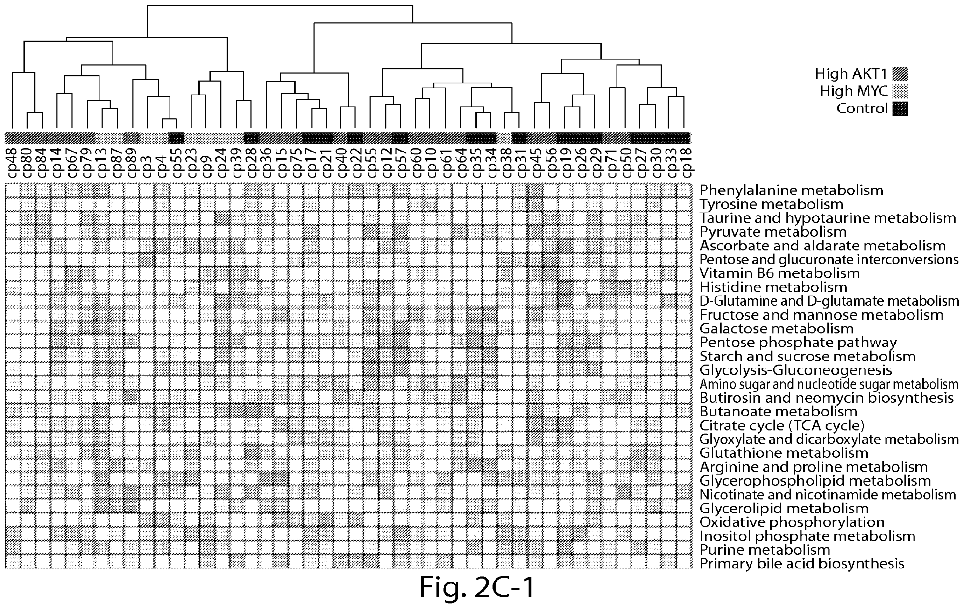

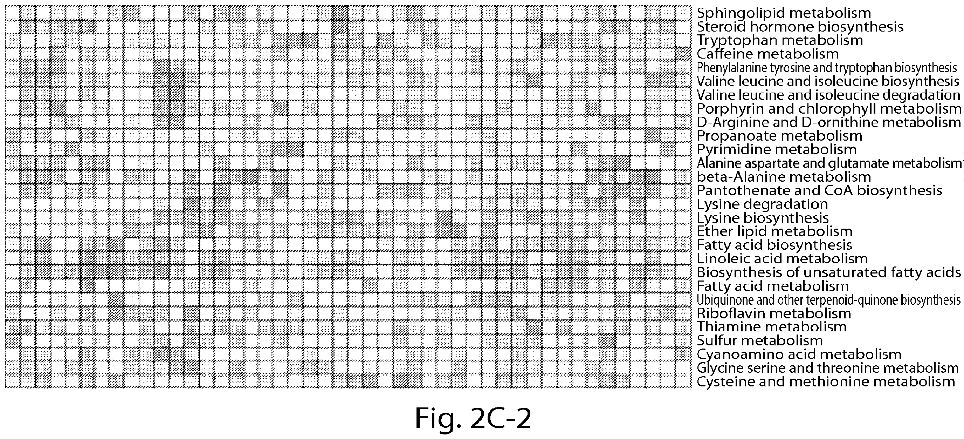

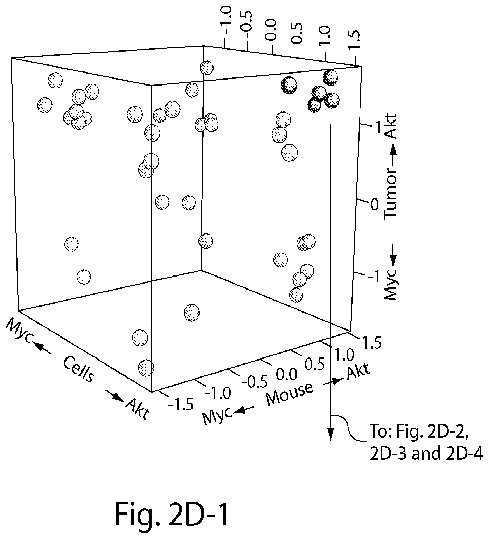

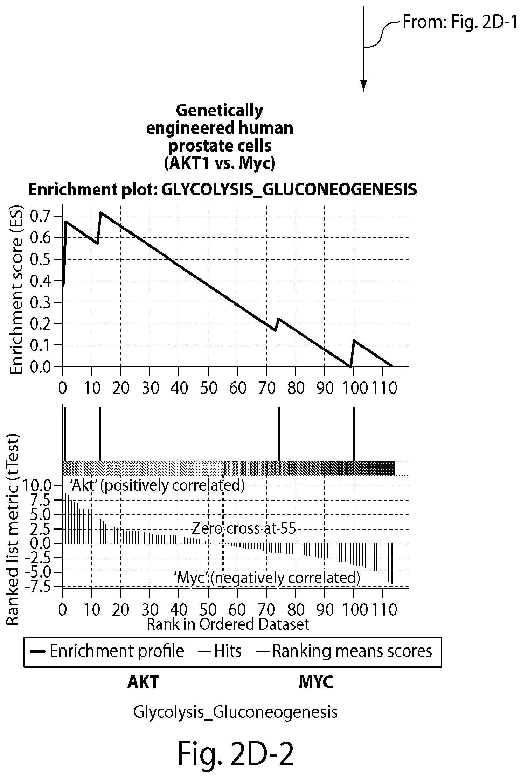

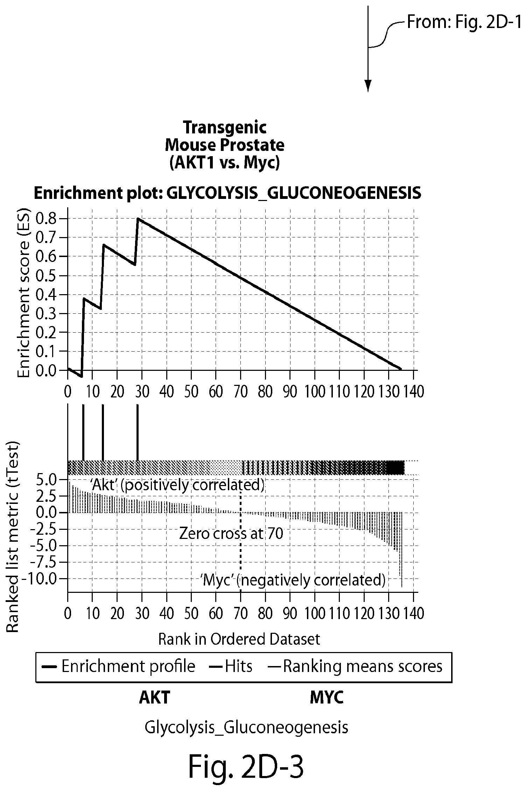

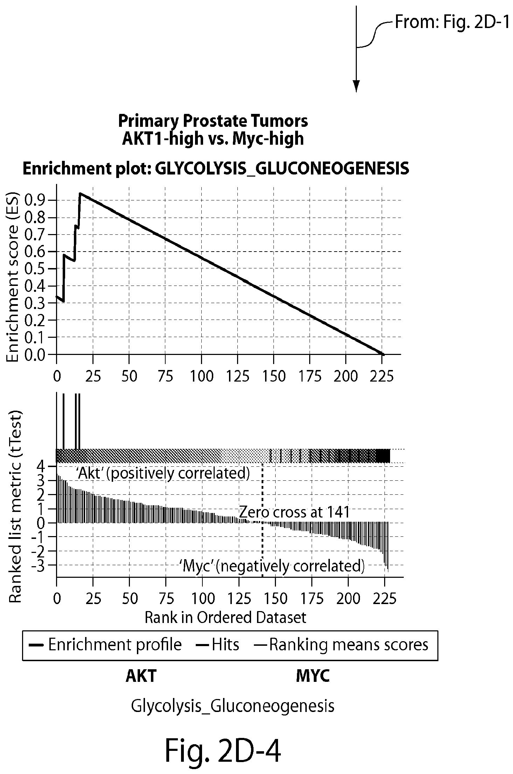

FIG 2. Enrichment of metabolic pathways across classes and systems. In heatmaps (A) through (C) the normalized enrichment scores of the most significantly enriched pathways within each of the 3 systems - cells, mice and human tumors are shown. Each row represents a KEGG pathway and each column an individual sample. Brown/green colors are used to denote high/low enrichment. Hierarchical clustering is used for unsupervised identification of the higher-level enrichment classes, which are well preserved across all 3 systems. The phenotypic labels of the samples are indicated as by a colored band on top of the heatmap, while the dendrogram represents the distances among them. In plot (D), we summarize the overall differential enrichments across the two classes of samples, Akt versus Myc, with simultaneous metabolic set enrichment analysis (akin to gene set enrichment analysis) measurements in all 3 systems. This information is depicted as points in 3-dimensional space, where each point represents a particular pathway, and each dimension a system. Enrichment of a pathway in Akt versus Myc overexpressed classes are given by positive and negative scores respectively. The top 5 positively enriched pathways (i.e. in high Akt samples) in all 3 systems, and the top 2 negatively enriched pathways (i.e. in high Myc samples) in all 3 systems, as chosen with an enrichment p-value threshold of 0.05, are highlighted as red and green points respectively.

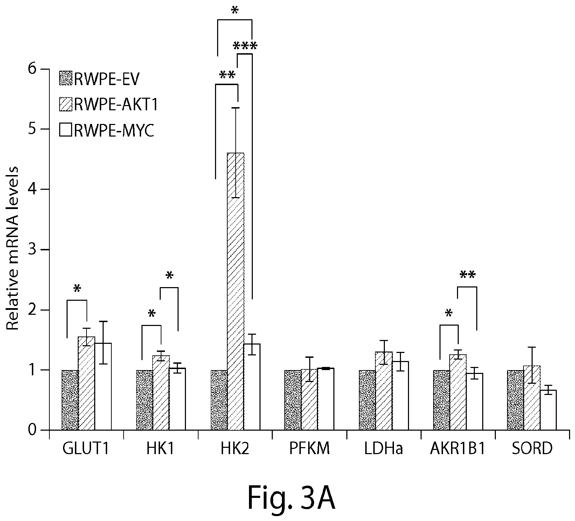

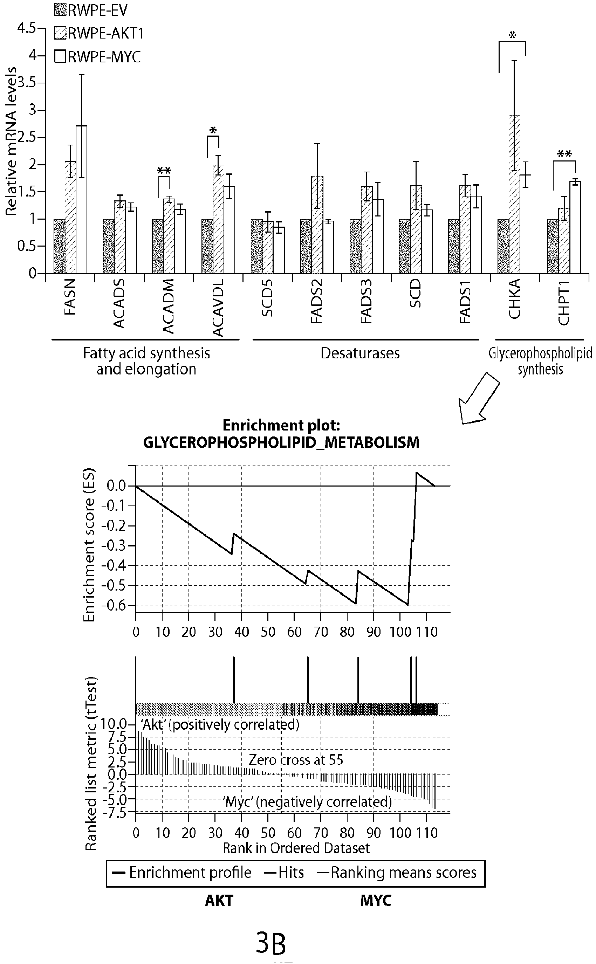

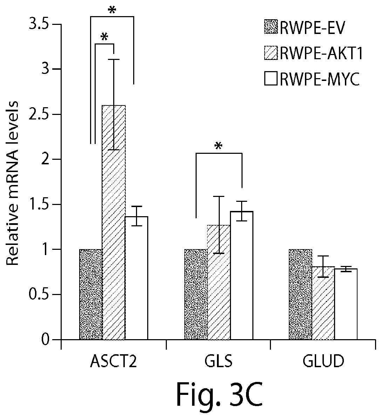

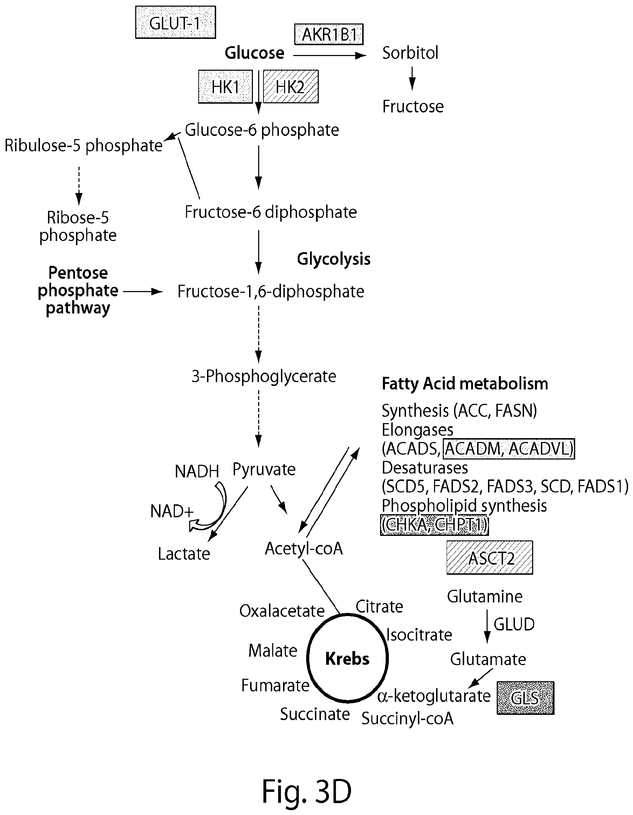

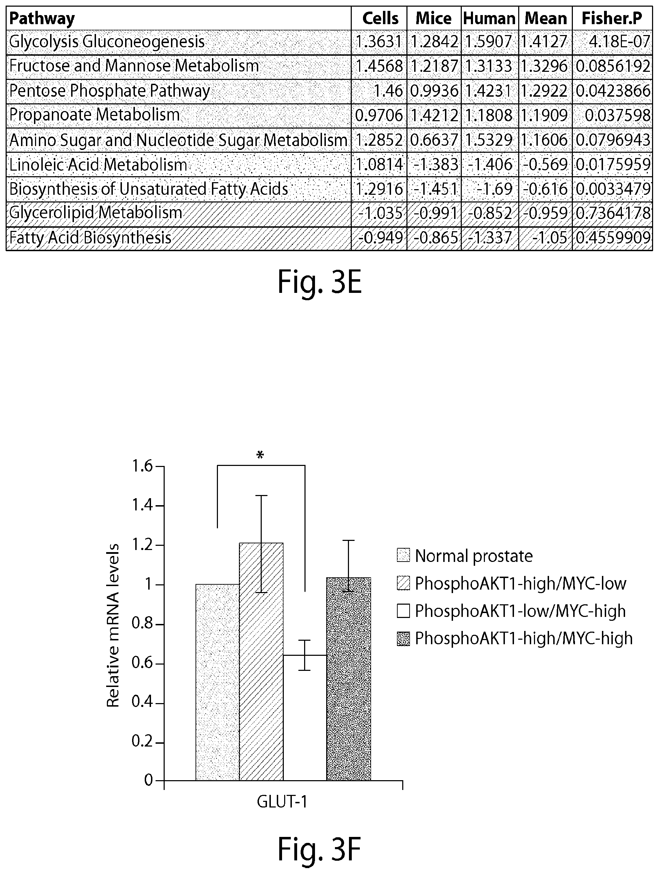

Fig. 3. Relative mRNA expression of metabolic genes in RWPE-1 engineered cells. (A) Glucose metabolism; (B) Lipid metabolism; (C) Glutamine metabolism. (D) Diagram showing metabolic enzymes up-regulated in RWPE-AKT (red), RWPE-MYC (green) cells relative to control (blue) or to each other. (E) For each pathway, its normalized enrichment scores in each system and their average are shown. The top 5 most enriched pathways in the high-Akt samples across all 3 systems are shown in red. The top 5 most enriched pathways in the high-Myc samples across all 3 systems are shown in green. Also shown in light green that some pathways which have high enrichments in Akt-high both mice and human tumors have low enrichments in cells. (F) Relative mRNA levels of GLUT-1 in human prostate tumors.

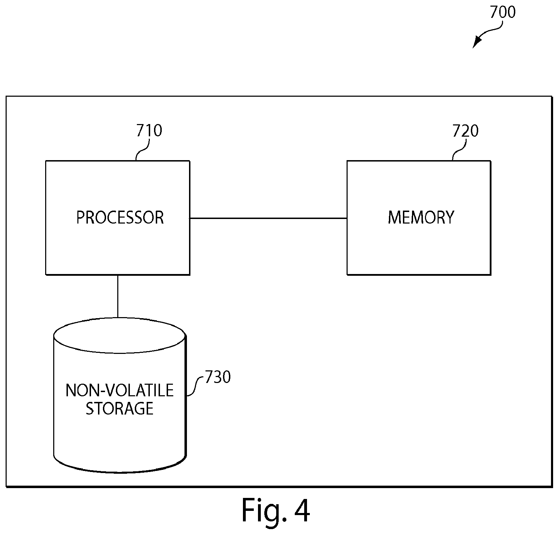

FIG. 4 is an illustrative implementation of a computer system.

### DETAILED DESCRIPTION OF THE INVENTION

A fundamental unanswered question in cancer biology has been whether metabolic changes are similar in cancers driven by different oncogenes or whether each genetic alteration induces a specific metabolic profile. This invention is based, at least in part, on the surprising discovery that metabolic profiles are specific to oncogenes driving human tumors, specifically prostate cancer. Thus, prostate tumors exhibit metabolic fingerprints of their molecular phenotypes, which impacts metabolic diagnostics and targeted therapeutics. Accordingly, aspects of the invention relate to methods aim at indirectly identifying Akt1 and Myc -driven tumors. The metabolic profiles of the tumors are compared to appropriate reference metabolic profiles to determine if the tumor is "driven" by either Akt1 or Myc oncogenes. This methodology can also be applied to other oncogenes (or tumor suppressor genes), combination of these and to any other type of cancer.

According to some aspects of the invention, a method to identify Akt1 and Myc status in a prostate tumor is provided. The method comprises analyzing, with at least one computer processor, a profile of at least 5 metabolites in a prostate tumor sample obtained from a subject to assign an Aktl and Myc status to the sample, wherein: the at least 5 metabolites are differentially produced in prostate tumors with high Akt1 expression versus prostate tumors with high Myc expression, wherein the metabolites are selected from Table 1; wherein the expression profile of metabolites is compared to an appropriate reference profile of the metabolites, wherein the appropriate reference profile of the metabolites comprises profiles of the metabolites in prostate tumor with high Akt1 expression, in prostate tumor with low Akt1 expression, in prostate tumor with high Myc expression, and in prostate tumor with low Myc expression; and wherein the processor assigns a status of high Akt1/high Myc, high Akt1/low Myc, low Aktl /high Myc, or low Akt1/low Myc to the sample.

The *AKT1* (v-akt murine thymoma viral oncogene homolog 1, also called *AKT*) gene encodes a serine/threonine-protein kinase that is involved in cellular survival pathways, by inhibiting apoptotic processes. Akt1 is also able to induce protein synthesis pathways, and is therefore a key signaling protein in the cellular pathways that lead to skeletal muscle hypertrophy, and general tissue growth. Since it can block apoptosis, and thereby promote cell survival, Akt1 has been implicated as a major factor in many types of cancer. Akt1 was originally identified as the oncogene in the transforming retrovirus, AKT8 (Staal SP et al. (July 1977) "Isolation of transforming murine leukemia viruses from mice with a high incidence of spontaneous lymphoma". Proc. Natl. Acad. Sci. U.S.A. 74 (7): 3065-7).

Akt possesses a protein domain known as Pleckstrin Homology (PH) domain, which binds either PIP3 (phosphatidylinositol (3,4,5)-trisphosphate, PtdIns(3,4,5)P3) or PIP2 (phosphatidylinositol (3,4)-bisphosphate, PtdIns(3,4)P2). PI 3-kinases (phosphoinositide 3-kinase or PI3-K) are activated on receipt of chemical messengers which tell the cell to begin the growth process. For example, PI 3-kinases may be activated by a G protein coupled receptor or receptor tyrosine kinase such as the insulin receptor. Once activated, PI 3-kinase phosphorylates PIP2 to form PIP3. PI3K-generated PIP3 and PIP2 recruit Akt1 to the plasma membrane where it becomes phosphorylated by its activating kinases, such as, phosphoinositide dependent kinase 1 (PDK1). This phosphorylation leads to activation of Akt1.

As used herein "Myc" refers to a family of genes and corresponding polypeptides. The Myc family encompasses Myc proteins having Myc transcriptional activity, including but not limited to, c-Myc (GenBank Accession No P01106), N-Myc (GenBank Accession No P04198), L-Myc (GenBank Accession No. CAA30249), S-Myc (GenBank Accession No. BAA37155) and B-Myc (GenBank Accession No. NP_075815).

Myc is a regulator gene that encodes a transcription factor. Myc proteins are most closely homologous at the MB1 and MB2 regions in the N-terminal region and at the basic helix-loop-helix leucine zipper (bHLHLZ) motif in the C-terminal region (Osier et al. (2002) Adv Cancer Res 84:81-154; Grandori et al. (2000) Annu Rev Cell Dev Biol 16:653-699). In the human genome, Myc is located on chromosome 8 and is believed to regulate expression of 15% of all genes through binding Enhancer Box sequences (E-boxes) and recruiting histone acetyltransferases (HATs). By modifying the expression of its target genes, Myc activation results in numerous biological effects. The first to be discovered was its capability to drive cell proliferation (upregulates cyclins, downregulates p21), but it also plays a very important role in regulating cell growth (upregulates ribosomal RNA and proteins), apoptosis (downregulates Bcl-2), differentiation and stem cell self-renewal. Myc is a very strong proto-oncogene and it is very often found to be upregulated in many types of cancers.

Between 30 and 70% of prostate tumors have genomic loss of phosphatase and tensin homolog (PTEN), leading to constitutively active phosphatidylinositol 3-kinase/protein Kinase B (PI3K/AKT) pathway, while 8q amplification including the MYC gene occurs in ∼30% of prostate tumors. Thus, these are recognized as the most frequent genetic alterations in prostate tumors. Both activated Akt and especially Myc overexpression faithfully reproduce the stages of human prostate carcinogenesis in genetically engineered mice (GEMMs). Recent literature shows that MYC promotes glutaminolysis, whereas AKT activation is associated with enhanced aerobic glycolysis and/or increased expression of glycolytic enzymes in different cell types, including prostate. However, the impact of these oncogenes or the genomic alterations causing their activation on the metabolome of human prostate tumors had not been fully elucidated.

"Assign an Akt1 status" means identifying, with at least one processor, the sample as having a metabolite profile that is similar to or characteristic of a prostate tumor with high Akt1 expression or with low Akt1 expression. "Assign a Myc status" means identifying, with at least one processor, the sample as having a metabolite profile that is similar to or characteristic of a prostate tumor with high Myc expression or with low Myc expression. The sample is assigned by the processor a metabolic status of high Akt1/high Myc, high Akt1/low Myc, low Akt 1/high Myc, or low Akt1/low Myc.

As used herein, a "high Akt1" or a "high Myc" metabolic status indicates that the expression level of Akt1 or Myc in the sample is similar to or characteristic of prostate tumors having constitutively activated (phosphorylated) Ak1 or prostate tumors overexpressing Myc. In some embodiments, a "high Akt1" or a "high Myc" status indicates that the expression level of Akt1 or Myc in the sample is similar to or characteristic of prostate cells having constitutively activated (phosphorylated) Akt1 or overexpressing Myc. In some embodiments, a "high Akt1" status indicates that the expression level of Akt1 in the sample is at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 100-fold, or more higher than that in prostate tumors or prostate cells in which Akt1 is not constitutively activated. In some embodiments, a "high Myc" status indicates that the expression level of Myc in the sample is at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 100-fold, or more higher than that in prostate tumors or prostate cells in which Myc is not overexpressed.

Conversely, a "low Akt1" status indicates that the expression level of Akt1 in the sample is similar to or characteristic of prostate tumors or prostate cells in which Akt1 is not constitutively activated. A "low Myc" status indicates that the expression level of Myc in the sample is similar to or characteristic of prostate tumors or prostate cells in which Myc is not overexpressed. In some embodiments, a "low Akt1" or a "low Myc" status indicates that the expression level of Akt1 or Myc in the sample is at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 100-fold, or more lower than that in prostate tumors or prostate cells in which Akt1 is not constitutively activated or Myc is not overexpressed.

As used herein, "metabolites" are small molecule compounds, such as substrates for enzymes of metabolic pathways, intermediates of such pathways or the products produced by a metabolic pathway. Metabolic pathways are well known in the art, and include, for example, citric acid cycle, respiratory chain, glycolysis, gluconeogenesis, hexose monophosphate pathway, oxidative pentose phosphate pathway, production and β-oxidation of fatty acids, urea cycle, amino acid biosynthesis pathways, protein degradation pathways, amino acid degrading pathways, and biosynthesis or degradation of lipids, proteins, and nucleic acids. Accordingly, small molecule compound metabolites may be composed of the following classes of compounds: alcohols, alkanes, alkenes, alkines, aromatic compounds, ketones, aldehydes, carboxylic acids, esters, amines, imines, amides, cyanides, amino acids, peptides, thiols, thioesters, phosphate esters, sulfate esters, thioethers, sulfoxides, ethers, or combinations or derivatives of the aforementioned compounds.

Preferably, a metabolite has a molecular weight of 50 Da (Dalton) to 30,000 Da, most preferably less than 30,000 Da, less than 20,000 Da, less than 15,000 Da, less than 10,000 Da, less than 8,000 Da, less than 7,000 Da, less than 6,000 Da, less than 5,000 Da, less than 4,000 Da, less than 3,000 Da, less than 2,000 Da, less than 1,000 Da, less than 500 Da, less than 300 Da, less than 200 Da, less than 100 Da. Preferably, a metabolite has, however, a molecular weight of at least 50 Da. Most preferably, a metabolite in accordance with the present invention has a molecular weight of 50 Da up to 1,500 Da.

In some embodiments, at least some of the metabolites used in the methods described herein are differentially produced in prostate tumors with high Akt1 expression versus prostate tumors with high Myc expression. In some embodiments, the metabolites that are differentially produced in prostate tumors with high Akt1 expression versus prostate tumors with high Myc expression are used in the methods described herein. By "differentially produced" it means that the average level of a metabolite in subjects with prostate tumors having high Akt1 expression has a statistically significant difference from that in subjects with prostate tumors having high Myc expression. For example, a significant difference that indicates differentially produced metabolite may be detected when the metabolite is present in prostate tumor with high Akt1 expression and absent in a prostate tumor with high Myc expression or vice versa. A significant difference that indicates differentially produced metabolite may be detected when the level of the metabolite in a prostate tumor sample of a subject with high Akt1 expression is at least 1%, at least 5%, at least 10%, at least 25%, at least 50%, at least 100%, at least 250%, at least 500%, or at least 1000% higher, or lower, than that of a subject with high Myc expression. Similarly, a significant difference may be detected when the level of a metabolite in a prostate tumor sample of a subject with high Aktl expression is at least 2-fold, at least 3-fold, at least 4-fold, at least 5-fold, at least 6-fold, at least 7-fold, at least 8-fold, at least 9-fold, at least 10-fold, at least 20-fold, at least 30-fold, at least 40-fold, at least 50-fold, at least 100-fold, or more higher, or lower, than that of a subject with high Myc expression. Significant differences may be identified by using an appropriate statistical test. Tests for statistical significance are well known in the art and are exemplified in Applied Statistics for Engineers and Scientists by Petruccelli, Chen and Nandram 1999 Reprint Ed. In some embodiments, the differentially produced metabolites are selected using a criteria of false discovery rate <0.2. In some embodiments, the differentially produced metabolites are selected using a criteria of p value <0.05. In some embodiments, the metabolites used in the methods described herein are selected from Table 1 or Table 2. In some embodiments, the metabolites used in the methods described herein comprise at least 10, at least 20, at least 30, at least 40, at least 50, at least 75, at least 100, at least 200, at least 300 of the metabolites described in Table 1 or Table 2.

As used herein, a "subject" refers to mammal, including humans and non-humans, such as primates. Typically the subject is a male human, and has been diagnosed as having a prostate tumor. In some embodiments, the subject may be diagnosed as having prostate tumor using one or more of the following tests: digital rectal exam (DRE), prostate imaging, biopsy with Gleason grading evaluation, presence of tumor markers such as prostate-specific antigen (PSA) and prostate cancer staging (Lumen et al. Screening and early diagnosis of prostate cancer: an update. Acta Clin Belg. 2012 Jul-Aug;67(4):270-5). In some embodiments, the subject has one or more clinical symptoms of prostate tumor. A variety of clinical symptoms of prostate cancer are known in the art. Examples of such symptoms include, but are not limited to, frequent urination, nocturia (increased urination at night), difficulty starting and maintaining a steady stream of urine, hematuria (blood in the urine), dysuria (painful urination) and bone pain.

Cancer or neoplasia is characterized by deregulated cell growth and division. A tumor arising in a tissue originating from endoderm or exoderm is called a carcinoma, and one arising in tissue originating from mesoderm is known as a sarcoma (Darnell, J. (1990) Molecular Cell Biology, Third Ed., W.H. Freeman, NY). Cancers may originate due to a mutation in an oncogene, or by inactivation of a tumor-suppressing genes (Weinberg, R.A. (Sept. 1988) Scientific Amer. 44-51). Examples of cancers include, but are not limited to cancers of the nervous system, breast, retina, lung, skin, kidney, liver, pancreas, genito-urinary tract, gastrointestinal tract, cancers of bone, and cancers of hematopoietic origin such as leukemias and lymphomas. In one embodiment of the present invention, the cancer is prostate cancer.

In some embodiments, the methods described herein are performed using a biological sample obtained from a subject. The term "biological sample" refers to a sample derived from a subject, e.g., a patient. Non-limiting examples of the biological sample include blood, serum, urine, and tissue. In some embodiments, the biological sample is a prostate tumor sample. Obtaining a prostate tumor sample from a subject means taking possession of a prostate tumor sample of the subject. The person obtaining a prostate tumor sample from a subject and performing an assay to measure a profile of metabolites in the sample does not necessarily obtain the sample from the subject. The sample may be removed from the subject by a medical practitioner (e.g., a doctor, nurse, or a clinical laboratory practitioner), and then provided to the person performing the assay to measure a profile of metabolites. The sample may be provided to the person performing an assay to measure the profile of metabolites by the subject or by a medical practitioner (e.g., a doctor, nurse, or a clinical laboratory practitioner). The person performing an assay to measure the profile of metabolites may have obtained a prostate tumor sample from the subject by removing the sample from the subject.

It is to be understood that a prostate tumor sample may be processed in any appropriate manner to facilitate measuring profiles of metabolites. For example, biochemical, mechanical and/or thermal processing methods may be appropriately used to isolate a biomolecule of interest from a prostate tumor sample. The levels of the metabolites may also be determined in a prostate tumor sample directly. The levels of the metabolites may be measured by performing an assay, such as but not limited to, mass spectroscopy, positron emission tomography, gas chromatography (GC-MS) or HPLC liquid chromatography (LC-MS), [(18)F]-fluorodeoxyglucose positron emission tomography (FDG-PET), and magnetic resonance spectroscopic imaging (MRSI). Other appropriate methods for determining levels of metabolites will be apparent to the skilled artisan.

The methods disclosed herein typically comprise performing an assay to measure a profile of metabolites and comparing, with at least one processor, the profile of the metabolites to an appropriate reference profile. In some embodiments, the levels of at least 10, at least 25, at least 50, at least 75, at least 100, at least 125, at least 150, at least 175, at least 200, at least 225, at least 250, at least 500, at least 750, at least 1000 or at least 1500 metabolites are measured and compared to assign an Akt1 and Myc status to the sample based on results of the comparison.

The assigned Akt1 and Myc status along with additional information such as the results of a PSA test and prostate imaging, can be used to determine the therapeutic options available to the subject. A report summarizing the results of the analysis, i.e. the assigned Akt1 and Myc status of the sample and any other information pertaining to the analysis could optionally be generated as part of the analysis (which may be interchangeably referred to herein as "providing" a report, "producing" a report, or "generating" a report). Examples of reports may include, but are not limited to, reports in paper (such as computer-generated printouts of test results) or equivalent formats and reports stored on computer readable medium (such as a CD, computer hard drive, or computer network server, etc.). Reports, particularly those stored on computer readable medium, can be part of a database (such as a database of patient records, which may be a "secure database" that has security features that limit access to the report, such as to allow only the patient and the patient's medical practitioners to view the report, for example). In addition to, or as an alternative to, generating a tangible report, reports can also be displayed on a computer screen (or the display of another electronic device or instrument).

A report can further be transmitted, communicated or reported (these terms may be used herein interchangeably), such as to the individual who was tested, a medical practitioner (e.g., a doctor, nurse, clinical laboratory practitioner, genetic counselor, etc.), a healthcare organization, a clinical laboratory, and/or any other party intended to view or possess the report. The act of 'transmitting' or 'communicating' a report can be by any means known in the art, based on the form of the report, and includes both oral and non-oral transmission. Furthermore, "transmitting" or "communicating" a report can include delivering a report ("pushing") and/or retrieving ("pulling") a report. For example, non-oral reports can be transmitted/communicated by such means as being physically transferred between parties (such as for reports in paper format), such as by being physically delivered from one party to another, or by being transmitted electronically or in signal form (e.g., via e-mail or over the internet, by facsimile, and/or by any wired or wireless communication methods known in the art), such as by being retrieved from a database stored on a computer network server, etc.

The Akt1 and Myc status of the sample isolated from a subject is assigned by comparing the profile of metabolites of the sample to an appropriate reference profile of the metabolites. An appropriate reference profile of the metabolites can be determined or can be a pre-existing reference profile. An appropriate reference profile includes profiles of the metabolites in prostate tumor with high Akt1 expression (i.e. prostate tumor or prostate cells having constitutively activated (phosphorylated) Ak1), in prostate tumor with low Akt1 expression (i.e. prostate tumor or prostate cells not having constitutively activated Ak1), in prostate tumor with high Myc expression (i.e. prostate tumor or prostate cells overexpressing Myc), and in prostate tumor with low Myc expression (i.e. prostate tumor or prostate cells not overexpressing Myc). A lack of a significant difference between the metabolic profile determined from the subject and the appropriate reference profile is indicative of the Akt1 and Myc status of the sample.

In some embodiments, the methods described herein involve using at least one processor programmed to recognize profiles of high Akt1 versus low Akt1 expressing tumors and high Myc versus low Myc expressing tumors to assign an Aktl and Myc status to the sample. The at least one processor assigns an Akt1 and Myc status to the sample isolated from the subject based on the profile of the metabolites of the sample. Typically the at least one processor is programmed using samples for which the Akt1 and Myc status has already been ascertained. Once the at least one processor is programmed, it may be applied to metabolic profiles obtained from a prostate tumor sample in order to assign an Akt1 and Myc status to the sample isolated from the subject. Thus, the methods may involve analyzing the metabolic profiles using one or more programmed processors to assign an Akt1 and Myc status to the sample based on the levels of the metabolites. The subject may be further diagnosed, e.g., by a health care provider, based on the assigned status.

The at least one processor may be programmed to assign a Akt1 and Myc status to a sample using one or more of a variety of techniques known in the art. For example, the at least one processor may be programmed to assign a Akt1 and Myc status using techniques including, but not limited to, logistic regression, partial least squares, linear discriminant analysis, regularized regression, quadratic discriminant analysis, neural network, naive Bayes, C4.5 decision tree, k-nearest neighbor, random forest, and support vector machine. The at least one processor may be programmed to assign a Akt1 and Myc status to a sample using a data set comprising profiles of the metabolites that are produced in high Akt1 prostate tumors, low Akt1 prostate tumors, high Myc prostate tumors and low Myc prostate tumors. The data set may also comprise metabolic profiles of control individuals identified as not having prostate tumor.

In some embodiments, the at least one processor is programmed to assign a Akt1 and Myc status to a sample using cluster analysis. Cluster analysis or clustering refers to assigning a objects in a set of objects into groups (called clusters) so that the objects in the same cluster are more similar (in some sense or another) to each other than to those in other clusters. Cluster analysis itself is not embodied in a single algorithm, but describes a general task to be solved. Cluster analysis may be performed using various algorithms that differ significantly in their notion of what constitutes a cluster and how to efficiently find them. Popular notions of clusters include groups with small distances among the cluster members, dense areas of the data space, intervals or particular statistical distributions. In some embodiments, one or more particular algorithms used to perform cluster analysis are selected from the group consisting of: hierarchical clustering, k-mean clustering, distribution-based clustering, and density-based clustering.

A confidence value can also be determined to specify the degree of confidence with which the at least one programmed processor has classified a biological sample. There may be instances in which a sample is tested, but does not belong, or cannot be reliably assigned a particular classification with sufficient confidence. This evaluation may be performed by utilizing a threshold in which a sample having a confidence value below the determined threshold is a sample that cannot be classified with sufficient confidence (e.g., a "no call"). In such instances, the classifier may provide an indication that the confidence value is below the threshold value. In some embodiments, the sample is then manually classified to assign an Akt1 and Myc status to the sample.

As will be appreciated by the skilled artisan, the strength of the status assigned to a sample by the at least one programmed processor may be assessed by a variety of parameters including, but not limited to, the accuracy, sensitivity, specificity and area under the receiver operation characteristic curve. Methods for computing accuracy, sensitivity and specificity are known in the art. The at least one programmed processor may have an accuracy of at least 60%, at least 65%, at least 70%, at least 75%, at least 80%, at least 85%, at least 90%, at least 95%, at least 99%, or more. The at least one programmed processor may have an accuracy score in a range of about 60% to 70%, 70% to 80%, 80% to 90%, or 90% to 100%. The at least one programmed processor may have a sensitivity score of at least 60%, at least 65%, at least 70%, at least 75%, at least 80%, at least 85%, at least 90%, at least 95%, at least 99%, or more. The at least one programmed processor may have a sensitivity score in a range of about 60% to 70%, 70% to 80%, 80% to 90%, or 90% to 100%. The at least one programmed processor may have a specificity score of at least 60%, at least 65%, at least 70%, at least 75%, at least 80%, at least 85%, at least 90%, at least 95%, at least 99%, or more. The at least one programmed processor may have a specificity score in a range of about 60% to 70%, 70% to 80%, 80% to 90%, or 90% to 100%.

The above-described embodiments of the present invention can be implemented in any of numerous ways. For example, the embodiments may be implemented using hardware, software or a combination thereof. When implemented in software, the software code can be executed on any suitable processor or collection of processors, whether provided in a single computer or distributed among multiple computers. It should be appreciated that any component or collection of components that perform the functions described above can be generically considered as one or more controllers that control the above-discussed functions. The one or more controllers can be implemented in numerous ways, such as with dedicated hardware, or with general purpose hardware (e.g., one or more processors) that is programmed using microcode or software to perform the functions recited above.

In this respect, it should be appreciated that one implementation of the embodiments of the present invention comprises at least one non-transitory computer-readable storage medium (e.g., a computer memory, a USB drive, a flash memory, a compact disk, a tape, etc.) encoded with a computer program (i.e., a plurality of instructions), which, when executed on a processor, performs the above-discussed functions of the embodiments of the present invention. The computer-readable storage medium can be transportable such that the program stored thereon can be loaded onto any computer resource to implement the aspects of the present invention discussed herein. In addition, it should be appreciated that the reference to a computer program which, when executed, performs the above-discussed functions, is not limited to an application program running on a host computer. Rather, the term computer program is used herein in a generic sense to reference any type of computer code (e.g., software or microcode) that can be employed to program a processor to implement the above-discussed aspects of the present invention.

An illustrative implementation of a computer system 700 that may be used in connection with any of the embodiments of the invention described herein is shown in FIG. 4. The computer system 700 may include one or more processors 710 and one or more computer-readable tangible non-transitory storage media (e.g., memory 720, one or more non-volatile storage media 730, or any other suitable storage device). The processor 710 may control writing data to and reading data from the memory 720 and the non-volatile storage device 730 in any suitable manner, as the aspects of the present invention described herein are not limited in this respect. To perform any of the functionality described herein, the processor 710 may execute one or more instructions stored in one or more computer-readable storage media (e.g., the memory 720), which may serve as tangible non-transitory computer-readable storage media storing instructions for execution by the processor 710.

According to the disclosure, the results of the methods may be useful in informing a treatment of a prostate tumor.In some instances of the disclosure, a method to treat prostate tumor comprises obtaining a biological sample from a subject; measuring a level of sarcosine in the sample; comparing the level of sarcosine in the sample to a control sarcosine level; and treating the subject with a Myc inhibitor when the measured level of sarcosine in the sample is increased relative to the control level.

Sarcosine, also known as N-methylglycine, is an intermediate and byproduct in glycine synthesis and degradation. Sarcosine is metabolized to glycine by the enzyme sarcosine dehydrogenase, while glycine-N-methyl transferase generates sarcosine from glycine. The level of sarcosine in the sample is measured using one or more of mass spectroscopy, nuclear magnetic resonance or chromatography. As described herein, the biological sample includes, but is not limited to urine, blood, serum, plasma, and tissue.

"Treat," "treating" and "treatment" encompasses an action that occurs while a subject is suffering from a condition which reduces the severity of the condition or retards or slows the progression of the condition ("therapeutic treatment"). "Treat," "treating" and "treatment" also encompasses an action that occurs before a subject begins to suffer from the condition and which inhibits or reduces the severity of the condition ("prophylactic treatment").

An Akt1 inhibitor includes, but is not limited to (a) a low molecular weight compound or high molecular weight compound which inhibits the phosphorylation of Akt1, (b) a low molecular weight compound or high molecular weight compound which inhibits the expression of Akt1, (c) an antibody which inhibits the phosphorylation of Akt1, (d) an antibody which inhibits the expression of Akt1, (e) a siRNA or shRNA against a polynucleotide encoding Akt1, (f) an antisense polynucleotide comprising a nucleotide sequence complementary or substantially complementary to the nucleotide sequence of a polynucleotide encoding Akt1, or comprising a part of said nucleotide sequence, (g) a ribozyme directed to a polynucleotide encoding Akt1, (h) a mutant of Akt1 which dominant-negatively acts on Akt1 or a polynucleotide encoding said mutant, and (i) an aptamer against Akt1. Akt1 inhibitors include Perifosine, Miltefosine, MK2206 (Hirai et al. Mol Cancer Ther. 2010 Jul;9(7):1956-67), GSK690693 (Rhodes et al. Cancer Res April 1, 2008 68; 2366), GDC-0068 (Saura et al. J Clin Oncol 30, 2012 (suppl; abstr 3021), or AZD5363 (Davies et al. (Mol Cancer Ther. 2012 Apr;11(4):873-87).

A Myc inhibitor includes, but is not limited to (a) a low molecular weight compound or high molecular weight compound which inhibits the expression of Myc, (b) an antibody which inhibits the expression of Myc, (e) a siRNA or shRNA against a polynucleotide encoding Myc, (f) an antisense polynucleotide comprising a nucleotide sequence complementary or substantially complementary to the nucleotide sequence of a polynucleotide encoding Myc, or comprising a part of said nucleotide sequence, (g) a ribozyme directed to a polynucleotide encoding Myc, (h) a mutant of Myc which dominant-negatively acts on Myc or a polynucleotide encoding said mutant, and (i) an aptamer against Myc. Myc inhibitors include 10058-F4 (Huang et al. Exp Hematol. 2006 Nov;34(11):1480-9.), JQ1 (Delmore et al. Cell. 2011 Sep 16;146(6):904-17) and Omomyc (Soucek et al. Cancer Res June 15, 2002 62; 3507).

The present invention and disclosure is further illustrated by the following Examples.

### EXAMPLES

### Methods:

### Generation of AKT1- and MYC-overexpressing RWPE-1

Immortalized human prostate epithelial RWPE-1 cells were infected with pBABE retroviral constructs of myristoylated *AKT1* (RW-AKT1) or *MYC* (RW-MYC), containing a puromycin resistance gene. Infection of pBABE vector alone (RW-EV) was used as a control. Cells were transduced through infection in the presence of polybrene (8 µg/mL), and retroviral supernatants were replaced with fresh media after 4 hours of incubation. Twenty-four hours later, Puromycin selection (1 µg/mL) was started. Cells were grown in phenol red-free Minimum Essential Media (MEM) supplemented with 10% Fetal Bovine Serum (FBS), 0.1 mM non-essential amino acids, 1 mM sodium pyruvate and penicillin-streptomycin (Gibco, Life Technologies).

### Transgenic mice

Ventral prostate lobes were isolated from 13 week-old MPAKT (4) and Lo-Myc (5) transgenic mice and from age-matched wild-type mice (FVB strain) within 10 minutes after CO₂ euthanasia. Tissues were snap-frozen in isopropanol cooled with dry ice immediately following harvest and stored at -80°C until metabolite extraction.

### Human prostate tissues

Fresh-frozen, optimal cutting temperature (OCT) compound-embedded radical prostatectomy samples were obtained from the Institutional tissue repository at the Dana-Farber Cancer Institute/Brigham and Women's Hospital (40 tumors and 21 normals) and from an independent collection of archival tissues (21 tumors and 4 normals; Dana-Farber Cancer Institute). All samples were collected with informed consent approved by the Institutional Review Board.

The presence and percentage of tumor was assessed in each tissue sample on frozen sections. One case was excluded from the study because of no tumor evidence. DNA, RNA and proteins were purified from serial 8-µm sections of each OCT-embedded tissue block. The remaining tissue was processed for metabolite extraction.

### Metabolite profiling

Metabolite profiling analysis was performed by Metabolon Inc. (Durham, NC) as previously described (Evans, A.M., DeHaven, C.D., Barrett, T., Mitchell, M. & Milgram, E. Integrated, nontargeted ultrahigh performance liquid chromatography/electrospray ionization tandem mass spectrometry platform for the identification and relative quantification of the small-molecule complement of biological systems. Anal Chem 81, 6656-6667 (2009); Sha, W., et al. Metabolomic profiling can predict which humans will develop liver dysfunction when deprived of dietary choline. FASEB J 24, 2962-2975 (2010)).
***Sample Accessioning.*** Each sample received was accessioned into the Metabolon LIMS system and was assigned by the LIMS a unique identifier that was associated with the original source identifier only. This identifier was used to track all sample handling, tasks, results etc. The samples (and all derived aliquots) were tracked by the LIMS system. All portions of any sample were automatically assigned their own unique identifiers by the LIMS when a new task is created; the relationship of these samples is also tracked. All samples were maintained at -80 °C until processed.
***Sample Preparation**.* Samples were prepared using the automated MicroLab STAR® system (Hamilton Robotics, Inc., NV). A recovery standard was added prior to the first step in the extraction process for QC purposes. Sample preparation was conducted using aqueous methanol extraction process to remove the protein fraction while allowing maximum recovery of small molecules. The resulting extract was divided into four fractions: one for analysis by UPLC/MS/MS (positive mode), one for UPLC/MS/MS (negative mode), one for GC/MS, and one for backup. Samples were placed briefly on a TurboVap® (Zymark) to remove the organic solvent. Each sample was then frozen and dried under vacuum. Samples were then prepared for the appropriate instrument, either UPLC/MS/MS or GC/MS.
***Ultrahigh performance liquid chromatography*/*Mass Spectroscopy (UPLC*/*MS*/*MS)***. The LC/MS portion of the platform was based on a Waters ACQUITY ultra-performance liquid chromatography (UPLC) and a Thermo-Finnigan linear trap quadrupole (LTQ) mass spectrometer, which consisted of an electrospray ionization (ESI) source and linear ion-trap (LIT) mass analyzer. The sample extract was dried then reconstituted in acidic or basic LC-compatible solvents, each of which contained 8 or more injection standards at fixed concentrations to ensure injection and chromatographic consistency. One aliquot was analyzed using acidic positive ion optimized conditions and the other using basic negative ion optimized conditions in two independent injections using separate dedicated columns. Extracts reconstituted in acidic conditions were gradient eluted using water and methanol containing 0.1% formic acid, while the basic extracts, which also used water/methanol, contained 6.5mM Ammonium Bicarbonate. The MS analysis alternated between MS and data-dependent MS² scans using dynamic exclusion. Raw data files are archived and extracted as described below.
***Gas chromatography*/*Mass Spectroscopy (GC*/*****MS).*** The samples destined for GC/MS analysis were re-dried under vacuum desiccation for a minimum of 24 hours prior to being derivatized under dried nitrogen using bistrimethyl-silyl-triflouroacetamide (BSTFA). The GC column was 5% phenyl and the temperature ramp was from 40° to 300° C in a 16 minute period. Samples were analyzed on a Thermo-Finnigan Trace DSQ fast-scanning single-quadrupole mass spectrometer using electron impact ionization. The instrument was tuned and calibrated for mass resolution and mass accuracy on a daily basis. The information output from the raw data files was automatically extracted as discussed below.
***Quality assurance*/*QC.*** For QA/QC purposes, additional samples were included with each day's analysis. These samples included extracts of a pool of well-characterized human plasma, extracts of a pool created from a small aliquot of the experimental samples, and process blanks. QC samples were spaced evenly among the injections and all experimental samples were randomly distributed throughout the run. A selection of QC compounds was added to every sample for chromatographic alignment, including those under test. These compounds were carefully chosen so as not to interfere with the measurement of the endogenous compounds.
***Data extraction and compound identification.*** Raw data was extracted, peak-identified and QC processed using Metabolon's hardware and software. These systems are built on a web-service platform utilizing Microsoft's .NET technologies, which run on high-performance application servers and fiber-channel storage arrays in clusters to provide active failover and load-balancing (Dehaven, C.D., Evans, A.M., Dai, H. & Lawton, K.A. Organization of GC/MS and LC/MS metabolomics data into chemical libraries. J Cheminform 2, 9 (2010)). Compounds were identified by comparison to library entries of purified standards or recurrent unknown entities. Metabolon maintains a library based on authenticated standards that contains the retention time/index (RI), mass to charge ratio (*m*/*z*), and chromatographic data (including MS/MS spectral data) on all molecules present in the library. Furthermore, biochemical identifications are based on three criteria: retention index within a narrow RI window of the proposed identification, nominal mass match to the library +/- 0.2 amu (atomic mass units), and the MS/MS forward and reverse scores between the experimental data and authentic standards. The MS/MS scores are based on a comparison of the ions present in the experimental spectrum to the ions present in the library spectrum. While there may be similarities between these molecules based on one of these factors, the use of all three data points can be utilized to distinguish and differentiate biochemicals. More than 2400 commercially available purified standard compounds have been acquired and registered into LIMS for distribution to both the LC and GC platforms for determination of their analytical characteristics.
***Data analysis:*** For studies spanning multiple days, a data normalization step is performed to correct variation resulting from instrument inter-day tuning differences. Essentially, each compound is corrected in run-day blocks by registering the medians to equal one (1.00) and normalizing each data point proportionately (termed the "block correction"). For studies that do not require more than one day of analysis, no normalization is necessary, other than for purposes of data visualization. Second, for each sample, metabolite values are normalized by cell count (cell lines) or tissue weight (mouse or human prostate tissue). Third, median scaling of each metabolite across all samples and imputation of each metabolite by the minimum observed value of that compound were performed. Finally, quantile normalization of every sample was applied to ensure statistically comparable distributions. To obtain differential metabolites across 3 classes, MYC-high, phosphoAKT-high and control, we used the one class-versus-all permutation based t test, as implemented in GenePattern (Reich, M., et al. GenePattern 2.0. Nat Genet 38, 500-501 (2006)) to identify compounds associated with MYC or AKT overexpression. A p-value threshold of 0.05 was used to determine the significant compounds. GeneSet Enrichment Analysis (GSEA) (Subramanian, A., et al. Gene set enrichment analysis: a knowledge-based approach for interpreting genome-wide expression profiles. Proc Natl Acad Sci U S A 102, 15545-15550 (2005)) was used to measure the enrichment of KEGG defined pathways²³ both within (i) individual samples and (ii) across MYC-high and AKT-high samples, as previously described (Subramanian, A., et al. Gene set enrichment analysis: a knowledge-based approach for interpreting genome-wide expression profiles. Proc Natl Acad Sci U S A 102, 15545-15550 (2005); Barbie, D.A., et al. Systematic RNA interference reveals that oncogenic KRAS-driven cancers require TBK1. Nature 462, 108-112 (2009). Gene set-size-normalized enrichment scores (NES) from GSEA were used to determine the extent and direction of enrichment for each pathway in different systems that were represented by at least 2 metabolites. The mean NES of the 3 systems was computed for each pathway and the pathways that are consistently enriched across all systems were detected as outliers using box-and-whisker plots (with 75% or more times the inter-quartile range from the box).

### Single Nucleotide Polymorphisms (SNP) arrays

Two-hundred-fifty ng of DNA extracted from 60 prostate tumors and 6 matched normal tissue samples were labeled and hybridized to the Affymetrix 250K Sty I array to obtain signal intensities and genotype calls (Microarray core facility, Dana-Farber Cancer Institute). Signal intensities were normalized against data from normal samples. Copy-number profiles were inferred and the significance of somatic copy-number alterations was determined using the GISTIC module in GenePattern. The heat map was generated using DChip 2010.01 (http://biosun1.harvard.edu/complab/dchip/download.htm).

### mRNA expression analysis

Total RNA was isolated from RWPE-EV, RWPE-AKT1 and RWPE-MYC cells (RNeasy Micro Kit, Qiagen Inc., CA) and from the prostate tumors and matched normal controls (AllPrep DNA/RNA Micro Kit, Qiagen Inc.). Two micrograms of RNA from each isogenic cell line were retro-transcribed with the SuperScript™ First-Strand Synthesis System (Invitrogen, Life Technologies Corporation, NY), and 5 ng of cDNA were used per each gene expression reaction with the specific TaqMan probe (Applied Biosystems). For the human prostate tissues, 300-400 ng of purified RNA were retro-transcribed using High Capacity cDNA Reverse transcription kit (Applied Biosystems). One hundred ng of cDNA was used to perform relative real time PCR using custom micro fluidic cards (Taqman Custom Arrays, Applied Biosystems) and Applied Biosystems 7900 HT Fast Real-Time System, as described by the manufacturer. All samples were run in duplicate and normalized to the average of actin, gus and 18S rRNA, which have stable expression in our experimental conditions. Data were analyzed using the ΔΔCt method and obtained values were expressed as n-fold the calibrator (RWPE-1 cells or the average of 8 normal prostate tissues) set as 1. Probes and primers included in the fluidic card were purchased from Applied Biosystems. One-sample T-Test was applied and significance was defined with p<0.05.

### Results:

To profile the metabolic heterogeneity of prostate cancer in an oncogene-specific context, phosphorylated AKT1- or MYC-associated metabolomic signatures from prostate epithelial cells in monolayer culture, transgenic mouse prostate and primary nonmetastatic prostate tumors were integrated. The aim was to identify patterns of metabolomic changes that were different for the two oncogenes but common for the three biological systems.

First, it was determined whether genomic alterations at the PTEN or MYC loci would be predictive of active AKT1 or MYC overexpression in a cohort of 60 prostate tumors obtained from the Institutional Tissue Repository. These tumors were pathological stage T2, 22 high Gleason (4+3 or 4+4) and 38 low Gleason (3+3 or 3+4). Genomic DNA and proteins extracted from sections of each tumor or nontumoral matched control sample were assayed by Single Nucleotide Polymorphisms (SNP) arrays and western blotting (phosphorylated AKT1 and MYC). SNP arrays revealed that 20% of these tumors harbored 10q loss and 18% harbored 8q gain. K-means clustering of phosphorylated AKT1 and MYC western blot densitometric values was conducted in parallel to segregate 4 prostate tumor subgroups, i.e. phosphoAKT1-high/MYC-high, phosphoAKT1-high/MYC-low, phosphoAKT1-low/MYC-high and phosphoAKT1-low/MYC-low (Fig. 1B). Importantly, the genomic alterations only counted for 7/27 (26%) of phosphoAKT1-high tumors and for 2/15 (13%) of MYC-high tumors, suggesting the protein signature to be the most accurate to assess activation of AKT1 or MYC (Fig.1A). In addition, levels of phosphoAKT1 and MYC were not associated with the Gleason grade of the tumors.

Next, to define differential metabolic reprogramming induced by sole activation of AKT1 or overexpression of MYC in non-transformed prostate, mass-spectrometry based metabolomics of prostate epithelial RWPE-1 cells genetically engineered with constructs encoding myristoylated AKT1 or MYC, and transgenic mice expressing human myristoylated AKT1 or MYC in the prostate was performed. Interestingly, while both RW-AKT1 and RW-MYC cells showed significant changes in intermediates of glycolysis, only RW-AKT1 cells exhibited the aerobic glycolytic phenotype (Fig. 2A). These results were even more pronounced *in vivo* (FIG. 2B and Fig. 2C), with exclusively the MPAKT transgenic mouse prostate being characterized by both very high levels of glucose metabolism intermediates and lactate (Fig. 2B). In turn, MYC overexpression was associated with a distinctive signature of lipids, including enrichment of metabolites sets of unsaturated fatty acids both in transgenic mouse prostate and in human tumors. When applied to primary non-metastatic prostate tumors stratified by the expression levels of phospho-AKT1 and MYC, the pathway enrichment analysis revealed that MYC-high tumors rather show a negative enrichment of glycolysis compared to phosphoAKT1-high and nontumoral prostate tissue (Fig. 2C).

Next, the AKT1 and MYC metabolic signatures were compared directly. The list of metabolites with fold changes and p-values (phosphoAKT1-high vs. MYC-high) per data set (RWPE cells, probasin-driven transgenic mice and prostate tumors) is given in the Table 2. Pathway enrichment analysis by GSEA was used to determine which metabolic pathways were commonly enriched in the genetically engineered models and in the prostate tumor subgroups defined above, specifically comparing high AKT1 with high MYC background (Fig. 2D). Complete lists of the metabolite sets tested, the number of metabolites per set, and the enrichment scores are included in the Table 3. In detail, gene set-size-normalized enrichment scores (NES) from GSEA were used to determine the extent and direction of enrichment for each pathway in the 3 data sets. Five pathways with highly positive NES and 2 pathways with highly negative NES across biological systems were defined as outliers (FIG. 2D and FIG. 3E). This analysis showed that AKT1 exquisitely drives aerobic glycolysis and other glucose-related pathways such as the pentose phosphate shunt and fructose metabolism, whereas MYC is a promoter of lipid metabolism (FIG. 3E). On the one hand, enrichment of the glycerophospholipid, glycerolipid and pantothenate/coA biosynthesis metabolite sets, as well as higher levels of lipogenesis-feeding metabolites such as citrate, were distinctively associated with MYC overexpression in RWPE cells, suggesting that MYC induces synthesis and/or turnover of membrane lipids. This would be justified by the higher proliferation requirement of these cells. On the other hand, it was intriguing to find higher levels of omega-3 (docosapentaenoate and docosahexaenoate) and omega-6 (arachidonate, docosadienoate and dihomo-linolenate) fatty acids in the ventral prostate of Lo-MYC mice and in MYC-high/phosphoAKT1-low prostate tumors relative to MPAKT mice and phosphoAKT1-high/MYC-low tumors, respectively (FIG. 3E). These are essential fatty acids, therefore obtained from extracellular sources. Although the precise role of these unsaturated fatty acids in prostate cancer is not completely understood, the data reveals that prostate cells may increase their lipid needs early during transformation, as seen in Low-MYC mice. One possibility would be that these lipids are used as energy sources via oxidation.

Finally, it was determined whether the metabolome changes associated with the oncogenic transformation of prostate epithelial cells are accompanied by transcriptional changes in key metabolic enzymes. Consistent with the metabolite profiling of RWPE-1 cells, glycolytic enzymes such as the glucose transporter GLUT-1, the hexokinases 1 and 2, and the aldose reductase AKR1B1 were significantly increased upon AKT1 overexpression/activation (FIG. 3A, 3D), whereas only a moderate increase in hexokinase 2 occurred in RWPE-MYC cells. When looking at lipogenic enzymes, instead, two key enzymes of the glycerophospholipid metabolism, choline kinase and cholinephosphotransferase-1, were both induced by MYC overexpression (FIG. 3B,3D), validating the enrichment of the glycerophospholipid metabolic set in RWPE-MYC cells (Fig. 3B). The glutamine pathway was also affected by the activation/overexpression of AKT1 and MYC. While both oncogenes increased the mRNA levels of the neutral amino acid transporter ASCT2, only MYC significantly induced glutaminase, the glutaminolytic enzyme responsible for the conversion of glutamine into glutamate (FIG. 3C, 3D). In addition, sarcosine, an intermediate of the glycine and choline metabolism previously identified as a progression marker in prostate cancer, increased exclusively in the prostate of Lo-MYC mice. Associated with the sarcosine increase were a concomitant elevation of the intermediate betaine and a decrease in glycine levels. These results suggest a dysregulation of the sarcosine pathway upon MYC overexpression.

To identify unique mRNA expression changes in phosphoAKT1-low/MYC-high (n=5) and phosphoAKT1-low/MYC-high (n=13) prostate tumors, a qPCR-based expression profiling analysis was performed of 29 metabolic genes in the 2 tumor groups relative to normal prostate tissues (n=8). Consistent with the metabolomics results, high MYC expression in a phosphoAKT1-low context in human tumors was associated with decreased mRNA expression of the glucose transporter-1 (GLUT-1) (Fig. 3D, 3F). No decrease in GLUT-1 expression was found in phosphoAKT1-high/MYC-high tumors (n=3) (Fig. 4e). Altogether, these results suggest that MYC activation affects glucose uptake and glucose utilization rate in prostate tumors.

In summary, the data demonstrates that individual prostate tumors have distinct metabolic phenotypes resulting from their genetic complexity, and reveal a novel metabolic role for MYC in prostate cancer. The evidence that MYC overexpression inversely associates with GLUT-1 mRNA expression and with the AKT1-dependent "Warburg effect" metabolic phenotype in transformed prostate cells opens novel avenues for the metabolic imaging of prostate cancer patients whose tumors harbor 8q amplification or PTEN loss and/or show MYC or AKT1 activation. Through large-scale metabolite analyses and isotopic labeling approaches, as well as generation of metabolic set enrichment pathways, it was found that AKT1 drives primarily aerobic glycolysis while MYC does not elicit a Warburg-like effect and significantly enhances glycerophospholipid synthesis instead. This regulation is Gleason grade- and pathological stage-independent. These results demonstrates that human prostate tumors exhibit metabolic fingerprints of their molecular phenotypes, which may have impact on metabolic diagnostics and targeted therapeutics.

**Table 1: List of metabolites tested.**

| **Id** | **Compound** | **KE GG _Id** | **Family** | **Pathway** |
|---|---|---|---|---|
| M37180 | 2 amino p cresol sulfate | NA | Amino acid | Phenylalanine and tyrosine metabolism |
| M1126 | alanine | C00041 | Amino_acid | Alanine_and_aspartate_metabolism |
| M11398 | asparagine | C00152 | Amino_acid | Alanine_and_aspartate_metabolism |
| M1585 | N-acetylalanine | C02847 | Amino_acid | Alanine_and_aspartate_metabolism |
| M15996 | aspartate | C00049 | Amino_acid | Alanine_and_aspartate_metabolism |
| M22185 | N-acetylaspartate | C01042 | Amino_acid | Alanine_and_aspartate_metabolism |
| M3155 | 3-ureidopropionate | C02642 | Amino_acid | Alanine_and_aspartate_metabolism |
| M443 | aspartate | C00049 | Amino_acid | Alanine_and_aspartate_metabolism |
| M55 | beta-alanine | C00099 | Amino_acid | Alanine_and_aspartate_metabolism |
| M1577 | 2-aminobutyrate | C02261 | Amino_acid | Butanoate_metabolism |
| M27718 | creatine | C00300 | Amino_acid | Creatine_metabolism |
| M513 | creatinine | C00791 | Amino_acid | Creatine_metabolism |
| M1302 | methionine | C00073 | Amino_acid | Cysteine,_methionine,_SAM,_taurine_metabolism |
| M15705 | cystathionine | C02291 | Amino_acid | Cysteme,_methionme,_SAM,_taurine_metabolism |
| M1589 | N-acetylmethionine | C02712 | Amino_acid | Cysteine,_methionine,_SAM ,_taurine_metabolism |
| M15948 | S-adenosylhomocyst eine | C00021 | Amino_acid | Cysteine,_methionine,_SAM,_taurine_metabolism |
| M21044 | 2-hydroxybutyrate | C05984 | Amino_acid | Cysteine,_methionine,_SAM,_taurine_metabolism |
| M2125 | taurine | C00245 | Amino_acid | Cysteine,_methionine,_SAM,_taurine_metabolism |
| M31453 | cysteine | C00097 | Amino_acid | Cysteine,_methionine,_SAM,_taurine_me tabolism |
| M31454 | cystine | C00491 | Amino_acid | Cysteine,_methionine,_S_AM,_taurine_metabolism |
| M590 | hypo taurine | C00519 | Amino_acid | Cysteine,_methionine,_SAM,_taurine_metabolism |
| M1416 | gamma-aminobutyrate | C00334 | Amino_acid | Glutamate_metabolism |
| M1647 | glutamine | C00064 | Amino_acid | Glutamate_metabolism |
| M32672 | pyroglutamine | NA | Amino_acid | Glutamate_metabolism |
| M33487 | glutamate, gamma-methyl ester | NA | Amino_acid | Glutamate_metabolism |
| M33943 | N-acetylglutamine | C02716 | Amino_acid | Glutamate_metabolism |
| M35665 | N-acetyl-aspartyl-glutamate | C12270 | Amino_acid | Glutamate_metabolism |
| M53 | glutamine | C00064 | Amino_acid | Glutamate_metabolism |
| M57 | glutamate | C00025 | Amino_acid | Glutamate_metabolism |
| M1494 | 5-oxoproline | C01879 | Amino_acid | Glutathione_metabolism |
| M15731 | s-lactoylglutathione | C03451 | Amino_acid | Glutathione_metabolism |
| M2127 | glutathione, reduced | C00051 | Amino_acid | Glutathione_metabolism |
| M27727 | glutathione, oxidized | C00127 | Amino_acid | Glutathione_metabolism |
| M33016 | ophthalmate | NA | Amino_acid | Glutathione_metabolism |
| M34592 | ophthalmate | NA | Amino_acid | Glutathione_metabolism |
| M35159 | cysteine-glutathione disulfide | NA | Amino_acid | Glutathione_metabolism |
| M11777 | glycine | C00037 | Amino_acid | Glycine,_serine_and_threonine_metabolism |
| M1284 | threonine | C00188 | Amino_acid | Glycine,_serine_and_threonine_metabolism |
| M1516 | sarcosine | C00213 | Amino_acid | Glycine,_serine_and_threonine_metabolism |
| M1648 | serine | C00065 | Amino_acid | Glycine,_serine_and_threonine_metabolism |
| M3141 | betaine | C00719 | Amino_acid | Glycine,_serine_and_threonine_metabolism |
| M33939 | N-acetylthreonine | C01118 | Amino_acid | Glycine,_serine_and_threonine_metabolism |
| M37076 | N-acetylserine | NA | Amino_acid | Glycine,_serine_and_threonine_metabolism |
| M15681 | 4-guanidinobutanoat e | C01035 | Amino_acid | Guanidino_and_acetamido_metabolism |
| M15677 | 3 -methylhistidine | C01152 | Amino_acid | Histidine_metabolism |
| M1574 | histamine | C00388 | Amino_acid | Histidine_metabolism |
| M32350 | 1-methylimidazoleac etate | C05828 | Amino_acid | Histidine_metabolism |
| M59 | histidine | C00135 | Amino_acid | Histidine_metabolism |
| M607 | urocanate | C00785 | Amino_acid | Histidine_metabolism |
| M1301 | lysine | C00047 | Amino_acid | Lysine_metabolism |
| M1444 | pipecolate | C00408 | Amino_acid | Lysine_metabolism |
| M1495 | saccharopine | C00449 | Amino_acid | Lysine_metabolism |
| M35439 | glutaroyl carnitine | NA | Amino_acid | Lysine_metabolism |
| M36752 | N6-acetyllysine | C02727 | Amino_acid | Lysine_metabolism |
| M396 | glutarate | C00489 | Amino_acid | Lysine_metabolism |
| M6146 | 2-aminoadipate | C00956 | Amino_acid | Lysine_metabolism |
| M1299 | tyrosine | C00082 | Amino_acid | Phenylalanine_&_tyrosine_metabolism |
| M32197 | 3-(4-hydroxyphenyl)lac tate | C03672 | Amino_acid | Phenylalanine_&_tyrosine_metabolism |
| M32553 | phenol sulfate | C02180 | Amino_acid | Phenylalanine_&_tyrosine_metabolism |
| M33945 | phenylacetylglycin e | C05598 | Amino_acid | Phenylalanine_&_tyrosine_metabolism |
| M35126 | phenylacetylglutamine | C05597 | Amino_acid | Phenylalanine_&_tyrosine_metabolism |
| M36103 | p-cresol sulfate | C01468 | Amino_acid | Phenylalanine_&_tyrosine_metabolism |
| M64 | phenylalanine | C00079 | Amino_acid | Phenylalanine_&_tyrosine_metabolism |
| M1408 | putrescine | C00134 | Amino_acid | Polyamine_metabolism |
| M1419 | 5-methylthioadenosine | C00170 | Amino_acid | Polyamine_metabolism |
| M15496 | agmatine | C00179 | Amino_acid | Polyamine_metabolism |
| M37496 | N-acetylputrescine | C02714 | Amino_acid | Polyamine_metabolism |
| M485 | spermidine | C00315 | Amino_acid | Polyamine_metabolism |
| M603 | spermine | C00750 | Amino_acid | Polyamine_metabolism |
| M15140 | kynurenine | C00328 | Amino_acid | Tryptophan_metabolism |
| M18349 | indolelactate | C02043 | Amino_acid | Tryptophan_metabolism |
| M2342 | serotonin | C00780 | Amino_acid | Tryptophan_metabolism |
| M27672 | 3-indoxyl sulfate | NA | Amino_acid | Tryptophan_metabolism |
| M32675 | c-glycosyltryptophan | NA | Amino_acid | Tryptophan_metabolism |
| M33959 | N-acetyltryptophan | C03137 | Amino_acid | Tryptophan_metabolism |
| M37097 | tryptophan betaine | C09213 | Amino_acid | Tryptophan_metabolism |
| M437 | 5-hydroxyindoleacetate | C05635 | Amino_acid | Tryptophan_metabolism |
| M54 | tryptophan | C00078 | Amino_acid | Tryptophan_metabolism |
| M1366 | trans-4-hydroxyproline | C01157 | Amino_acid | Urea_cycle;_arginine-,_proline-,_ metabolism |
| M1493 | ornithine | C00077 | Amino_acid | Urea_cycle;_arginine-,_proline-,_ metabolism |
| M1638 | arginine | C00062 | Amino_acid | Urea_cycle;_arginine-,_proline-,_ metabolism |
| M1670 | urea | C00086 | Amino_acid | Urea_cycle;_arginine-,_proline-,_ metabolism |
| M1898 | proline | C00148 | Amino_acid | Urea_cycle;_arginine-,_proline-,_ metabolism |
| M2132 | citrulline | C00327 | Amino_acid | Urea_cycle;_arginine-,_proline-,_ metabolism |
| M34384 | stachydrine | C10172 | Amino_acid | Urea_cycle;_arginine-,_proline-,_ metabolism |
| M36808 | dimethylarginine | C03626 | Amino_acid | Urea_cycle;_arginine-,_proline-,_ metabolism |
| M1125 | isoleucine | C00407 | Amino_acid | Valine,_leucine_and_isoleucine_metabolism |
| M12129 | beta-hydroxyisovalerate | NA | Amino_acid | Valine,_leucine_and_isoleucine_metabolism |
| M1649 | valine | C00183 | Amino_acid | Valine,_leucine_and_isoleucine_metabolism |
| M32776 | 2-methylbutyroylcarnitine | NA | Amino_acid | Valine,_leucine_and_isoleucine_metabolism |
| M33441 | isobutyrylcarnitine | NA | Amino_acid | Valine,_leucine_and_isoleucine_metabolism |
| M33937 | alpha-hydroxyisovalerate | NA | Amino_acid | Valine,_leucine_and_isoleucine_metabolism |
| M34407 | isovalerylcarnitine | NA | Amino_acid | Valine,_leucine_and_isoleucine_metabolism |
| M35107 | isovalerylglycine | NA | Amino_acid | Valine,_leucine_and_isoleucine_metabolism |
| M35428 | tiglyl carnitine | NA | Amino_acid | Valine,_leucine_and_isoleucine_metabolism |
| M35431 | 2-methylbutyroylcarnitine | NA | Amino_acid | Valine,_leucine_and_isoleucine_metabolism |
| M35433 | hydroxyisovaleroylcarnitine | NA | Amino_acid | Valine,_leucine_and_isoleucine_metabolism |
| M60 | leucine | C00123 | Amino_acid | Valine,_leucine_and_isoleucine_metabolism |
| M15095 | N-acetylglucosamine | C03878 | Carbohydrate | Aminosugars_metabolism |
| M15096 | N-acetylglucosamine | C00140 | Carbohydrate | Aminosugars_metabolism |
| M15821 | fucose | C00382 | Carbohydrate | Aminosugars_metabolism |
| M1592 | N-acetylneuraminate | C00270 | Carbohydrate | Aminosugars_metabolism |
| M32377 | N-acetylneuraminate | C00270 | Carbohydrate | Aminosugars_metabolism |
| M33477 | erythronate | NA | Carbohydrate | Aminosugars_metabolism |
| M12055 | galactose | C01662 | Carbohydrate | Fructose,_mannose,_galactose,_ starch,_and_sucrose_metabolism |
| M1470 | mannose-6-phosphate | C00275 | Carbohydrate | Fructose,_mannose,_galactose,_starch,_and_sucrose_metabolism |
| M15053 | sorbitol | C00794 | Carbohydrate | Fructose,_mannose,_galactose,_starch,_and_sucrose_metabolism |
| M15335 | mannitol | C00392 | Carbohydrate | Fructose,_mannose,_galactose,_starch,_and_sucrose_metabolism |
| M15804 | maltose | C00208 | Carbohydrate | Fructose,_mannose,_galactose,_ starch,_and_sucrose_metabolism |
| M15877 | maltotriose | C01835 | Carbohydrate | Fructose,_mannose,_galactose,_starch,_and_sucrose_metabolism |
| M15910 | maltotetraose | C02052 | Carbohydrate | Fructose,_mannose,_galactose,_starch,_and_sucrose_metabolism |
| M31266 | fructose | C00095 | Carbohydrate | Fructose,_mannose,_galactose,_starch,_and_sucrose_metabolism |
| M577 | fructose | C00095 | Carbohydrate | Fructose,_mannose,_galactose,_starch,_and_sucrose metabolism |
| M584 | mannose | C00159 | Carbohydrate | Fructose,_mannose,_galactose,_starch,_and_sucrose_metabolism |
| M12021 | fructose-6-phosphate | C05345 | Carbohydrate | Glycolysis,_gluconeogenesis,_pyruvate_metabolism |
| M1414 | 3-phosphoglycerate | C00597 | Carbohydrate | Glycolysis,_gluconeogenesis,_pyruvate_metabolism |
| M15443 | glucuronate | C00191 | Carbohydrate | Glycolysis,_gluconeogenesis,_pyruvate_metabolism |
| M1572 | glycerate | C00258 | Carbohydrate | Glycolysis,_gluconeogenesis,_pyruvate_metabolism |
| M15926 | fructose 1,6-bisphosphate | C05378 | Carbohydrate | Glycolysis,_gluconeogenesis,_pyruvate_metabolism |
| M20488 | glucose | C00293 | Carbohydrate | Glycolysis,_gluconeogenesis,_pyruvate_metabolism |
| M20675 | 1,5-anhydroglucitol | C07326 | Carbohydrate | Glycolysis,_gluconeogenesis,_pyruvate_metabolism |
| M31260 | glucose-6-phosphate | C00668 | Carbohydrate | Glycolysis,_gluconeogenesis,_pyruvate_metabolism |
| M36984 | Isobar: fructose 1,6-diphosphate, glucose 1,6-diphosphate | NA | Carbohydrate | Glycolysis,_gluconeogenesis,_pyruvate_metabolism |
| M527 | lactate | C00186 | Carbohydrate | Glycolysis,_gluconeogenesis,_pyruvate_metabolism |
| M599 | pyruvate | C00022 | Carbohydrate | Glycolysis,_gluconeogenesis,_pyruvate_metabolism |
| M12083 | ribose | C00121 | Carbohydrate | Nucleotide_sugars,_pentose_metabolism |
| M1475 | ribulose 5-phosphate | C00199 | Carbohydrate | Nucleotide_sugars,_pentose_metabolism |
| M15442 | 6-phosphogluconate | C00345 | Carbohydrate | Nucleotide_sugars,_pentose_metabolism |
| M15772 | ribitol | C00474 | Carbohydrate | Nucleotide_sugars,_pentose_metabolism |
| M15835 | xylose | NA | Carbohydrate | Nucleotide_sugars,_pentose_metabolism |
| M15964 | arabitol | C00474 | Carbohydrate | Nucleotide_sugars,_pentose_metabolism |
| M18344 | xylulose | C00310 | Carbohydrate | Nucleotide_sugars,_pentose_metabolism |
| M2763 | UDP-glucuronate | C00167 | Carbohydrate | Nucleotide_sugars,_pentose_metabolism |
| M32344 | UDP-glucose | C00029 | Carbohydrate | Nucleotide_sugars,_pentose_metabolism |
| M32976 | UDP-glucose | C00029 | Carbohydrate | Nucleotide_sugars,_pentose_metabolism |
| M35162 | UDP-N-acetylglucosamine | C00043 | Carbohydrate | Nucleotide_sugars,_pentose_metabolism |
| M35855 | ribulose | C00309 | Carbohydrate | Nucleotide_sugars,_pentose_metabolism |
| M4966 | xylitol | C00379 | Carbohydrate | Nucleotide_sugars,_pentose_metabolism |
| M561 | ribose 5-phosphate | C00117 | Carbohydrate | Nucleotide_sugars,_pentose_metabolism |
| M575 | arabinose | C00181 | Carbohydrate | Nucleotide_sugars,_pentose_metabolism |
| M587 | gluconate | C00257 | Carbohydrate | Nucleotide_sugars,_pentose_metabolism |
| M1640 | ascorbate | C00072 | Cofactors_and_vitamins | Ascorbate_and_aldarate_metabolism |
| M33454 | gulono-1,4-lactone | C01040 | Cofactors_and_vitamins | Ascorbate_and_aldarate_metabolism |
| M32593 | heme* | C00032 | Cofactors_and_vitamins | Hemoglobin_ and_porphyrin |
| M1899 | quinolinate | C03722 | Cofactors_and_vitamins | Nicotinate_and_nicotinamide_metabolism |
| M22152 | nicotinamide ribonucleotide | C00455 | Cofactors_and_vitamins | Nicotinate_and_nicotinamide_metabolism |
| M27665 | 1-methylnicotinamide | C02918 | Cofactors_and_vitamins | Nicotinate_and_nicotinamide_metabolism |
| M31475 | nicotinamide adenine dinucleotide reduced | C00004 | Cofactors_and_vitamins | Nicotinate_and_nicotinamide_metabolism |
| M32380 | nicotinamide adenine dinucleotide phosphate | C00005 | Cofactors_and_vitamins | Nicotinate_and_nicotinamide_metabolism |
| M32401 | trigonelline | C01004 | Cofactors_and_vitamins | Nicotinate_and_nicotinamide_metabolism |
| M33013 | nicotinamide riboside | C03150 | Cofactors_and_vitamins | Nicotinate_and_nicotinamide_metabolism |
| M5278 | nicotinamide adenine dinucleotide | C00003 | Cofactors_and_vitamins | Nicotinate_and_nicotinamide_metabolism |
| M558 | adenosine 5'diphosphoribose | C00301 | Cofactors_and_vitamins | Nicotinate_and_nicotinamide_metabolism |
| M594 | nicotinamide | C00153 | Cofactors_and_vitamins | Nicotinate_and_nicotinamide_metabolism |
| M1508 | pantothenate | C00864 | Cofactors_and_vitamins | Pantothenate_and_CoA_metabolism |
| M18289 | 3'-dephosphocoenzyme A | C00882 | Cofactors_and_vitamins | Pantothenate_and_CoA_metabolism |
| M2936 | coenzyme A | C00010 | Cofactors_and_vitamins | Pantothenate_and_CoA_metabolism |
| M1827 | riboflavin | C00255 | Cofactors_and_vitamins | Riboflavin_metabolism |
| M2134 | flavin adenine dinucleotide | C00016 | Cofactors_and_vitamins | Riboflavin_metabolism |
| M5341 | thiamin | C00378 | Cofactors_and_vitamins | Thiamine_metabolism |
| M1561 | alpha-tocopherol | C02477 | Cofactors_and_vitamins | Tocopherol_metabolism |
| M33420 | gamma-tocopherol | C02483 | Cofactors_and_vitamins | Tocopherol_metabolism |
| M31555 | pyridoxate | C00847 | Cofactors_and_vitamins | Vitamin_B6_metabolism |
| M12025 | cis-aconitate | C00417 | Energy | Krebs_cycle |
| M12110 | isocitrate | C00311 | Energy | Krebs_cycle |
| M1303 | malate | C00149 | Energy | Krebs_cycle |
| M1437 | succinate | C00042 | Energy | Krebs_cycle |
| M1564 | citrate | C00158 | Energy | Krebs_cycle |
| M1643 | fumarate | C00122 | Energy | Krebs_cycle |
| M33453 | alpha-ketoglutarate | C00026 | Energy | Krebs_cycle |
| M37058 | succinylcarnitine | NA | Energy | Krebs_cycle |
| M11438 | phosphate | C00009 | Energy | Oxidative_phosphorylation |
| M15488 | acetyl phosphate | C00227 | Energy | Oxidative_phosphorylation |
| M2078 | pyrophosphate | C00013 | Energy | Oxidative_phosphorylation |
| M1114 | deoxycholate | C04483 | Lipid | Bile_acid_metabolism |
| M15500 | carnitine | C00487 | Lipid | Carnitine_metabolism |
| M22189 | palmitoylcarnitine | C02990 | Lipid | Carnitine_metabolism |
| M32198 | acetylcarnitine | C02571 | Lipid | Carnitine_metabolism |
| M32328 | hexanoylcarnitine | C01585 | Lipid | Carnitine_metabolism |
| M32654 | 3-dehydrocarnitine | C02636 | Lipid | Carnitine_metabolism |
| M34409 | stearoylcarnitine | NA | Lipid | Carnitine_metabolism |
| M35160 | oleoylcarnitine | NA | Lipid | Carnitine_metabolism |
| M36747 | deoxycarnitine | C01181 | Lipid | Carnitine_metabolism |
| M7746 | prostaglandin E2 | C00584 | Lipid | Eicosanoid |
| M18467 | eicosapentaenoate | C06428 | Lipid | Essential_fatty_acid |
| M19323 | docosahexaenoate | C06429 | Lipid | Essential_fatty_acid |
| M32504 | docosapentaenoate | C16513 | Lipid | Essential_fatty_acid |
| M34035 | linolenate [alpha or gamma (18:3n3 or 6)] | C06427 | Lipid | Essential_fatty_acid |
| M35718 | dihomo-linolenate | C03242 | Lipid | Essential_fatty_acid |
| M37478 | docosapentaenoate | C06429 | Lipid | Essential_fatty_acid |
| M31850 | butyrylglycine | NA | Lipid | Fatty_acid,_beta-oxidation |
| M35436 | hexanoylglycine | NA | Lipid | Fatty_acid,_beta-oxidation |
| M18362 | azelate | C08261 | Lipid | Fatty_acid,_dicarboxylate |
| M31787 | 3-carboxy-4-methyl-5-propyl-2-furanpropanoate | NA | Lipid | Fatty_acid,_dicarboxylate |
| M32398 | sebacate | C08277 | Lipid | Fatty_acid,_dicarboxylate |
| M37253 | 2-hydroxyglutarate | C02630 | Lipid | Fatty_acid,_dicarboxylate |
| M36802 | n-Butyl Oleate | NA | Lipid | Fatty_acid,_ester |
| M17945 | 2-hydroxystearate | C03045 | Lipid | Fatty_acid,_monohydroxy |
| M34585 | 4-hydroxybutyrate | C00989 | Lipid | Fatty_acid,_monohydroxy |
| M35675 | 2-hydroxypalmitate | NA | Lipid | Fatty_acid,_monohydroxy |
| M37752 | 13-HODE 9-HODE | NA | Lipid | Fatty_acid,_monohydroxy |
| M34406 | valerylcarnitine | NA | Lipid | Fatty_acid_metabolism |
| M32412 | butyrylcarnitine | C02862 | Lipid | Fatty_acid_metabolism_(also_BCAA_me tabolism) |
| M32452 | propionylcarnitine | C03017 | Lipid | Fatty_acid_metabolism_(also_BCAA_me tabolism) |
| M12102 | phosphoethanolamine | C00346 | Lipid | Glycerolipid_metabolism |
| M1497 | ethanolamine | C00189 | Lipid | Glycerolipid_metabolism |
| M15122 | glycerol | C00116 | Lipid | Glycerolipid_metabolism |
| M15365 | glycerol 3-phosphate | C00093 | Lipid | Glycerolipid_metabolism |
| M15506 | choline | C00114 | Lipid | Glycerolipid_metabolism |
| M15990 | glycerophosphorylcholine | C00670 | Lipid | Glycerolipid_metabolism |
| M1600 | phosphoethanolamine | C00346 | Lipid | Glycerolipid_metabolism |
| M34396 | choline phosphate | C00588 | Lipid | Glycerolipid_metabolism |
| M34418 | cytidine 5'-diphosphocholine | C00307 | Lipid | Glycerolipid_metabolism |
| M37455 | glycerophosphoethanolamine | C01233 | Lipid | Glycerolipid_metabolism |
| M1481 | inositol 1-phosphate | C01177 | Lipid | Inositol_metabolism |
| M19934 | myo-inositol | C00137 | Lipid | Inositol_metabolism |
| M32379 | scyllo-inositol | C06153 | Lipid | Inositol_metabolism |
| M542 | 3-hydroxybutyrate | C01089 | Lipid | Ketone_bodies |
| M1105 | linoleate | C01595 | Lipid | Long_chain_fatty_acid |
| M1110 | arachidonate | C00219 | Lipid | Long_chain_fatty_acid |
| M1121 | margarate | NA | Lipid | Long_chain_fatty_acid |
| M1336 | palmitate | C00249 | Lipid | Long_chain_fatty_acid |
| M1356 | nonadecanoate | C16535 | Lipid | Long_chain_fatty_acid |
| M1358 | stearate | C01530 | Lipid | Long_chain_fatty_acid |
| M1359 | oleate | C00712 | Lipid | Long_chain_fatty_acid |
| M1361 | pentadecanoate | C16537 | Lipid | Long_chain_fatty_acid |
| M1365 | myristate | C06424 | Lipid | Long_chain_fatty_acid |
| M17805 | dihomo-linoleate | C16525 | Lipid | Long_chain_fatty_acid |
| M32415 | docosadienoate | C16533 | Lipid | Long_chain_fatty_acid |
| M32417 | docosatrienoate | C16534 | Lipid | Long_chain_fatty_acid |
| M32418 | myristoleate | C08322 | Lipid | Long_chain_fatty_acid |
| M32501 | dihomo-alpha-linolenate | NA | Lipid | Long_chain_fatty_acid |
| M32980 | adrenate | C16527 | Lipid | Long_chain_fatty_acid |
| M33447 | palmitoleate | C08362 | Lipid | Long_chain_fatty_acid |
| M33587 | eicosenoate | NA | Lipid | Long_chain_fatty_acid |
| M33970 | cis-vaccenate | C08367 | Lipid | Long_chain_fatty_acid |
| M33971 | 10-heptadecenoate | NA | Lipid | Long_chain_fatty_acid |
| M33972 | 10-nonadecenoate | NA | Lipid | Long_chain_fatty_acid |
| M35174 | mead acid | NA | Lipid | Long_chain_fatty_acid |
| M19260 | 1-oleoylglycerophos phoserine | NA | Lipid | Lysolipid |
| M19324 | 1-stearoylglyceropho sphoinositol | NA | Lipid | Lysolipid |
| M32635 | 1-linoleoylglyceroph osphoethanolamine | NA | Lipid | Lysolipid |
| M33871 | 1-eicosadienoylglyce rophosphocholine | NA | Lipid | Lysolipid |
| M33955 | 1-palmitoylglycerop hosphocholine | C04102 | Lipid | Lysolipid |
| M33960 | 1-oleoylglycerophos phocholine | C03916 | Lipid | Lysolipid |
| M33961 | 1-stearoylglyceropho sphocholine | NA | Lipid | Lysolipid |
| M34214 | 1-arachidonoylglycer ophosphoinositol | NA | Lipid | Lysolipid |
| M34258 | 2-docosahexaenoylgl ycerophosphoetha nolamine | NA | Lipid | Lysolipid |
| M34416 | 1-stearoylglyceropho sphoethanolamine | NA | Lipid | Lysolipid |
| M34419 | 1-linoleoylglyceroph osphocholine | C04100 | Lipid | Lysolipid |
| M34656 | 2-arachidonoylglycer ophosphoethanola mine | NA | Lipid | Lysolipid |
| M34875 | 2-docosapentaenoylg lycerophosphoetha nolamine | NA | Lipid | Lysolipid |
| M35186 | 1-arachidonoylglycer ophosphoethanola mine | NA | Lipid | Lysolipid |
| M35253 | 2-palmitoylglycerop hosphocholine | NA | Lipid | Lysolipid |
| M35254 | 2-oleoylglycerophos phocholine | NA | Lipid | Lysolipid |
| M35256 | 2-arachidonoylglycer ophosphocholine | NA | Lipid | Lysolipid |
| M35257 | 2-linoleoylglyceroph osphocholine | NA | Lipid | Lysolipid |
| M35305 | 1-palmitoylglycerop hosphoinositol | NA | Lipid | Lysolipid |
| M35626 | 1-myristoylglycerop hosphocholine | NA | Lipid | Lysolipid |
| M35628 | 1-oleoylglycerophos phoethanolamine | NA | Lipid | Lysolipid |
| M35631 | 1-palmitoylglycerop hosphoethanolamine | NA | Lipid | Lysolipid |
| M35687 | 2-oleoylglycerophos phoethanolamine | NA | Lipid | Lysolipid |
| M35688 | 2-palmitoylglycerop hosphoethanolamine | NA | Lipid | Lysolipid |
| M36602 | 1-oleoylglycerophos phoinositol | NA | Lipid | Lysolipid |
| M12035 | pelargonate | C01601 | Lipid | Medium_chain_fatty_acid |
| M12067 | undecanoate | NA | Lipid | Medium_chain_fatty_acid |
| M1642 | caprate | C01571 | Lipid | Medium_chain_fatty_acid |
| M1644 | heptanoate | NA | Lipid | Medium_chain_fatty_acid |
| M1645 | laurate | C02679 | Lipid | Medium_chain_fatty_acid |
| M33968 | 5-dodecenoate | NA | Lipid | Medium_chain_fatty_acid |
| M21127 | 1-palmitoylglycerol | NA | Lipid | Monoacylglycerol |
| M21188 | 1-stearoylglycerol | D01947 | Lipid | Monoacylglycerol |
| M27447 | 1 -linoleoylglycerol | NA | Lipid | Monoacylglycerol |
| M33419 | 2-palmitoylglycerol | NA | Lipid | Monoacylglycerol |
| M34397 | 1-arachidonylglycero 1 | C13857 | Lipid | Monoacylglycerol |
| M18790 | acetylcholine | C01996 | Lipid | Neurotransmitter |
| M17747 | sphingosine | C00319 | Lipid | Sphingolipid |
| M19503 | stearoyl sphingomyelin | C00550 | Lipid | Sphingolipid |
| M37506 | palmitoyl sphingomyelin | NA | Lipid | Sphingolipid |
| M32425 | dehydroisoandrost erone sulfate | C04555 | Lipid | Sterol/Steroid |
| M33997 | campesterol | C01789 | Lipid | Sterol/Steroid |
| M35092 | 7-beta-hydroxycholesterol | C03594 | Lipid | Sterol/Steroid |
| M36776 | 7-alpha-hydroxy-3-oxo-4-cholestenoate | C17337 | Lipid | Sterol/Steroid |
| M37202 | 4-androsten-3beta,17beta-diol disulfate 1 | NA | Lipid | Sterol/Steroid |
| M63 | cholesterol | C00187 | Lipid | Sterol/Steroid |
| M37419 | 1-heptadecanoylglyc erophosphoethanol amine | NA | No_Super_Pat hway | No_Pathway |
| M37070 | methylphosphate | NA | Nucleotide | Purine_and_pyrimidine_metabolism |
| M1123 | inosine | C00294 | Nucleotide | Purine_metabolism,_(hypo)xanthine/inosine_containing |
| M15076 | 2'-deoxyinosine | C05512 | Nucleotide | Purine_metabolism,_(hypo)xanthine/inosine_containing |
| M15136 | xanthosine | C01762 | Nucleotide | Purine_metabolism,_(hypo)xanthine/inosine_containing |
| M3127 | hypoxanthine | C00262 | Nucleotide | Purine_metabolism,_(hypo)xanthine/inosine_containing |
| M3147 | xanthine | C00385 | Nucleotide | Purine_metabolism,_(hypo)xanthine/inosine_containing |
| M15650 | N1-methyladenosine | C02494 | Nucleotide | Purine_metabolism,_adenine_containing |
| M18360 | adenylosuccinate | C03794 | Nucleotide | Purine_metabolism,_adenine_containing |
| M3108 | adenosine 5'-diphosphate | C00008 | Nucleotide | Purine_metabolism,_adenine_containing |
| M3234 2 | adenosine 5'-monophosphate | C00020 | Nucleotide | Purine_metabolism,_adenine_containing |
| M3344 9 | adenosine 5'-triphosphate | C00002 | Nucleotide | Purine_metabolism,_adenine_containing |
| M3514 2 | adenosine 3'-monophosphate | C01367 | Nucleotide | Purine_metabolism,_adenine_containing |
| M3681 5 | adenosine 2'-monophosphate | C00946 | Nucleotide | Purine_metabolism,_adenine_containing |
| M554 | adenine | C00147 | Nucleotide | Purine_metabolism,_adenine_containing |
| M555 | adenosine | C00212 | Nucleotide | Purine_metabolism,_adenine_containing |
| M1573 | guanosine | C00387 | Nucleotide | Purine_metabolism,_guanine_containing |
| M2849 | guanosine 5'-monophosphate | C00144 | Nucleotide | Purine_metabolism,_guanine_containing |
| M3160 9 | N1-methylguanosine | NA | Nucleotide | Purine_metabolism,_guanine_containing |
| M3235 2 | guanine | C00242 | Nucleotide | Purine_metabolism,_guanine_containing |
| M418 | guanine | C00242 | Nucleotide | Purine_metabolism,_guanine_containing |
| M1107 | allantoin | C02350 | Nucleotide | Purine_metabolism,_urate_metabolism |
| M1604 | urate | C00366 | Nucleotide | Purine_metabolism,_urate_metabolism |
| M3746 5 | cytosine 2' 3' cyclic monophosphate | NA | Nucleotide | Pyrimidine metabolism (cytidine-containing) |
| M2372 | cytidine 5'-monophosphate | C00055 | Nucleotide | Pyrimidine_metabolism,_cytidine_containing |
| M2959 | cytidine-3'-monophosphate | C05822 | Nucleotide | Pyrimidine_metabolism,_cytidine_containing |
| M514 | cytidine | C00475 | Nucleotide | Pyrimidine_metabolism,_cytidine_containing |
| M1505 | orotate | C00295 | Nucleotide | Pyrimidine_metabolism,_orotate_containing |
| M1566 | 3-aminoisobutyrate | C05145 | Nucleotide | Pyrimidine_metabolism,_thymine_containing;_Valine,_leucine_and_isoleucine_metabolism/ |
| M1559 | 5,6-dihydrouracil | C00429 | Nucleotide | Pyrimidine_metabolism,_uracil_containing |
| M2856 | uridine 5'-monophosphate | C00105 | Nucleotide | Pyrimidine_metabolism,_uracil_containing |
| M33442 | pseudouridine | C02067 | Nucleotide | Pyrimidine_metabolism,_uracil_containing |
| M37137 | uridine-2',3'-cyclic monophosphate | C02355 | Nucleotide | Pyrimidine_metabolism,_uracil_containing |
| M5345 | uridine 5'-diphosphate | C00015 | Nucleotide | Pyrimidine_metabolism,_uracil_containing |
| M605 | uracil | C00106 | Nucleotide | Pyrimidine_metabolism,_uracil_containing |
| M606 | uridine | C00299 | Nucleotide | Pyrimidine_metabolism,_uracil_containing |
| M22171 | glycylproline | NA | Peptide | Dipeptide |
| M22175 | aspartylphenylalan ine | NA | Peptide | Dipeptide |
| M31530 | threonylphenylalan ine | NA | Peptide | Dipeptide |
| M32393 | glutamylvaline | NA | Peptide | Dipeptide |
| M32394 | pyroglutamylvalin e | NA | Peptide | Dipeptide |
| M33958 | glycyltyrosine | NA | Peptide | Dipeptide |
| M34398 | glycylleucine | C02155 | Peptide | Dipeptide |
| M35637 | cysteinylglycine | C01419 | Peptide | Dipeptide |
| M36659 | glycylisoleucine | NA | Peptide | Dipeptide |
| M36756 | leucylleucine | C11332 | Peptide | Dipeptide |
| M36761 | isoleucylisoleucine | NA | Peptide | Dipeptide |
| M37093 | alanylleucine | NA | Peptide | Dipeptide |
| M37098 | alanyltyrosine | NA | Peptide | Dipeptide |
| M15747 | anserine | C01262 | Peptide | Dipeptide_derivative |
| M1633 | homocarnosine | C00884 | Peptide | Dipeptide_derivative |
| M1768 | carnosine | C00386 | Peptide | Dipeptide_derivative |
| M18369 | gamma-glutamylleucine | NA | Peptide | gamma-glutamyl |
| M2730 | gamma-glutamylglutamine | NA | Peptide | gamma-glutamyl |
| M36738 | gamma-glutamylglutamate | NA | Peptide | gamma-glutamyl |
| M37063 | gamma-glutamylalanine | NA | Peptide | gamma-glutamyl |
| M37539 | gamma-glutamylmethionin e | NA | Peptide | gamma-glutamyl |
| M34456 | gamma-glutamylisoleucine | NA | Peptide | g-glutamyl |
| M15753 | hippurate | C01586 | Xenobiotics | Benzoate_metabolism |
| M18281 | 2-hydroxyhippurate | C07588 | Xenobiotics | Benzoate_metabolism |
| M35320 | catechol sulfate | C00090 | Xenobiotics | Benzoate_metabolism |
| M36098 | 4-vinylphenol sulfate | C05627 | Xenobiotics | Benzoate_metabolism |
| M36099 | 4-ethylphenylsulfate | NA | Xenobiotics | Benzoate_metabolism |
| M1554 | 2-ethylhexanoate | NA | Xenobiotics | Chemical |
| M20714 | methyl-alpha-glucopyranoside | C03619 | Xenobiotics | Chemical |
| M27728 | glycerol 2-phosphate | C02979 | Xenobiotics | Chemical |
| M27743 | triethyleneglycol | NA | Xenobiotics | Chemical |
| M12032 | 4-acetamidophenol | C06804 | Xenobiotics | Drug |
| M33080 | N-ethylglycinexylidi de | C16561 | Xenobiotics | Drug |
| M33173 | 2-hydroxyacetamino phen sulfate | NA | Xenobiotics | Drug |
| M33178 | 2-methoxyacetamino phen sulfate | NA | Xenobiotics | Drug |
| M33423 | p-acetamidophenylglucuronide | NA | Xenobiotics | Drug |
| M3434 6 | desmethylnaproxen sulfate | NA | Xenobiotics | Drug |
| M3436 5 | 3-(cystein-S-yl)acetaminophen | NA | Xenobiotics | Drug |
| M3566 1 | lidocaine | D00358 | Xenobiotics | Drug |
| M3746 8 | penicillin G | C05551 | Xenobiotics | Drug |
| M3747 5 | 4-acetaminophen sulfate | C06804 | Xenobiotics | Drug |
| M3863 7 | cinnamoylglycine | NA | Xenobiotics | Food component (plant) |
| M1833 5 | quinate | C00296 | Xenobiotics | Food_component/Plant |
| M3244 8 | genistein | C06563 | Xenobiotics | Food_component/Plant |
| M3245 3 | daidzein | C10208 | Xenobiotics | Food_component/Plant |
| M3393 5 | piperine | C03882 | Xenobiotics | Food_component/Plant |
| M3745 9 | ergothioneine | C05570 | Xenobiotics | Food_component/Plant |
| M2069 9 | erythritol | C00503 | Xenobiotics | Sugar,_sugar_substitute,_starch |
| M1825 4 | paraxanthine | C13747 | Xenobiotics | Xanthme_metabolism |
| M1839 2 | theobromine | C07480 | Xenobiotics | Xanthme_metabolism |
| M3440 0 | 1,7-dimethylurate | C16356 | Xenobiotics | Xanthme_metabolism |
| M569 | caffeine | C07481 | Xenobiotics | Xanthme_metabolism |

Table 2: Metabolite concentration fold changes and p-values for RWPE-AKT1 cells, MPAKT mice and phosphoAKT1-high/MYC-low tumors compared to RWPE-MYC cells, Lo-MYC mice and MYC-high/phosphoAKT1-low tumors, respectively.

**Table 2: RWPE cells**

| **Metabolite** | **KEG G ID** | **Statisti c** | **Pvalue** | **BH** | **Fold Change (RWPE-AKT1/RWPE-MYC)** |
|---|---|---|---|---|---|
| fructose_1,6-bisphosphate | C05378 | 119.8676864 | 0.009998 | 0.020353072 | 4.738624407 |
| glucose | C00267 | 20.65226182 | 0.009998 | 0.020353072 | 51.51377553 |
| kynurenine | C00328 | 15.70155617 | 0.009998 | 0.020353072 | 3.045622149 |
| hypoxanthine | C00262 | 13.70619099 | 0.009998 | 0.020353072 | 2.286526654 |
| 1-palmitoylglycerophosphoch oline | C04102 | 10.4032463 | 0.009998 | 0.020353072 | 5.157499278 |
| ribulose_5-phosphate | C00117.2 | 9.265638432 | 0.009998 | 0.020353072 | 3.76062704 |
| arachidonate | C00219 | 9.18187886 | 0.009998 | 0.020353072 | 2.097490562 |
| docosahexaenoate | C06429 | 9.07763373 | 0.009998 | 0.020353072 | 2.48420095 |
| ribose_5-phosphate | C00117 | 8.418309746 | 0.009998 | 0.020353072 | 9.618227338 |
| N-acetylneuraminate | C00270 | 8.277850689 | 0.009998 | 0.020353072 | 2.462617276 |
| palmitoylcarnitine | C02990 | 7.163347714 | 0.009998 | 0.020353072 | 4.155427482 |
| docosapentaenoate | C16513 | 6.356127711 | 0.009998 | 0.020353072 | 2.024159333 |
| lactate | C00186 | 6.086634561 | 0.009998 | 0.020353072 | 1.979031832 |
| threonine | C00188 | 5.424535734 | 0.009998 | 0.020353072 | 1.20625138 |
| sphingosine | C00319 | 4.927267217 | 0.009998 | 0.020353072 | 3.942420982 |
| malate | C00149 | 4.84868646 | 0.009998 | 0.020353072 | 1.180212973 |
| putrescine | C00134 | 4.363517765 | 0.009998 | 0.020353072 | 1.716300482 |
| carnitine | C00487 | 4.149148079 | 0.016996601 | 0.032840889 | 1.181253854 |
| serine | C00065 | 4.145286144 | 0.009998 | 0.020353072 | 1.286416228 |
| glutamine | C00064 | 4.145166486 | 0.009998 | 0.020353072 | 1.45086936 |
| tryptophan | C00078 | 4.120933202 | 0.009998 | 0.020353072 | 1.207529259 |
| isoleucine | C00407 | 4.01686246 | 0.018196361 | 0.033457825 | 1.291948938 |
| histidine | C00135 | 3.745126323 | 0.009998 | 0.020353072 | 1.448776697 |
| leucine | C00123 | 3.59956152 | 0.009998 | 0.020353072 | 1.325546255 |
| UDP-glucuronate | C00167 | 3.543974822 | 0.016196761 | 0.032393521 | 1.33853376 |
| phenylalanine | C00079 | 3.404997548 | 0.009998 | 0.020353072 | 1.248853872 |
| guanine | C00242 | 3.315805992 | 0.009998 | 0.020353072 | 2.620464264 |
| tyrosine | C00082 | 3.291334315 | 0.009998 | 0.020353072 | 1.289289976 |
| proline | C00148 | 3.26925609 | 0.009998 | 0.020353072 | 1.594939743 |
| oleate | C00712 | 3.260383573 | 0.031793641 | 0.050973139 | 1.191404393 |
| stearate | C01530 | 3.037917062 | 0.028194361 | 0.046581988 | 1.140029894 |
| asparagine | C00152 | 2.969467579 | 0.018196361 | 0.033457825 | 1.270943015 |
| uracil | C00106 | 2.962293391 | 0.025394921 | 0.043863954 | 1.32443449 |
| nicotinamide_adenine_dinu cleotide reduced | C00004 | 2.84879095 | 0.032193561 | 0.050973139 | 1.380002307 |
| 1-oleoylglycerophosphocholi ne | C03916 | 2.674874262 | 0.009998 | 0.020353072 | 2.483788951 |
| ornithine | C00077 | 2.561400158 | 0.060387922 | 0.091789642 | 1.253497526 |
| gulono-1,4-lactone | C01040 | 2.218229087 | 0.047990402 | 0.07393116 | 1.552385728 |
| valine | C00183 | 2.04152656 | 0.076984603 | 0.112515958 | 1.191354427 |
| uridine | C00299 | 1.623228155 | 0.134573085 | 0.184835322 | 1.33283102 |
| inosine | C00294 | 1.605454242 | 0.155968806 | 0.206749348 | 1.340389058 |
| lysine | C00047 | 1.584268139 | 0.141171766 | 0.19094534 | 1.151833443 |
| choline | C00114 | 1.474667131 | 0.203759248 | 0.263960844 | 1.345176677 |
| adenosine_5'-triphosphate | C00002 | 1.429848319 | 0.215156969 | 0.275594319 | 1.257939688 |
| acetylcarnitine | C02571 | 1.198386024 | 0.24715057 | 0.306251793 | 1.205609184 |
| eicosapentaenoate | C06428 | 1.00058253 | 0.322135573 | 0.394875864 | 1.294857331 |
| 3-phosphoglycerate | C00597 | 0.902580834 | 0.398520296 | 0.468364059 | 1.306676106 |
| propionylcarnitine | C03017 | 0.839896929 | 0.396920616 | 0.468364059 | 1.091033094 |
| beta-alanine | C00099 | 0.596360195 | 0.564487103 | 0.625214763 | 1.100163038 |
| methionine | C00073 | 0.585286137 | 0.638072386 | 0.6994255 | 1.048323339 |
| betaine | C00719 | 0.487208252 | 0.659468106 | 0.715993944 | 1.085759788 |
| alanine | C00041 | 0.458235877 | 0.797640472 | 0.82664558 | 1.014615243 |
| glutathione,_oxidized | C00127 | 0.456820572 | 0.698860228 | 0.737685796 | 1.026015667 |
| adrenate | C16527 | 0.119820035 | 0.99320136 | 0.99320136 | 1.081585609 |
| UDP-N-acetylglucosamine | C00043 | 0.097138409 | 0.912417516 | 0.928710686 | 1.020584246 |
| glycine | C00037 | 0.082512239 | 0.922415517 | 0.930578486 | 1.004706923 |
| nicotinamide | C00153 | - 0.112901051 | 0.896620676 | 0.920853667 | 1.02609055 |
| cholesterol | C00187 | - 0.319104758 | 0.769646071 | 0.804950936 | 1.020336263 |
| glutamate | C00025 | - 0.374926118 | 0.685462907 | 0.737195957 | 1.012534599 |
| urea | C00086 | - 0.427064095 | 0.696860628 | 0.737685796 | 1.082956245 |
| gamma-amino butyrate | C00334 | - 0.590636165 | 0.564887023 | 0.625214763 | 1.078318168 |
| 5-oxoproline | C01879 | - 0.651258364 | 0.518696261 | 0.597286603 | 1.101709722 |
| palmitate | C00249 | - 0.687287197 | 0.5034993 | 0.585703268 | 1.065072979 |
| UDP-glucose | C00029 | - 0.829664305 | 0.548490302 | 0.619088064 | 1.25586995 |
| S-adenosylhomocysteine | C00021 | - 0.942596354 | 0.363727255 | 0.436472705 | 1.060712256 |
| ascorbate | C00072 | - 0.951130088 | 0.530693861 | 0.604991002 | 1.196504701 |
| pentadecanoate | C16537 | - 0.977207694 | 0.350729854 | 0.425353227 | 1.560279788 |
| guanosine_5'-_monophosphate | C00144 | - 1.291713384 | 0.226154769 | 0.283314766 | 1.298414486 |
| caprate | C01571 | - 1.322571824 | 0.193561288 | 0.253632032 | 1.12199237 |
| 5-methylthioadenosine | C00170 | - 1.3813 70394 | 0.220755849 | 0.279624075 | 1.106583491 |
| adenosine_5'-diphosphate | C00008 | - 1.5052 98233 | 0.00959808 | 0.020353072 | 1.596442366 |
| fructose-6-phosphate | C05345 | - 1.6478 14474 | 0.142371526 | 0.19094534 | 1.614290762 |
| cytidine_5'-diphosphocholine | C00307 | - 1.6484 25787 | 0.101179764 | 0.142401149 | 1.118772265 |
| guanosine | C00387 | - 1.7932 86389 | 0.098780244 | 0.140761848 | 1.462171557 |
| inositol_1-phosphate | C01177 | - 1.7951 27438 | 0.119176165 | 0.165683936 | 1.527744384 |
| adenine | C00147 | - 1.7995 49967 | 0.073185363 | 0.108352356 | 1.246252244 |
| pelargonate | C01601 | - 1.8394 28678 | 0.087382523 | 0.1260963 | 1.24618128 |
| hypotaurine | C00519 | - 2.0727 58483 | 0.064187163 | 0.096280744 | 1.42070142 |
| cysteine | C00097 | - 2.2224 96709 | 0.031993601 | 0.050973139 | 2.267677642 |
| adenylosuccinate | C03794 | - 2.2873 17591 | 2.00E-04 | 9.12E-04 | 10.83302465 |
| linoleate | C01595 | - 2.3455 38274 | 0.045990802 | 0.071821252 | 1.19157127 |
| arginine | C00062 | - 2.3557 86576 | 2.00E-04 | 9.12E-04 | 1.498777516 |
| glycerol_3-phosphate | C00093 | - 2.3626 1644 | 0.026194761 | 0.043914746 | 1.512845547 |
| scyllo- inositol | C06153 | - 2.4444 98861 | 0.017796441 | 0.033457825 | 1.570691312 |
| palmitoleate | C08362 | - 2.469099284 | 0.023195361 | 0.041972558 | 1.348678628 |
| pyrophosphate | C00013 | - 2.499282383 | 2.00E-04 | 9.12E-04 | 22.19112918 |
| spermidine | C00315 | - 2.547175419 | 0.024195161 | 0.04309763 | 2.930265588 |
| creatine | C00300 | - 2.807552448 | 0.025194961 | 0.043863954 | 1.511098738 |
| glutathione,_reduced | C00051 | - 2.833137036 | 0.00879824 | 0.020353072 | 1.274109649 |
| laurate | C02679 | - 2.94364984 | 2.00E-04 | 9.12E-04 | 1.467115317 |
| acetylphosphate | C00227 | - 3.048003937 | 2.00E-04 | 9.12E-04 | 1.224222457 |
| adenosine | C00212 | - 3.176824097 | 0.025994801 | 0.043914746 | 1.301957807 |
| nicotinamide_adenine_dinu cleotide_phosphate | C00005 | - 3.261185332 | 0.016996601 | 0.032840889 | 1.631472907 |
| myristoleate | C08322 | - 3.297885963 | 2.00E-04 | 9.12E-04 | 1.709976347 |
| glucose-6-phosphate | C00668 | - 3.660174305 | 2.00E-04 | 9.12E-04 | 2.345491734 |
| citrate | C00158 | - 3.834436092 | 0.00859828 | 0.020353072 | 1.236763118 |
| cytidine_5'-monophosphate | C00055 | - 4.096483485 | 2.00E-04 | 9.12E-04 | 1.811603131 |
| myristate | C06424 | - 4.20707113 | 0.00679864 | 0.020353072 | 1.489863819 |
| myo-inositol | C00137 | - 4.259788648 | 2.00E-04 | 9.12E-04 | 1.370642583 |
| fumarate | C00122 | - 4.268976999 | 2.00E-04 | 9.12E-04 | 1.510804551 |
| uridine_5'-monophosphate | C00105 | - 4.310103285 | 2.00E-04 | 9.12E-04 | 1.922261646 |
| spermine | C00750 | - 4.526787877 | 2.00E-04 | 9.12E-04 | 3.934229574 |
| glycerophosphorylcholine | C00670 | - 4.609315684 | 2.00E-04 | 9.12E-04 | 7.148421913 |
| 1 -methylnicotinamide | C02918 | - 5.093201852 | 2.00E-04 | 9.12E-04 | 1.259641237 |
| butyrylcarnitine | C02862 | - 5.435624344 | 2.00E-04 | 9.12E-04 | 1.544844116 |
| fructose | C00095 | - 6.698792894 | 2.00E-04 | 9.12E-04 | 2.160039345 |
| choline_phosphate | C00588 | - 8.453823521 | 2.00E-04 | 9.12E-04 | 1.810762669 |
| adenosine_5'-monophosphate | C00020 | - 8.969613192 | 2.00E-04 | 9.12E-04 | 2.021279539 |
| S-lactoylglutathione | C03451 | - 10.3263094 | 2.00E-04 | 9.12E-04 | 3.238772345 |
| aspartate | C00049 | - 10.42113385 | 2.00E-04 | 9.12E-04 | 1.672765754 |
| pantothenate | C00864 | - 10.55863989 | 2.00E-04 | 9.12E-04 | 2.38346229 |
| nicotinamide_adenine_dinucleotide | C00003 | - 10.70673596 | 2.00E-04 | 9.12E-04 | 2.061232441 |
| phosphate | C00009 | - 10.87211685 | 2.00E-04 | 9.12E-04 | 1.939572376 |
| glycerol | C00116 | - 11.18675245 | 2.00E-04 | 9.12E-04 | 1.612824216 |
| flavin_adenine_dinucleotide | C00016 | - 15.61444522 | 2.00E-04 | 9.12E-04 | 2.813638126 |

**Table 2: Mice**

| **Metabolite** | **KEG GID** | **Statistic** | **Pvalue** | **BH** | **Fold Change (MPAKT/Lo-MYC)** |
|---|---|---|---|---|---|
| cholesterol | C00187 | 5.731030747 | 0.00219956 | 0.014957009 | 1.314480145 |
| orotate | C00295 | 4.846016945 | 0.00219956 | 0.014957009 | 5.324861974 |
| isoleucine | C00407 | 4.802230236 | 0.00219956 | 0.014957009 | 1.78958409 |
| acetylcarnitine | C02571 | 4.38451587 | 0.00219956 | 0.014957009 | 1.702913689 |
| valine | C00183 | 4.070684752 | 0.00379924 | 0.022465072 | 1.381314289 |
| propionylcarnitine | C03017 | 4.024578503 | 0.00419916 | 0.022843431 | 1.772345283 |
| cytidine_5'-monophosphate | C00055 | 3.928335838 | 0.00219956 | 0.014957009 | 1.662146089 |
| thiamin | C00378 | 3.454652887 | 0.00779844 | 0.030216179 | 1.598836673 |
| malate | C00149 | 3.222867661 | 0.0079984 | 0.030216179 | 1.426765535 |
| lactate | C00186 | 3.172803844 | 0.0069986 | 0.029744051 | 1.803881231 |
| glycine | C00037 | 3.153068661 | 0.018796241 | 0.058097471 | 1.31995762 |
| serine | C00065 | 3.057757208 | 0.016196761 | 0.053094143 | 1.552959004 |
| riboflavin | C00255 | 3.019909796 | 0.014397121 | 0.049630074 | 1.64953552 |
| leucine | C00123 | 2.931057916 | 0.00919816 | 0.033809454 | 1.261816088 |
| scyllo-inositol | C06153 | 2.792377804 | 0.00219956 | 0.014957009 | 3.705486601 |
| mannose | C00159 | 2.752696427 | 0.00219956 | 0.014957009 | 1.959596598 |
| citrate | C00158 | 2.734987498 | 0.030993801 | 0.08781577 | 1.527179249 |
| tryptophan | C00078 | 2.583459194 | 0.00659868 | 0.028949049 | 1.571086987 |
| fructose-6-phosphate | C05345 | 2.580081431 | 0.026594681 | 0.077533429 | 2.491828548 |
| sorbitol | C00794 | 2.443734936 | 0.01159768 | 0.041507488 | 8.880967365 |
| butyrylcarnitine | C02862 | 2.386996272 | 0.026394721 | 0.077533429 | 2.60845214 |
| choline | C00114 | 2.268940153 | 0.068186363 | 0.165595452 | 1.257780595 |
| uridine-2',3'-cyclic_monophosphate | C02355 | 2.172778942 | 0.0079984 | 0.030216179 | 3.159365678 |
| ascorbate | C00072 | 2.146212519 | 0.048790242 | 0.127605248 | 7.139154413 |
| ribulose_5-phosphate | C00199 | 2.132110125 | 0.034593081 | 0.094093181 | 2.065713503 |
| aspartate | C00049 | 2.086957772 | 0.014597081 | 0.049630074 | 1.69706794 |
| phenylalanine | C00079 | 2.02154555 | 0.054189162 | 0.136476408 | 1.319360097 |
| spermidine | C00315 | 1.885729393 | 0.097180564 | 0.207358528 | 1.869906627 |
| prostaglandin.E2 | C00584 | 1.861764058 | 0.105978804 | 0.215121155 | 3.173288966 |
| glucose-6-phosphate | C00668 | 1.838232381 | 0.091381724 | 0.203736302 | 1.818559261 |
| glycerol | C00116 | 1.744469954 | 0.095380924 | 0.207358528 | 1.378443437 |
| N-acetylglucosamine | C03878 | 1.744364762 | 0.111377724 | 0.216066376 | 5.212405739 |
| adenosine_2'-monophosphate | C00946 | 1.730163833 | 0.185562887 | 0.286779008 | 2.223593936 |
| fructose | C00095 | 1.708754 | 0.00219956 | 0.014957009 | 2.546501911 |
| lysine | C00047 | 1.689904121 | 0.115976805 | 0.216066376 | 1.800193662 |
| glycerol_2-phosphate | C02979 | 1.674246236 | 0.072785443 | 0.170669314 | 1.795693849 |
| tyrosine | C00082 | 1.650959636 | 0.113977205 | 0.216066376 | 1.166769141 |
| mannose-6-phosphate | C00275 | 1.607439929 | 0.132373525 | 0.23687894 | 1.371543598 |
| threonine | C00188 | 1.588042323 | 0.152369526 | 0.254368638 | 1.326419463 |
| ergothioneine | C05570 | 1.566794854 | 0.146370726 | 0.250529894 | 2.047100977 |
| hypotaurine | C00519 | 1.563831201 | 0.153369326 | 0.254368638 | 1.663143775 |
| phenylacetylglycine | C05598 | 1.526261401 | 0.211757648 | 0.299140172 | 2.122666545 |
| phenol_sulfate | C02180 | 1.458425372 | 0.184163167 | 0.286779008 | 2.08333105 |
| hypoxanthine | C00262 | 1.403555145 | 0.184763047 | 0.286779008 | 1.187168862 |
| cis-vaccenate | C08367 | 1.388921857 | 0.24015197 | 0.329905736 | 1.602106655 |
| adenosine_5'-monophosphate | C00301 | 1.376700664 | 0.206958608 | 0.299140172 | 1.737664047 |
| ribose_5-phosphate | C00117 | 1.373386856 | 0.201959608 | 0.299140172 | 1.702070354 |
| glycerol_3-phosphate | C00093 | 1.341635345 | 0.204359128 | 0.299140172 | 1.315510733 |
| creatine | C00300 | 1.290483341 | 0.230153969 | 0.319397345 | 1.179965058 |
| methionine | C00073 | 1.227369038 | 0.25634873 | 0.348634273 | 1.225165514 |
| cystine | C00491 | 1.11255097 | 0.268346331 | 0.361337633 | 1.656942531 |
| erythritol | C00503 | 1.109359211 | 0.367326535 | 0.471286875 | 2.612403046 |
| ribose | C00121 | 0.966345111 | 0.357128574 | 0.465415488 | 1.283462636 |
| isocitrate | C00311 | 0.942410074 | 0.359328134 | 0.465415488 | 1.220029866 |
| carnitine | C00487 | 0.920360002 | 0.403719256 | 0.508387211 | 1.067957102 |
| glucuronate | C00191 | 0.896194973 | 0.579084183 | 0.673123495 | 1.21671571 |
| cis-aconitate | C00417 | 0.678902233 | 0.49030194 | 0.600730304 | 1.092276423 |
| spermine | C00750 | 0.650288357 | 0.536692661 | 0.651698232 | 1.157754191 |
| adenosine_5'diphosphorib ose | C00020 | 0.612073031 | 0.554089182 | 0.661018673 | 1.074048371 |
| proline | C00148 | 0.536285082 | 0.571685663 | 0.670252156 | 1.08788306 |
| 7-beta-hydroxycholesterol | C03594 | 0.511913231 | 0.657068586 | 0.750935527 | 1.13242841 |
| oleate | C00712 | 0.495364144 | 0.903619276 | 0.945789468 | 1.24564639 |
| guanine | C00242 | 0.306116255 | 0.911017796 | 0.945789468 | 1.101595185 |
| N1-methyladenosine | C02494 | 0.293397754 | 0.788642272 | 0.875090023 | 1.058292571 |
| S-adenosylhomocysteine | C00021 | 0.287354295 | 0.785042991 | 0.875090023 | 1.067193013 |
| 2-hydroxystearate | C03045 | 0.20387351 | 0.826234753 | 0.903294541 | 1.053769973 |
| arabitol | C00474 | 0.166523847 | 0.908218356 | 0.945789468 | 1.046380428 |
| ethanolamine | C00189 | 0.160019445 | 0.879024195 | 0.941317248 | 1.033889323 |
| inositol_1-phosphate | C01177 | 0.126909671 | 0.902619476 | 0.945789468 | 1.032424174 |
| beta-alanine | C00099 | 0.029358416 | 0.954609078 | 0.976141614 | 1.00604818 |
| urea | C00086 | - 0.011263049 | 0.968006399 | 0.982454255 | 1.003877952 |
| glutamine | C00064 | - 0.040947438 | 0.985602879 | 0.992903641 | 1.007969293 |
| fucose | C00382 | - 0.079293687 | 0.99580084 | 0.99580084 | 1.021033485 |
| stearate | C01530 | - 0.120565217 | 0.940611878 | 0.969115268 | 1.027200106 |
| N-acetylneuraminate | C00270 | - 0.241637282 | 0.839432114 | 0.90605371 | 1.080760497 |
| glycerophosphorylcholine | C00670 | - 0.26074411 | 0.791441712 | 0.875090023 | 1.031687002 |
| alanine | C00041 | - 0.339449007 | 0.74605079 | 0.845524228 | 1.072507219 |
| daidzein | C10208 | - 0.396828559 | 0.830233953 | 0.903294541 | 1.132101137 |
| phosphoethanolamine | C00346 | - 0.529127619 | 0.616476705 | 0.710515524 | 1.137601266 |
| guanosine | C00387 | - 0.612164197 | 0.569286143 | 0.670252156 | 1.137600738 |
| creatinine | C00791 | - 0.612569424 | 0.543291342 | 0.653872765 | 1.117408011 |
| cytidine | C00475 | - 0.752474325 | 0.483103379 | 0.597291451 | 1.038949728 |
| hippurate | C01586 | - 0.963850443 | 0.411517696 | 0.513453273 | 1.812097642 |
| dimethylarginine | C03626 | - 1.010556019 | 0.330333933 | 0.436169077 | 1.226991293 |
| palmitoleate | C08362 | - 1.027651832 | 0.373725255 | 0.475015277 | 1.48008998 |
| allantoin | C02350 | - 1.091601809 | 0.322535493 | 0.430047324 | 1.22909512 |
| 1-oleoylglycerophosphocholine | C03916 | - 1.235651207 | 0.144171166 | 0.250529894 | 2.299674594 |
| 1-palmitoylglycerophosphocholine | C04102 | - 1.313110736 | 0.182963407 | 0.286779008 | 2.398375439 |
| N-acetylglutamine | C02716 | - 1.32387446 | 0.209958008 | 0.299140172 | 1.344494727 |
| inosine | C00294 | - 1.328685132 | 0.213357329 | 0.299140172 | 1.049579003 |
| nonadecanoate | C16535 | - 1.356220614 | 0.204559088 | 0.299140172 | 1.242624843 |
| uridine | C00299 | - 1.386031066 | 0.204759048 | 0.299140172 | 1.208592686 |
| glycerate | C00258 | - 1.394835645 | 0.165566887 | 0.27129032 | 1.56500217 |
| urocanate | C00785 | - 1.414697383 | 0.196760648 | 0.299140172 | 1.066816486 |
| stachydrine | C10172 | - 1.423477496 | 0.181763647 | 0.286779008 | 1.076436018 |
| arabinose | C00181 | - 1.631168606 | 0.147370526 | 0.250529894 | 1.08338153 |
| linolenate_[alpha_or_gam ma;_(18:3n3_or_6)] | C06427 | - 1.636346218 | 0.138372326 | 0.244397874 | 2.095455054 |
| genistein | C06563 | - 1.642382231 | 0.125774845 | 0.228071719 | 1.133305744 |
| trigonelline | C01004 | - 1.647807362 | 0.112977405 | 0.216066376 | 1.478233048 |
| erythronate | C01620 | - 1.727643931 | 0.115576885 | 0.216066376 | 1.092209671 |
| xylitol | C00379 | - 1.743195697 | 0.112777445 | 0.216066376 | 1.091316003 |
| palmitate | C00249 | - 1.746051908 | 0.124575085 | 0.228071719 | 1.40183288 |
| campesterol | C01789 | - 1.806224883 | 0.103979204 | 0.214260178 | 1.854858177 |
| 4-guanidinobutanoate | C01035 | - 1.835399006 | 0.069986003 | 0.166984147 | 1.794083739 |
| 1-methylimidazoleacetate | C05828 | - 1.861896377 | 0.102179564 | 0.213791088 | 1.094316851 |
| choline_phosphate | C00588 | - 1.868693128 | 0.097580484 | 0.207358528 | 1.360639257 |
| cystathionine | C02291 | - 1.940376865 | 0.075984803 | 0.174045191 | 2.476129175 |
| 3-ureidopropionate | C02642 | - 1.994480853 | 0.076784643 | 0.174045191 | 1.102582726 |
| adenosine_3'-monophosphate | C01367 | - 2.008881945 | 0.067186563 | 0.165595452 | 1.516930206 |
| cysteine | C00097 | - 2.187203269 | 0.045590882 | 0.121575685 | 1.946237595 |
| uridine_5'-monophosphate | C00105 | - 2.196658136 | 0.00759848 | 0.030216179 | 3.259640455 |
| 5-oxoproline | C01879 | - 2.226686297 | 0.017396521 | 0.055021554 | 2.154245824 |
| alpha-tocopherol | C02477 | - 2.23824325 | 0.050589882 | 0.129815546 | 1.111658614 |
| adenine | C00147 | - 2.457363752 | 0.032193561 | 0.089353558 | 1.87737174 |
| pantothenate | C00864 | - 2.554952589 | 0.016396721 | 0.053094143 | 2.761840905 |
| docosahexaenoate | C06429 | - 2.682822525 | 0.00019996 | 0.002472233 | 2.150603511 |
| docosapentaenoate | C16513 | - 2.718069448 | 0.0029994 | 0.018541746 | 1.835848861 |
| pyridoxate | C00847 | - 2.738352083 | 0.026794641 | 0.077533429 | 1.140050442 |
| cytidine_5'-diphosphocholine | C00307 | - 3.093460689 | 0.00659868 | 0.028949049 | 1.687784683 |
| arginine | C00062 | - 3.178293058 | 0.00639872 | 0.028949049 | 1.197039482 |
| linoleate | C01595 | - 3.341037454 | 0.00479904 | 0.024172943 | 2.648550608 |
| 5-methylthioadenosine | C00170 | - 3.672915952 | 0.00559888 | 0.027194561 | 1.77421305 |
| 3-dehydrocarnitine | C02636 | - 3.812488098 | 0.00439912 | 0.023010782 | 3.030002448 |
| xanthine | C00385 | - 3.974250304 | 0.00019996 | 0.002472233 | 1.416735201 |
| glutamate | C00025 | - 4.027289964 | 0.00419916 | 0.022843431 | 1.68866346 |
| phosphate | C00009 | - 4.356812631 | 0.00259948 | 0.016834728 | 1.351048477 |
| arachidonate | C00219 | - 4.527712505 | 0.00019996 | 0.002472233 | 2.05447056 |
| betaine | C00719 | - 4.787930679 | 0.00019996 | 0.002472233 | 2.132690945 |
| nicotinamide | C00153 | - 4.833362163 | 0.00019996 | 0.002472233 | 1.242336465 |
| taurine | C00245 | - 4.890479424 | 0.00219956 | 0.014957009 | 1.3311185 |
| adenosine | C00212 | - 5.526740727 | 0.00019996 | 0.002472233 | 2.130704285 |
| pseudouridine | C02067 | - 5.635590595 | 0.00019996 | 0.002472233 | 2.21339505 |
| UDP-glucose | C00029 | - 5.738020226 | 0.00019996 | 0.002472233 | 2.727880622 |
| cytidine-3'-monophosphate | C05822 | - 5.842264043 | 0.00019996 | 0.002472233 | 3.0266933 |
| dihomo-linolenate | C03242 | - 12.06944017 | 0.00019996 | 0.002472233 | 4.764943624 |
| sarcosine | C00213 | - 25.32566958 | 0.00019996 | 0.002472233 | 13.98934706 |

**Table 2: Human tumors**

| **Metabolite** | **KEGG ID** | **Statisti c** | **Pvalue** | **BH** | **Fold Change (PhosphoAKT1-high/MYC-high)** |
|---|---|---|---|---|---|
| fructose-6-phosphate | C05345 | 3.81110406 | 0.00019996 | 0.045590882 | 3.631619045 |
| uridine | C00299 | 3.5590535 | 0.00119976 | 0.078155797 | 1.296349317 |
| leucylleucine | C11332 | 3.224640404 | 0.017396521 | 0.305108209 | 2.165606551 |
| creatine | C00300 | 3.164706233 | 0.014597081 | 0.277344531 | 1.33537068 |
| cytidine | C00475 | 3.00590461 | 0.027194561 | 0.401769646 | 2.333657123 |
| lactate | C00186 | 2.953716944 | 0.013197361 | 0.277344531 | 1.388641177 |
| cytidine_5'-monophosphate | C00055 | 2.879610664 | 0.013797241 | 0.277344531 | 1.568545877 |
| UDP-N-acetylglucosamine | C00043 | 2.860988679 | 0.020195961 | 0.328905647 | 1.984143569 |
| inosine | C00294 | 2.760442558 | 0.014397121 | 0.277344531 | 1.491261092 |
| histamine | C00388 | 2.536010991 | 0.048590282 | 0.443143371 | 2.471158482 |
| phenol_sulfate | C02180 | 2.4373911 | 0.054189162 | 0.457597369 | 2.039715077 |
| glutathione,_reduced | C00051 | 2.396276322 | 0.047990402 | 0.443143371 | 2.100982459 |
| 1,5-anhydroglucitol | C07326 | 2.341062169 | 0.047590482 | 0.443143371 | 1.635022329 |
| pyruvate | C00022 | 2.305345621 | 0.069386123 | 0.465295176 | 1.743049791 |
| maltotriose | C01835 | 2.290135808 | 0.080583883 | 0.483503299 | 3.655638074 |
| urea | C00086 | 2.284307214 | 0.066386723 | 0.465295176 | 2.103980913 |
| glucose-6-phosphate | C00668 | 2.279352365 | 0.064187163 | 0.465295176 | 2.329128567 |
| S-adenosylhomocysteine | C00021 | 2.273586198 | 0.032793441 | 0.439817919 | 1.352588589 |
| taurine | C00245 | 2.190941908 | 0.075784843 | 0.47685598 | 1.77187529 |
| glutathione,_oxidized | C00127 | 2.187730524 | 0.067986403 | 0.465295176 | 2.01563179 |
| maltotetraose | C02052 | 2.163987577 | 0.114177165 | 0.542341532 | 2.146561165 |
| adenosine_5'diphospho ribose | C00301 | 2.151206354 | 0.091381724 | 0.514525666 | 1.995777382 |
| 5-methylthioadenosine | C00170 | 2.102798431 | 0.056988602 | 0.464050047 | 1.341762849 |
| ascorbate | C00072 | 2.089903443 | 0.093981204 | 0.514525666 | 1.847117019 |
| mannose-6-phosphate | C00275 | 2.038634098 | 0.134373125 | 0.567353196 | 1.841302621 |
| maltose | C00208 | 1.978487143 | 0.086782643 | 0.507344685 | 2.10652292 |
| guanosine | C00387 | 1.946126345 | 0.066186763 | 0.465295176 | 1.184773035 |
| N-acetylneuraminate | C00270 | 1.874857437 | 0.212557489 | 0.660763847 | 1.684556067 |
| glutamine | C00064 | 1.864128402 | 0.111377724 | 0.542341532 | 1.283122061 |
| mannitol | C00392 | 1.85417422 | 0.159168166 | 0.613957209 | 1.771333318 |
| dehydroisoandrosterone sulfate | C04555 | 1.853918677 | 0.123775245 | 0.547090582 | 1.411160733 |
| catechol_sulfate | C00090 | 1.800513285 | 0.124775045 | 0.547090582 | 1.588529525 |
| trans-4-hydroxyproline | C01157 | 1.795999807 | 0.161567686 | 0.613957209 | 1.442095143 |
| phenylacetylglutamine | C05597 | 1.775341794 | 0.279144171 | 0.684353452 | 2.577157033 |
| N-acetyl-aspartyl-glutamate | C12270 | 1.768713793 | 0.173365327 | 0.630226896 | 1.637257633 |
| creatinine | C00791 | 1.74789424 | 0.120975805 | 0.547090582 | 1.274099083 |
| nicotinamide | C00153 | 1.700772921 | 0.152569486 | 0.610277944 | 1.25418923 |
| N-acetylaspartate | C01042 | 1.696249859 | 0.199960008 | 0.651298312 | 1.569771486 |
| ergothioneine | C05570 | 1.646630524 | 0.185562887 | 0.630226896 | 1.307208836 |
| beta-alanine | C00099 | 1.626964981 | 0.176364727 | 0.630226896 | 1.477852965 |
| mannose | C00159 | 1.626534076 | 0.203759248 | 0.654325473 | 1.416041172 |
| tryptophan_betaine | C09213 | 1.603211561 | 0.181163767 | 0.630226896 | 1.497837098 |
| choline_phosphate | C00588 | 1.599288114 | 0.217356529 | 0.660763847 | 2.134075496 |
| piperine | C03882 | 1.587917194 | 0.208958208 | 0.660763847 | 1.496167125 |
| theobromine | C07480 | 1.542597536 | 0.25634873 | 0.671810465 | 1.699841541 |
| hippurate | C01586 | 1.532123814 | 0.23815237 | 0.66628602 | 1.87941845 |
| inositol_1-phosphate | C01177 | 1.500814292 | 0.198760248 | 0.651298312 | 1.283656967 |
| 3 -methylhistidine | C01152 | 1.495733462 | 0.182563487 | 0.630226896 | 1.153833753 |
| coenzyme_A | C00010 | 1.483056194 | 0.273945211 | 0.684353452 | 1.362349373 |
| cysteinylglycine | C01419 | 1.477806304 | 0.187962408 | 0.630226896 | 1.313383292 |
| glycerol_3-phosphate | C00093 | 1.454030776 | 0.229154169 | 0.665396035 | 1.303381796 |
| adenosine_5'-diphosphate | C00008 | 1.431174873 | 0.236352729 | 0.66628602 | 1.484766413 |
| deoxycholate | C04483 | 1.398727925 | 0.5054989 | 0.76214757 | 1.357954808 |
| phenylacetylglycine | C05598 | 1.395250142 | 0.466706659 | 0.749359987 | 1.391377916 |
| N-acetylputrescine | C02714 | 1.39274986 | 0.293341332 | 0.689503336 | 1.789939705 |
| hexanoylcarnitine | C01585 | 1.363520304 | 0.340331934 | 0.718056389 | 1.503644559 |
| 4-acetamidophenol | C06804.2 | 1.355041212 | 0.444111178 | 0.729782101 | 1.479625675 |
| nicotinamide_adenine_dinucleotide | C00003 | 1.3448852 | 0.273945211 | 0.684353452 | 1.792508776 |
| myo-inositol | C00137 | 1.333683666 | 0.24255149 | 0.66628602 | 1.28150763 |
| cholesterol | C00187 | 1.330887533 | 0.276944611 | 0.684353452 | 1.138722283 |
| 3 -aminoisobutyrate | C05145 | 1.307978379 | 0.381923615 | 0.718056389 | 1.602561188 |
| adenosine | C00212 | 1.253618674 | 0.269346131 | 0.684353452 | 1.713276852 |
| phosphate | C00009 | 1.229934813 | 0.24075185 | 0.66628602 | 1.09104206 |
| penicillin_ G | C05551 | 1.205383457 | 0.703059388 | 0.914378922 | 1.406842867 |
| aspartate | C00049 | 1.201319034 | 0.286942611 | 0.686237752 | 1.308740642 |
| scyllo-inositol | C06153 | 1.190527917 | 0.337732454 | 0.718056389 | 1.50662793 |
| urate | C00366 | 1.177640063 | 0.332133573 | 0.718056389 | 1.301153419 |
| 7-alpha-hydroxy-3-oxo-4-cholestenoate | C17337 | 1.1766 47545 | 0.343731254 | 0.718056389 | 1.281875544 |
| pipecolate | C00408 | 1.1736 63806 | 0.416116777 | 0.729782101 | 1.504667056 |
| nicotinamide_adenine_ dinucleotide_reduced | C00004 | 1.1723 02867 | 0.474305139 | 0.750520241 | 1.563657688 |
| anserine | C01262 | 1.1584 06973 | 0.390521896 | 0.718056389 | 1.210618102 |
| paraxanthine | C13747 | 1.1542 35688 | 0.48930214 | 0.750872644 | 1.531871859 |
| phosphoethanolamine | C00346 | 1.1426 17764 | 0.348930214 | 0.718056389 | 1.494782606 |
| citrate | C00158 | 1.0987 33522 | 0.331533693 | 0.718056389 | 1.24868118 |
| alpha-tocopherol | C02477 | 1.0852 10151 | 0.387522496 | 0.718056389 | 1.290527511 |
| p-cresol_sulfate | C01468 | 1.0672 45671 | 0.449510098 | 0.732059302 | 1.460053194 |
| arabitol | C00532 | 1.0486 87356 | 0.367926415 | 0.718056389 | 1.203265501 |
| uridine_5'-diphosphate | C00015 | 1.0115 88945 | 0.375124975 | 0.718056389 | 1.103932441 |
| 3'-dephosphocoenzyme_A | C00882 | 1.0115 88945 | 0.375124975 | 0.718056389 | 1.103932441 |
| quinolinate | C03722 | 1.0115 88945 | 0.375124975 | 0.718056389 | 1.103932441 |
| 2'-deoxyinosine | C05512 | 1.0115 88945 | 0.375124975 | 0.718056389 | 1.103932441 |
| sebacate | C08277 | 1.0115 88945 | 0.375124975 | 0.718056389 | 1.103932441 |
| azelate | C08261 | 1.0115 88945 | 0.375124975 | 0.718056389 | 1.103932441 |
| 6-phosphogluconate | C00345 | 1.0115 88945 | 0.375124975 | 0.718056389 | 1.103932441 |
| fructose | C00095 | 0.9987 32523 | 0.477304539 | 0.750520241 | 1.348612629 |
| homocarnosine | C00884 | 0.9739 96509 | 0.433713257 | 0.729782101 | 1.118525395 |
| erythritol | C00503 | 0.9680 81213 | 0.366326735 | 0.718056389 | 1.223530988 |
| 2-hydroxyglutarate | C02630 | 0.9036 70379 | 0.49070186 | 0.750872644 | 1.239519184 |
| flavin_adenine_dinucleotide | C00016 | 0.8972 86084 | 0.407718456 | 0.729782101 | 1.091839845 |
| 3-phosphoglycerate | C00597 | 0.8926 37896 | 0.427114577 | 0.729782101 | 1.335202731 |
| glycerophosphorylcholine | C00670 | 0.892261738 | 0.415916817 | 0.729782101 | 1.256250614 |
| ribose | C00121 | 0.879737026 | 0.644071186 | 0.86892444 | 1.307059676 |
| acetylcholine | C01996 | 0.879122109 | 0.444911018 | 0.729782101 | 1.370712507 |
| xylulose | C00310 | 0.837886371 | 0.48930214 | 0.750872644 | 1.514763173 |
| 1,7-dimethylurate | C16356 | 0.836286685 | 0.464707059 | 0.749359987 | 1.091151065 |
| spermine | C00750 | 0.815371256 | 0.476704659 | 0.750520241 | 1.353323086 |
| carnosine | C00386 | 0.789437851 | 0.789842032 | 0.920337145 | 1.25247823 |
| pseudouridine | C02067 | 0.771980805 | 0.481103779 | 0.750872644 | 1.144829751 |
| xylitol | C00379 | 0.746479833 | 0.49470106 | 0.751945611 | 1.193974941 |
| agmatine | C00179 | 0.724132598 | 0.74005199 | 0.916091782 | 1.424265546 |
| 5-hydroxyindoleacetate | C05635 | 0.704121017 | 0.75164967 | 0.916091782 | 1.316137169 |
| isocitrate | C00311 | 0.697255242 | 0.515096981 | 0.762611114 | 1.206810699 |
| 2-hydroxystearate | C03045 | 0.695011635 | 0.696860628 | 0.914378922 | 1.320713987 |
| pyridoxate | C00847 | 0.694372025 | 0.547690462 | 0.790626685 | 1.083699919 |
| 4-acetaminophensulfate | C06804 | 0.669644149 | 0.940211958 | 0.988971436 | 1.324502651 |
| glycerol_2-phosphate | C02979 | 0.654025364 | 0.705258948 | 0.914378922 | 1.192696288 |
| galactose | C01662 | 0.63022944 | 0.903019396 | 0.985112068 | 1.335333127 |
| 2-aminobutyrate | C02261 | 0.613266453 | 0.603079384 | 0.838427436 | 1.102669936 |
| 2-hydroxybutyrate | C05984 | 0.603030254 | 0.713857229 | 0.914378922 | 1.179598702 |
| glycylleucine | C02155 | 0.53338166 | 0.771445711 | 0.916091782 | 1.147544092 |
| cis-aconitate | C00417 | 0.515881155 | 0.637072585 | 0.864598509 | 1.086153528 |
| caffeine | C07481 | 0.463470208 | 0.973205359 | 0.995026107 | 1.266641105 |
| heme | C00032 | 0.459503759 | 0.723255349 | 0.916091782 | 1.155470112 |
| 4-vinylphenol_sulfate | C05627 | 0.450840239 | 0.700859828 | 0.914378922 | 1.045559892 |
| serotonin | C00780 | 0.411133551 | 0.922615477 | 0.988971436 | 1.257767671 |
| indolelactate | C02043 | 0.407493479 | 0.711257748 | 0.914378922 | 1.042746396 |
| uridine_5'-monophosphate | C00105 | 0.386922582 | 0.74545091 | 0.916091782 | 1.059177357 |
| ribulose | C00309 | 0.386267724 | 0.735252949 | 0.916091782 | 1.131219725 |
| adenosine_5'-triphosphate | C00002 | 0.381622434 | 0.791041792 | 0.920337145 | 1.168782463 |
| histidine | C00135 | 0.378215548 | 0.74785043 | 0.916091782 | 1.053692586 |
| N-acetylthreonine | C01118 | 0.372813536 | 0.765646871 | 0.916091782 | 1.055872423 |
| glucose | C00293 | 0.359947046 | 0.783643271 | 0.920337145 | 1.194566578 |
| 3-(4-hydroxyphenyl)lactate | C03672 | 0.35990493 | 0.856628674 | 0.962124816 | 1.047229626 |
| betaine | C00719 | 0.340011458 | 0.767646471 | 0.916091782 | 1.04759208 |
| adenine | C00147 | 0.327695462 | 0.75164967 | 0.916091782 | 1.082169065 |
| 2-aminoadipate | C00956 | 0.267758001 | 0.825434913 | 0.940995801 | 1.046817541 |
| arginine | C00062 | 0.251031631 | 0.839432114 | 0.947477831 | 1.036651855 |
| gamma-tocopherol | C02483 | 0.23180112 | 0.873425315 | 0.976181234 | 1.090608496 |
| spermidine | C00315 | 0.229310575 | 0.9910018 | 0.99540092 | 1.0838126 |
| mcotmamide_ribonucleotide | C00455 | 0.182058732 | 0.892221556 | 0.985112068 | 1.133131703 |
| lidocaine | D00358 | 0.172240862 | 0.911217756 | 0.988971436 | 1.028583696 |
| succinate | C00042 | 0.158521544 | 0.903019396 | 0.985112068 | 1.051040907 |
| sorbitol | C00794 | 0.10936012 | 0.943611278 | 0.988971436 | 1.042315556 |
| cytidine_5'-diphosphocholine | C00307 | 0.10159355 | 0.942811438 | 0.988971436 | 1.016316771 |
| methyl-alpha-glucopyranoside | C03619 | 0.09719625 | 0.965406919 | 0.991498997 | 1.048610069 |
| stearoyl_sphingomyelin | C00550 | 0.093779078 | 0.958608278 | 0.988971436 | 1.029155736 |
| putrescine | C00134 | 0.092245224 | 0.98840232 | 0.99540092 | 1.059834522 |
| 2-hydroxyhippurate | C07588 | 0.06330477 | 0.99040192 | 0.99540092 | 1.00706226 |
| docosatrienoate | C16534 | 0.032201351 | 0.995001 | 0.99540092 | 1.017231183 |
| kynurenine | C00328 | 0.022919927 | 0.957808438 | 0.988971436 | 1.004661722 |
| N-acetylglucosamine | C00140 | 0.01591034 | 0.957608478 | 0.988971436 | 1.004489199 |
| stearate | C01530 | - 0.01550318 | 0.99540092 | 0.99540092 | 1.002009807 |
| N-acetylmethionine | C02712 | - 0.020115272 | 0.941611678 | 0.988971436 | 1.003991726 |
| guanine | C00242 | - 0.035556161 | 0.921415717 | 0.988971436 | 1.01052316 |
| sphingosine | C00319 | - 0.037883518 | 0.949810038 | 0.988971436 | 1.023790417 |
| quinate | C00296 | - 0.098178665 | 0.894021196 | 0.985112068 | 1.050596138 |
| deoxycarnitine | C01181 | - 0.114532588 | 0.902219556 | 0.985112068 | 1.015591531 |
| proline | C00148 | - 0.197447279 | 0.830633873 | 0.942211558 | 1.021668257 |
| alanine | C00041 | - 0.222379819 | 0.799240152 | 0.920337145 | 1.025560863 |
| cysteine | C00097 | - 0.23066041 | 0.795240952 | 0.920337145 | 1.071373533 |
| gluconate | C00257 | - 0.240452951 | 0.948810238 | 0.988971436 | 1.204717508 |
| choline | C00114 | - 0.243434682 | 0.796640672 | 0.920337145 | 1.021974646 |
| acetylcarnitine | C02571 | - 0.244371553 | 0.807238552 | 0.924876331 | 1.049967419 |
| 1-linoleoylglycerophosphocholine | C04100 | - 0.254889749 | 0.75664867 | 0.916091782 | 1.135681491 |
| propionylcarnitine | C03017 | - 0.275132712 | 0.74245151 | 0.916091782 | 1.061850266 |
| saccharopine | C00449 | - 0.28725988 | 0.76044791 | 0.916091782 | 1.046367894 |
| palmitate | C00249 | - 0.354014159 | 0.712457508 | 0.914378922 | 1.039708784 |
| adenosine_5'-monophosphate | C00020 | - 0.365312674 | 0.680663867 | 0.91288409 | 1.102051391 |
| alpha-ketoglutarate | C00026 | - 0.365833807 | 0.768846231 | 0.916091782 | 1.276380806 |
| N-acetylalanine | C02847 | - 0.375076775 | 0.684663067 | 0.91288409 | 1.058891644 |
| glycerophosphoethanolamine | C01233 | - 0.459162522 | 0.616676665 | 0.847001684 | 1.370587487 |
| valine | C00183 | - 0.485648159 | 0.607478504 | 0.839424842 | 1.061104128 |
| malate | C00149 | - 0.506537595 | 0.599880024 | 0.838427436 | 1.087512039 |
| hypoxanthine | C00262 | - 0.511442281 | 0.623675265 | 0.851484793 | 1.072294605 |
| N-ethylglycinexylidide | C16561 | - 0.517259887 | 0.563487303 | 0.803362309 | 1.679342042 |
| gamma-amino butyrate | C00334 | - 0.517932626 | 0.567286543 | 0.803362309 | 1.217157809 |
| xanthine | C00385 | - 0.518175386 | 0.585082983 | 0.823450125 | 1.307873952 |
| 4-hydroxybutyrate | C00989 | - 0.558487839 | 0.542491502 | 0.790626685 | 1.333623809 |
| carnitine | C00487 | - 0.573912686 | 0.565886823 | 0.803362309 | 1.053788746 |
| myristate | C06424 | - 0.580215656 | 0.547890422 | 0.790626685 | 1.057091142 |
| 1-palmitoylglycerophosphocholine | C04102 | - 0.582903686 | 0.535692861 | 0.787986919 | 1.227319819 |
| fumarate | C00122 | - 0.5907 87109 | 0.51169 766 | 0.76252 9847 | 1.201943393 |
| pantothenate | C00864 | - 0.6194 7029 | 0.50809 838 | 0.76214 757 | 1.122139149 |
| hypotaurine | C00519 | - 0.6861 34721 | 0.43891 2218 | 0.72978 2101 | 1.404934212 |
| citrulline | C00327 | - 0.7172 1526 | 0.43971 2058 | 0.72978 2101 | 1.154093441 |
| N6-acetyllysine | C02727 | - 0.7266 22361 | 0.42291 5417 | 0.72978 2101 | 1.158806251 |
| nicotinamide_riboside | C03150 | - 0.7356 71863 | 0.43231 3537 | 0.72978 2101 | 1.333837262 |
| ethanolamine | C00189 | - 0.7441 0792 | 0.42671 4657 | 0.72978 2101 | 1.147102652 |
| serine | C00065 | - 0.7568 29716 | 0.41631 6737 | 0.72978 2101 | 1.139770884 |
| threonine | C00188 | - 0.7695 46045 | 0.41231 7536 | 0.72978 2101 | 1.13742408 |
| fucose | C00382 | - 0.7811 90415 | 0.37892 4215 | 0.71805 6389 | 1.304574983 |
| glycine | C00037 | - 0.8000 35095 | 0.43151 3697 | 0.72978 2101 | 1.099792275 |
| sarcosine | C00213 | - 0.8094 16649 | 0.36512 6975 | 0.71805 6389 | 1.37892641 |
| N-acetyltryptophan | C03137 | - 0.8131 14177 | 0.28574 2851 | 0.68623 7752 | 2.372048471 |
| asparagine | C00152 | - 0.8527 30293 | 0.38512 2975 | 0.71805 6389 | 1.149741446 |
| 1 -arachidonylglycerol | C13857 | - 0.8639 41256 | 0.34413 1174 | 0.71805 6389 | 1.154020568 |
| ornithine | C00077 | - 0.8872 08148 | 0.33473 3053 | 0.71805 6389 | 1.304123674 |
| butyrylcarnitine | C02862 | - 0.907591738 | 0.351329734 | 0.718056389 | 1.230570118 |
| 5,6-dihydrouracil | C00429 | - 0.932680872 | 0.337532494 | 0.718056389 | 1.358845909 |
| 1-oleoylglycerophosphocholine | C03916 | - 0.974716816 | 0.25614877 | 0.671810465 | 1.746513462 |
| glycerate | C00258 | - 1.011392979 | 0.288942212 | 0.686237752 | 1.250272854 |
| 1-stearoylglycerol | D01947 | - 1.014411909 | 0.311537692 | 0.717480746 | 1.248927124 |
| isoleucine | C00407 | - 1.044635456 | 0.266746651 | 0.684353452 | 1.127636431 |
| N1-methyladenosine | C02494 | - 1.047291868 | 0.308738252 | 0.717480746 | 1.105498762 |
| 3 -hydroxybutyrate | C01089 | - 1.101027329 | 0.217356529 | 0.660763847 | 1.838434169 |
| 5-oxoproline | C01879 | - 1.104600786 | 0.24895021 | 0.671810465 | 1.21147617 |
| tryptophan | C00078 | - 1.12193614 | 0.230553889 | 0.665396035 | 1.19928843 |
| ribitol | C00474 | - 1.125816518 | 0.223555289 | 0.665396035 | 1.374021712 |
| methionine | C00073 | - 1.129416374 | 0.178564287 | 0.630226896 | 1.243998417 |
| nonadecanoate | C16535 | - 1.151378406 | 0.225354929 | 0.665396035 | 1.456461102 |
| glutarate | C00489 | - 1.196421325 | 0.142371526 | 0.586168481 | 2.141975907 |
| glutamate | C00025 | - 1.254041416 | 0.25374925 | 0.671810465 | 1.105792357 |
| lysine | C00047 | - 1.254680803 | 0.161567686 | 0.613957209 | 1.478345503 |
| docosapentaenoate | C16513 | - 1.265656087 | 0.179364127 | 0.630226896 | 1.449894385 |
| dimethylarginine | C03626 | - 1.267623363 | 0.143971206 | 0.586168481 | 1.467794919 |
| eicosapentaenoate | C06428 | - 1.38501701 | 0.114177165 | 0.542341532 | 1.609937133 |
| riboflavin | C00255 | - 1.419242579 | 0.109378124 | 0.542341532 | 1.384043971 |
| linoleate | C01595 | - 1.435954261 | 0.119576085 | 0.547090582 | 1.354780572 |
| 3-dehydrocarnitine | C02636 | - 1.534202089 | 0.100379924 | 0.532247039 | 1.431415076 |
| adrenate | C16527 | - 1.549864721 | 0.113577285 | 0.542341532 | 1.361190854 |
| tyrosine | C00082 | - 1.555221319 | 0.094781044 | 0.514525666 | 1.214296185 |
| glycerol | C00116 | - 1.589969819 | 0.132973405 | 0.567353196 | 1.188535382 |
| palmitoleate | C08362 | - 1.597100074 | 0.072185563 | 0.470237381 | 1.380817004 |
| cystine | C00491 | - 1.618523501 | 0.043591282 | 0.443143371 | 2.303976537 |
| guanosine_5'-monophosphate | C00144 | - 1.653433932 | 0.077384523 | 0.47685598 | 1.460997765 |
| phenylalanine | C00079 | - 1.676772892 | 0.064187163 | 0.465295176 | 1.267094175 |
| dihomo-linoleate | C16525 | - 1.748264892 | 0.039392122 | 0.443143371 | 1.869440782 |
| linolenate_[alpha_or_gamma_(18:3n3_or_6)] | C06427 | - 1.786221798 | 0.053989202 | 0.457597369 | 1.502230891 |
| leucine | C00123 | - 1.795683069 | 0.035792841 | 0.443143371 | 1.368682405 |
| uracil | C00106 | - 1.819705221 | 0.037392521 | 0.443143371 | 1.882501068 |
| docosapentaenoate | C06429.2 | - 1.861003744 | 0.00239952 | 0.078155797 | 2.727908389 |
| docosadienoate | C16533 | - 1.879714016 | 0.041591682 | 0.443143371 | 1.872861712 |
| docosahexaenoate | C06429 | - 2.051674201 | 0.014197161 | 0.277344531 | 1.949122819 |
| arachidonate | C00219 | - 2.199592552 | 0.028194361 | 0.401769646 | 1.49143101 |
| xanthosine | C01762 | - 2.766594703 | 0.0019996 | 0.078155797 | 2.98512127 |
| dihomo-linolenate | C03242 | - 3.016825355 | 0.00219956 | 0.078155797 | 2.115186614 |
| cis-vaccenate | C08367 | - 3.242499914 | 0.00079984 | 0.078155797 | 2.464331393 |
| oleate | C00712 | - 3.455677401 | 0.0019996 | 0.078155797 | 1.718089283 |

Table 3: List of metabolite sets tested by GSEA in RWPE-AKT1 cells, MPAKT mice and phosphoAKT1-high/MYC-low tumors compared to RWPE-MYC cells, Lo-MYC mice and MYC-high/phosphoAKT1-low tumors, respectively.

**Table 3: GSEA RWPE-AKT1**

| **Metabolite set** | **No of metaboli tes** | **Normaliz ed Enrichme nt Score** | **NOM p-val** | **FDR q-val** | **FWER p-val** | **RANK AT MAX** |
|---|---|---|---|---|---|---|
| PENTOSE PHOSPHATEPATHWAY | 4 | 1.460002 | 0.028629856 | 0.9964033 | 0.565 | 2 |
| FRUCTOSE_AND_MANNOSE_METABOLISM | 4 | 1.4568312 | 0.12215321 | 0.50753045 | 0.573 | 1 |
| GLYCOLYSISGLUCONEOGENESIS | 5 | 1.3630538 | 0.15853658 | 0.7315131 | 0.792 | 13 |
| BIOSYNTHESIS_OF_UNSATURATED_FATTY_ACIDS | 9 | 1.2915634 | 0.24528302 | 0.8947219 | 0.937 | 11 |
| AMINO_SUGAR_AND_NUCLEOTIDE_SUGAR_METABOLISM | 7 | 1.2851669 | 0.21052632 | 0.7534751 | 0.944 | 7 |
| FATTY_ACID_METABOLISM | 2 | 1.2704923 | 0.14541833 | 0.682325 | 0.95 | 8 |
| PORPHYRIN_AND_CHLOROPHYLL_METABOLISM | 3 | 1.2340059 | 0.10080645 | 0.71324664 | 0.973 | 33 |
| D-GLUTAMINE_AND_D-GLUTAMATE_METABOLISM | 2 | 1.2266324 | 0.15240084 | 0.64646983 | 0.975 | 18 |
| LYSINE_DEGRADATION | 3 | 1.1647791 | 0.23246492 | 0.7812183 | 0.993 | 42 |
| VALINE_LEUCINE_AND_ISOLEUCINE_BIOSYNTHESIS | 4 | 1.1625785 | 0.24901961 | 0.7165096 | 0.997 | 36 |
| TRYPTOPHAN_METABOLISM | 2 | 1.1446722 | 0.156 | 0.7044717 | 0.999 | 4 |
| PHENYLALANINE_TYROSINE_AND_TRYPTOPHAN_BIOSYNTHESIS | 3 | 1.1393136 | 0.2672065 | 0.6605307 | 0.999 | 32 |
| SPHINGOLIPID_METABOLISM | 2 | 1.0989345 | 0.46747968 | 0.72400373 | 0.999 | 27 |
| LINOLEIC_ACID_METABOLISM | 2 | 1.0814053 | 0.4389313 | 0.72070676 | 1 | 10 |
| VALINE LEUCINE AND_ISOLEUCINE_DEGRADATION | 3 | 1.0684689 | 0.40944883 | 0.7040403 | 1 | 36 |
| PURINE_METABOLISM | 15 | 1.0494529 | 0.418 | 0.6976 | 1 | 18 |
| GLYOXYLATE_AND_DICARBOXYLATE_METABOLISM | 6 | 0.98523366 | 0.4792531 | 0.79075235 | 1 | 39 |
| PROPANOATE_METABOLISM | 3 | 0.97056115 | 0.54980844 | 0.7753743 | 1 | 47 |
| STARCH_AND_SUCROSE_METABOLISM | 6 | 0.96169555 | 0.43835616 | 0.748519 | 1 | 0 |
| PRIMARY_BILE_ACID_BIOSYNTHESIS | 2 | 0.8673413 | 0.7649484 | 0.87141985 | 1 | 57 |
| PENTOSE_AND_GLUCURONATE_INTERC ONVERSIONS | 2 | 0.8546371 | 0.7352342 | 0.8472796 | 1 | 21 |
| GALACTOSE_METABOLISM | 6 | 0.85244286 | 0.6832298 | 0.8113915 | 1 | 0 |
| BUTIROSIN_AND_NEOMYCIN_BIOSYNTHESIS | 2 | 0.785421 | 0.783838 4 | 0.86009914 | 1 | 55 |
| CYANOAMINO_ ACID _METABOLISM | 5 | 0.74070346 | 0.856262 86 | 0.87580043 | 1 | 28 |
| ASCORBATE_AND_ALDARATE_METABOLISM | 5 | 0.66054755 | 0.834331 33 | 0.9222075 | 1 | 22 |
| D-ARGININE_AND_D-ORNITHINE_METABOLISM | 2 | 0.49286503 | 0.983193 3 | 0.98765165 | 1 | 81 |

**Table 3: GSEA-RWPE-MYC**

| **Metabolite set** | **No of meta bolite s** | **Normalized Enrichment Score** | **NOM p-val** | **FDR q-val** | **FWER p-val** | **RANK AT MAX** |
|---|---|---|---|---|---|---|
| PANTOTHENATE_AND_COA_BIOSYNTHESIS | 6 | -1.3073608 | 0.098196395 | 1 | 0.933 | 14 |
| BETA-ALANINE_METABOLISM | 8 | -1.237877 | 0.20564516 | 1 | 0.969 | 24 |
| NICOTINATE_AND_NICOTINAMIDE_METABOLISM | 5 | -1.1971127 | 0.16875 | 1 | 0.988 | 19 |
| LYSINE_BIOSYNTHESIS | 2 | -1.1673465 | 0.20272905 | 1 | 0.988 | 14 |
| GLYCEROPHOSPHOLIPID_METABOLISM | 5 | -1.1504487 | 0.312 | 1 | 0.997 | 11 |
| BUTANOATE_METABOLISM | 3 | -1.1440222 | 0.2929293 | 1 | 0.997 | 19 |
| TAURINE_AND_HYPOTAURINE_METABOLISM | 5 | -1.1243932 | 0.26899385 | 1 | 0.997 | 43 |
| INOSITOL_PHOSPHATE_METABOLISM | 3 | -1.0681249 | 0.42519686 | 1 | 1 | 37 |
| PYRUVATE_METABOLISM | 4 | -1.0595751 | 0.37475345 | 1 | 1 | 2 |
| GLYCEROLIPID_METABOLISM | 3 | -1.0349437 | 0.49501 | 1 | 1 | 31 |
| OXIDATIVE_ PHOSPHORYLATION | 7 | -1.0332325 | 0.4569672 | 0.9870848 | 1 | 25 |
| ALANINE_ASPARTATE_AND_GLUTAMATE_METABOLISM | 8 | -1.0102847 | 0.47368422 | 0.9652419 | 1 | 28 |
| ARGININE_AND_PROLINE_METABOLISM | 13 | -1.006397 | 0.4853229 | 0.9018485 | 1 | 24 |
| CYSTEINE_AND_METHIONINE_METABOLISM | 8 | -0.9592696 | 0.50395256 | 0.9378031 | 1 | 35 |
| HISTIDINE_METABOLISM | 3 | -0.95365137 | 0.5246548 | 0.88732415 | 1 | 14 |
| FATTY_ACID_BIOSYNTHESIS | 7 | -0.9490036 | 0.55220884 | 0.8413623 | 1 | 29 |
| GLUTATHIONE_METABOLISM | 12 | -0.93477863 | 0.4864865 | 0.81286883 | 1 | 35 |
| CITRATE_CYCLE_TCA_CYCLE | 3 | -0.90559417 | 0.5530146 | 0.83068776 | 1 | 40 |
| PYRIMIDINE_METABOLISM | 8 | -0.8964586 | 0.562249 | 0.8050745 | 1 | 13 |
| GLYCINE_SERINE_AND_THREONINE_METABOLISM | 9 | -0.7700872 | 0.6830266 | 0.9393847 | 1 | 30 |
| TYROSINE_METABOLISM | 2 | -0.769539 | 0.73913044 | 0.895587 | 1 | 19 |
| PHENYLALANINE_METABOLISM | 3 | -0.5310729 | 0.9564356 | 1 | 1 | 19 |
| THIAMINE_METABOLISM | 3 | -0.48144296 | 0.97475725 | 1 | 1 | 30 |
| SULFUR_ METABOLISM | 2 | -0.44120446 | 0.9849906 | 0.9952599 | 1 | 30 |

**Table 3: GSEA MPAKT**

| **Metabolite set** | **No of metab olites** | **Normalized Enrichment Score** | **NOM p-val** | **FDR q-val** | **FWER p-val** | **RANK AT MAX** |
|---|---|---|---|---|---|---|
| PROPANOATE_METABOLISM | 3 | 1.4212209 | 0.007677543 | 1 | 0.654 | 11 |
| RIBOFLAVIN_METABOLISM | 3 | 1.372716 | 0.09445585 | 1 | 0.75 | 22 |
| PYRUVATE_METABOLISM | 2 | 1.3104335 | 0.07984791 | 1 | 0.877 | 12 |
| VALINE_LEUCINE_AND_ISOLEUCINE_DEGRADATION | 3 | 1.2896582 | 0.09981167 | 0.9193679 | 0.904 | 24 |
| GLYCOLYSIS_GLUCONEOGENESIS | 3 | 1.2842201 | 0.11821705 | 0.76036984 | 0.909 | 28 |
| FRUCTOSE_AND_MANNOSE_METABOLISM | 5 | 1.2186812 | 0.23224568 | 0.9087855 | 0.963 | 39 |
| VALINE_LEUCINE_AND_ISOLEUCINE_BIOSYNTHESIS | 4 | 1.203439 | 0.20229007 | 0.83821553 | 0.967 | 36 |
| SPHINGOLIPID_METABOLISM | 2 | 1.1720407 | 0.2967864 | 0.8420814 | 0.983 | 7 |
| CYANOAMINO_ACID_METABOLISM | 4 | 1.1263003 | 0.3490566 | 0.91562194 | 0.988 | 23 |
| CITRATE_CYCLE_TCA_CYCLE | 4 | 1.0926877 | 0.40726578 | 0.9433773 | 0.991 | 13 |
| LYSINE_BIOSYNTHESIS | 2 | 1.0827181 | 0.4215501 | 0.89252335 | 0.992 | 34 |
| LYSINE_DEGRADATION | 3 | 1.0561596 | 0.43202978 | 0.893319 | 0.994 | 34 |
| INOSITOL_PHOSPHATE_METABOLISM | 3 | 1.0481584 | 0.45901638 | 0.84673667 | 0.994 | 9 |
| PHENYLALANINE_TYROSINE_AND_TRYPTOPHAN_BIOSYNTHESIS | 3 | 1.030014 | 0.46780303 | 0.83334106 | 0.998 | 46 |
| PENTOSE_PHOSPHATE_PATHWAY | 6 | 0.99357647 | 0.541502 | 0.8660383 | 0.999 | 52 |
| GLYOXYLATE_AND_DICARBOXYLATE_METABOLISM | 9 | 0.99266577 | 0.4915254 | 0.81275177 | 0.999 | 18 |
| PRIMARY_BILE_ACID_BIOSYNTHESIS | 4 | 0.97943664 | 0.47991967 | 0.7916929 | 0.999 | 19 |
| PHENYLALANINE_METABOLISM | 4 | 0.9507707 | 0.55893534 | 0.80092 | 0.999 | 46 |
| GALACTOSE_METABOLISM | 6 | 0.9412344 | 0.6054159 | 0.77208287 | 0.999 | 33 |
| THIAMINE_METABOLISM | 4 | 0.934541 | 0.5882353 | 0.744193 | 0.999 | 18 |
| SULFUR_METABOLISM | 2 | 0.820349 | 0.72121215 | 0.8819321 | 1 | 7 |
| VITAMIN_B6_METABOLISM | 2 | 0.79861397 | 0.78313255 | 0.86963636 | 1 | 22 |
| PANTOTHENATE_AND_COA_BIOSYNTHESIS | 6 | 0.6654045 | 0.85265225 | 0.9783193 | 1 | 23 |
| AMINO_SUGAR_AND_NUCLEOTIDE_SUGAR_METABOLISM | 8 | 0.6636729 | 0.83472806 | 0.93915343 | 1 | 39 |
| STEROID_BIOSYNTHESIS | 2 | 0.6605123 | 0.85685885 | 0.9049515 | 1 | 19 |
| BETA-ALANINE_METABOLISM | 6 | 0.6585342 | 0.86159843 | 0.871574 | 1 | 26 |

**Table 3: GSEA Lo-MYC**

| **Metabolite set** | **No of metabolites** | **Normalized Enrichment Score** | **NOM p-val** | **FDR q-val** | **FWER p-val** | **RAN KAT MAX** |
|---|---|---|---|---|---|---|
| BIOSYNTHESIS_OF_UNSATURATED_FATTY_ACIDS | 9 | -1.4511175 | 0.05380334 | 0.99184 | 0.59 | 33 |
| LINOLEIC_ACID_METABOLISM | 3 | -1.3828204 | 0.021857923 | 0.8998 | 0.772 | 13 |
| ARGININE_AND_PROLINE_METABOLISM | 12 | -1.368322 | 0.13865547 | 0.66961 | 0.803 | 10 |
| D-GLUTAMINE_AND_D-GLUTAMATE_METABOLISM | 2 | -1.3359506 | 0.096114516 | 0.60689 | 0.848 | 10 |
| TAURINE_AND_HYPOTAURINE_METABOLISM | 5 | -1.302605 | 0.13806707 | 0.605 | 0.908 | 24 |
| PYRIMIDINE_METABOLISM | 13 | -1.2765912 | 0.16359918 | 0.58182 | 0.939 | 7 |
| PURINE_METABOLISM | 15 | -1.1867205 | 0.20042194 | 0.78346 | 0.976 | 25 |
| ASCORBATE_AND_ALDARATE _METABOLISM | 4 | -1.151681 | 0.27309236 | 0.80321 | 0.983 | 7 |
| PENTOSE_AND_GLUCURONATE_INTERCONVERSIONS | 6 | -1.126041 | 0.296 | 0.78761 | 0.992 | 7 |
| GLYCINE_SERINE_AND_THREONINE_METABOLISM | 12 | -1.0248519 | 0.428 | 1 | 0.996 | 8 |
| ARACHIDONIC_ACID_METABOLISM | 2 | -0.998357 | 0.5139442 | 0.97612 | 1 | 9 |
| GLYCEROLIPID_METABOLISM | 4 | -0.9906563 | 0.5187377 | 0.91307 | 1 | 7 |
| ALANINE_ASPARTATE_AND_GLUTAMATE_METABOLISM | 4 | -0.98792636 | 0.5254583 | 0.84914 | 1 | 10 |
| HISTIDINE_METABOLISM | 5 | -0.94616646 | 0.5529865 | 0.86822 | 1 | 10 |
| GLYCEROPHOSPHOLIPID_METABOLISM | 7 | -0.9143583 | 0.606403 | 0.8 6177 | 1 | 27 |
| FATTY_ACID_BIOSYNTHESIS | 4 | -0.8648357 | 0.65252525 | 0.89381 | 1 | 44 |
| STARCH_AND_SUCROSE_METABOLISM | 6 | -0.83841366 | 0.6825397 | 0.87951 | 1 | 7 |
| GLUTATHIONE_METABOLISM | 7 | -0.8241191 | 0.6639511 | 0.85319 | 1 | 24 |
| NICOTINATE_AND_NICOTINAMIDE_METABOLISM | 3 | -0.7469816 | 0.7590361 | 0.90931 | 1 | 32 |
| PORPHYRIN_AND_CHLOROPHYLL_METABOLISM | 3 | -0.7453042 | 0.79352224 | 0.86597 | 1 | 10 |
| CYSTEINE_AND_METHIONINE_METABOLISM | 9 | -0.69749016 | 0.8177966 | 0.875 75 | 1 | 26 |
| UBIQUINONE_AND_OTHER_TERPENOID-QUINONE_BIOSYNTHESIS | 2 | -0.60078293 | 0.9536842 | 0.9168 | 1 | 91 |

**Table 3: GSEA PhosphoAKIT1-high tumors**

| **Metabolite set** | **No of metab olites** | **Normaliz ed Enrichm ent Score** | **NOM p-val** | **FDR q-val** | **FWER p-val** | **RANK AT MAX** |
|---|---|---|---|---|---|---|
| GLYCOLYSIS_GLUCONEOGENESIS | 4 | 1.5907214 | 0 | 0.46191543 | 0.332 | 16 |
| AMINO_SUGAR_AND_NUCLEOTIDE_SUGAR_METABOLISM | 7 | 1.5328926 | 0.020072993 | 0.42452946 | 0.504 | 40 |
| PYRIMIDINE_METABOLISM | 12 | 1.4802719 | 0.052892562 | 0.45880678 | 0.674 | 39 |
| PYRUVATE_METABOLISM | 3 | 1.4683071 | 0.026 | 0.3751393 | 0.702 | 13 |
| PENTOSE_PHOSPHATE_PATHWAY | 7 | 1.4230571 | 0.095 | 0.44165498 | 0.82 | 16 |
| STARCH_AND_SUCROSE_METABOLISM | 4 | 1.3226093 | 0.10642202 | 0.7378345 | 0.961 | 25 |
| FRUCTOSE_AND_MANNOSE_METABOLISM | 6 | 1.3132623 | 0.13768116 | 0.671879 | 0.966 | 40 |
| CYSTEINE_AND_METHIONINE_METABOLISM | 11 | 1.2838272 | 0.19607843 | 0.70322126 | 0.98 | 22 |
| ASCORBATE_AND_ALDARATE_METABOLISM | 4 | 1.242083 | 0.21402878 | 0.7720861 | 0.992 | 58 |
| PROPANOATE_METABOLISM | 5 | 1.1808307 | 0.29681274 | 0.92195565 | 0.998 | 39 |
| NICOTINATE_AND_NICOTINAMIDE_METABOLISM | 8 | 1.1765169 | 0.274276 | 0.8520035 | 0.998 | 80 |
| ARGININE_AND_PROLINE_METABOLISM | 21 | 1.1571836 | 0.29952458 | 0.8452255 | 0.999 | 35 |
| INOSITOL_PHOSPHATE_METABOLISM | 3 | 1.1525284 | 0.31501058 | 0.79334915 | 0.999 | 64 |
| TAURINE_AND_HYPOTAURINE_METABOLISM | 7 | 1.1355695 | 0.34843206 | 0.78442246 | 0.999 | 18 |
| STEROID_HORMONE_BIOSYNTHESIS | 2 | 1.0969528 | 0.4081238 | 0.8339776 | 0.999 | 61 |
| BUTIROSIN_AND_NEOMYCIN_BIOSYNTHESIS | 2 | 1.0847456 | 0.38477367 | 0.8128595 | 0.999 | 7 |
| PURINE_METABOLISM | 18 | 1.0811335 | 0.38162544 | 0.77331376 | 0.999 | 65 |
| VITAMIN_B6_METABOLISM | 3 | 1.0634779 | 0.43485916 | 0.77049446 | 1 | 13 |
| HISTIDINE_METABOLISM | 9 | 1.0361688 | 0.3986135 | 0.78980684 | 1 | 69 |
| OXIDATIVE_PHOSPHORYLATION | 7 | 1.0176637 | 0.48181817 | 0.7894189 | 1 | 76 |
| PRIMARY_BILE_ACID_BIOSYNTHESIS | 4 | 0.9972558 | 0.5371094 | 0.79108274 | 1 | 66 |
| ALANINE_ASPARTATE_AND_GLUTAMATE_METABOLISM | 11 | 0.94110465 | 0.5704698 | 0.8591216 | 1 | 37 |
| GLUTATHIONE_METABOLISM | 12 | 0.92024606 | 0.5968992 | 0.8611452 | 1 | 23 |
| GLYCINE_SERINE_AND_THREONINE_METABOLISM | 12 | 0.91994816 | 0.5643739 | 0.82558614 | 1 | 13 |
| GLYCEROPHOSPHOLIPID_METABOLISM | 9 | 0.9101822 | 0.5694915 | 0.80967337 | 1 | 92 |
| TYROSINE_METABOLISM | 5 | 0.8141563 | 0.73867595 | 0.921916 | 1 | 13 |
| GALACTOSE_METABOLISM | 6 | 0.7993824 | 0.7218045 | 0.9076516 | 1 | 84 |
| D-GLUTAMINE_AND_D-GLUTAMATE_ METABOLISM | 3 | 0.79120994 | 0.7649186 | 0.88606244 | 1 | 28 |
| PHENYLALANINE_METABOLISM | 7 | 0.7823577 | 0.771518 | 0.8666423 | 1 | 53 |
| PANTOTHENATE_AND_COA_BIOSYNT HESIS | 10 | 0.7694747 | 0.74523395 | 0.8529612 | 1 | 79 |
| THIAMINE_METABOLISM | 4 | 0.7329624 | 0.80626225 | 0.87004304 | 1 | 13 |
| CITRATE_CYCLE_TCA_CYCLE | 8 | 0.64468 | 0.85315984 | 0.9347561 | 1 | 13 |
| PENTOSE_AND_GLUCURONATE_INTERCONVERSIONS | 7 | 0.598778 | 0.90226877 | 0.9441087 | 1 | 98 |
| GLYOXYLATE_AND_DICARBOXYLATE_METABOLISM | 12 | 0.5758591 | 0.9124767 | 0.933276 | 1 | 28 |

**Table 3: GSEA MTC-high tumors**

| **Metabolite set** | **No of meta bolite s** | **Normalized Enrichment Score** | **NOM p-val** | **FDR q-val** | **FWER p-val** | **RAN KAT MAX** |
|---|---|---|---|---|---|---|
| BIOSYNTHESIS_OF_UNSATURATED_FATTY_A CIDS | 13 | -1.6898948 | 0.004338395 | 0.17238313 | 0.18 | 26 |
| LINOLEIC_ACID_METABOLISM | 3 | -1.405524 | 0.0480167 | 0.9980702 | 0.823 | 22 |
| PHENYLALANINE_TYROSINE_AND_TRYPTOPHAN_BIOSYNTHESIS | 4 | -1.3494385 | 0.09210526 | 0.94579667 | 0.914 | 32 |
| FATTY_ACID_BIOSYNTHESIS | 5 | -1.3365041 | 0.1594203 | 0.7610707 | 0.931 | 17 |
| PORPHYRIN_AND_CHLOROPHYLL_METABOL ISM | 4 | -1.1784091 | 0.31692913 | 1 | 0.992 | 50 |
| LYSINE_DEGRADATION | 9 | -1.129812 | 0.33248731 | 1 | 0.996 | 61 |
| VALINE_LEUCINE_AND_ISOLEUCINE_DEGRADATION | 3 | -1.0934087 | 0.36734694 | 1 | 0.999 | 68 |
| RIBOFLAVIN_METABOLISM | 3 | -1.06163 | 0.44469026 | 1 | 1 | 31 |
| CYANOAMINO_ACID_METABOLISM | 5 | -0.99628294 | 0.5220264 | 1 | 1 | 51 |
| D-ARGININE_AND_D-ORNITHINE_ METABOLISM | 2 | -0.9939161 | 0.49372384 | 1 | 1 | 43 |
| SULFUR_METABOLISM | 3 | -0.97494125 | 0.51096493 | 1 | 1 | 109 |
| GLYCEROLIPID_METABOLISM | 3 | -0.85183764 | 0.65784115 | 1 | 1 | 39 |
| TRYPTOPHAN_METABOLISM | 6 | -0.8230189 | 0.7038044 | 1 | 1 | 149 |
| UBIQUINONE_AND_OTHER_TERPENOID-QUINONE_ BIOSYNTHESIS | 4 | -0.79604733 | 0.7002342 | 1 | 1 | 19 |
| CAFFEINE_METABOLISM | 6 | -0.750715 | 0.71938777 | 1 | 1 | 5 |
| SPHINGOLIPID_METABOLISM | 4 | -0.6737419 | 0.89498806 | 1 | 1 | 158 |
| BUTANOATE_METABOLISM | 9 | -0.6569208 | 0.86493504 | 1 | 1 | 71 |
| VALINE_LEUCINE_AND_ISOLEUCINE_BIOSYNTHESIS | 5 | -0.6417897 | 0.8487395 | 1 | 1 | 50 |
| ETHER_LIPID_METABOLISM | 2 | -0.63222766 | 0.9 | 1 | 1 | 139 |
| LYSINE_BIOSYNTHESIS | 5 | -0.5990712 | 0.943662 | 0.9970749 | 1 | 166 |
| BETA-ALANINE_METABOLISM | 12 | -0.5383798 | 0.9814324 | 0.99758583 | 1 | 190 |
| FATTY_ACID_METABOLISM | 3 | -0.50268257 | 0.98547214 | 0.9723302 | 1 | 28 |

## Claims

1. A method to identify Akt1 and Myc status in a prostate tumor comprising:
analyzing, with at least one computer processor, a profile of at least 5 metabolites in a prostate tumor sample obtained from a subject to assign an Akt1 and Myc status to the sample, wherein:
the at least 5 metabolites are differentially produced in prostate tumors with high Akt1 expression versus prostate tumors with high Myc expression, wherein the metabolites are selected from Table 1;
wherein the expression profile of metabolites is compared to an appropriate reference profile of the metabolites, wherein the appropriate reference profile of the metabolites comprises profiles of the metabolites in prostate tumor with high Akt1 expression, in prostate tumor with low Akt1 expression, in prostate tumor with high Myc expression, and in prostate tumor with low Myc expression; and
wherein the processor assigns a status of high Akt1/high Myc, high Akt1/low Myc, low Akt1 /high Myc, or low Akt1/low Myc to the sample.

2. The method of claim 1, wherein the method further comprises:
performing an assay to measure the profile of the at least 5 metabolites in the prostate tumor sample obtained from the subject.

3. The method of any one of claims 1-2, wherein the metabolic profile comprises at least 10, at least 25, at least 50, at least 75, at least 100, at least 125, at least 150, at least 175, at least 200, at least 225, at least 250, at least 275, at least 300, or at least 350 metabolites.

4. The method of any one of claims 1-3, wherein the metabolic profile of the tumor sample is measured using one or more of mass spectroscopy, nuclear magnetic resonance (NMR), or chromatography.

5. The method of any one of claims 1-4, wherein the profile of the at least 5 metabolites of the tumor sample is compared using cluster analysis, optionally wherein the cluster analysis is selected from the group consisting of: hierarchical clustering, k-mean clustering, distribution-based clustering, and density-based clustering.

6. The method of any one of claims 1-5, wherein the differentially produced metabolites are selected using a threshold of p value < 0.05.

7. The method of any one of claims 1-6, wherein the method further comprises:
determining a confidence value for the Akt1 and Myc status assigned to the sample; and
providing an indication of the confidence value and the Akt1 and Myc status assigned to the sample to a user, optionally wherein the method further comprises:
determining whether the confidence value is below a threshold value; and
providing an indication that the confidence value is below the threshold value.

8. A computer-readable storage medium encoded with a plurality of instructions that, when executed by at least one computer processor, performs a method according to any of claims 1 to 7.

9. The computer-readable storage medium of claim 8, wherein the method further comprises:
determining a confidence value for the Akt1 and Myc status assigned to the sample; and
providing an indication of the confidence value and the Akt1 and Myc status assigned to the sample to a user, optionally wherein the method further comprises:
determining whether the confidence value is below a threshold value; and
providing an indication that the confidence value is below the threshold value .

## Patentansprüche

1. Ein Verfahren zum Identifizieren des Akt1- und Myc-Status in einem Prostatatumor, das Folgendes beinhaltet:
Analysieren, mit mindestens einem Computerprozessor, eines Profils von mindestens 5 Metaboliten in einer von einem Individuum erhaltenen Prostatatumorprobe, um der Probe einen Akt1- und Myc-Status zuzuweisen, wobei:
die mindestens 5 Metaboliten differenziell in Prostatatumoren mit hoher Akt1-Expression gegenüber Prostatatumoren mit hoher Myc-Expression produziert werden, wobei die Metaboliten aus Tabelle 1 ausgewählt sind;
wobei das Expressionsprofil von Metaboliten mit einem angemessenen Bezugsprofil der Metaboliten verglichen wird, wobei das angemessene Bezugsprofil der Metaboliten Profile der Metaboliten in Prostatatumoren mit hoher Akt1-Expression, in Prostatatumoren mit niedriger Akt1-Expression, in Prostatatumoren mit hoher Myc-Expression und in Prostatatumoren mit niedriger Myc-Expression beinhaltet; und
wobei der Prozessor der Probe einen Status von hohem Akt1/hohem Myc, hohem Akt1/niedrigem Myc, niedrigem Akt1/hohem Myc oder niedrigem Akt1/niedrigem Myc zuweist.

2. Verfahren gemäß Anspruch 1, wobei das Verfahren ferner Folgendes beinhaltet:
Durchführen eines Assays zur Messung des Profils der mindestens 5 Metaboliten in der von dem Individuum erhaltenen Prostatatumorprobe.

3. Verfahren gemäß einem der Ansprüche 1-2, wobei das metabolische Profil mindestens 10, mindestens 25, mindestens 50, mindestens 75, mindestens 100, mindestens 125, mindestens 150, mindestens 175, mindestens 200, mindestens 225, mindestens 250, mindestens 275, mindestens 300 oder mindestens 350 Metaboliten beinhaltet.

4. Verfahren gemäß einem der Ansprüche 1-3, wobei das metabolische Profil der Tumorprobe unter Verwendung von einem von Massenspektroskopie, kernmagnetischer Resonanz (NMR) oder Chromatographie gemessen wird.

5. Verfahren gemäß einem der Ansprüche 1-4, wobei das Profil der mindestens 5 Metaboliten der Tumorprobe unter Verwendung der Clusteranalyse verglichen wird, wobei die Clusteranalyse optional aus der Gruppe ausgewählt ist, die aus Folgenden besteht: hierarchische Clusteranalyse, k-Means-Clusteranalyse, distributionsbasierte Clusteranalyse und dichtebasierte Clusteranalyse.

6. Verfahren gemäß einem der Ansprüche 1-5, wobei die differenziell produzierten Metaboliten unter Verwendung eines Schwellenwerts von p-Wert < 0,05 ausgewählt sind.

7. Verfahren gemäß einem der Ansprüche 1-6, wobei das Verfahren ferner Folgendes beinhaltet:
Bestimmen eines Konfidenzwerts für den der Probe zugewiesenen Akt1- und Myc-Status; und
Bereitstellen eines Hinweises auf den Konfidenzwert und den der Probe zugewiesenen Akt1- und Myc-Status für einen Benutzer, wobei das Verfahren optional ferner Folgendes beinhaltet:
Bestimmen, ob der Konfidenzwert unter einem Schwellenwert liegt; und
Bereitstellen eines Hinweises darauf, dass der Konfidenzwert unter dem Schwellenwert liegt.

8. Ein computerlesbares Speichermedium, in dem eine Vielzahl von Instruktionen kodiert sind, die, wenn sie von mindestens einem Computerprozessor ausgeführt werden, ein Verfahren gemäß einem der Ansprüche 1 bis 7 durchführen.

9. Computerlesbares Speichermedium gemäß Anspruch 8, wobei das Verfahren ferner Folgendes beinhaltet:
Bestimmen eines Konfidenzwerts für den der Probe zugewiesenen Akt1- und Myc-Status; und
Bereitstellen eines Hinweises auf den Konfidenzwert und den der Probe zugewiesenen Akt1- und Myc-Status für einen Benutzer, wobei das Verfahren optional ferner Folgendes beinhaltet:
Bestimmen, ob der Konfidenzwert unter einem Schwellenwert liegt; und
Bereitstellen eines Hinweises darauf, dass der Konfidenzwert unter dem Schwellenwert liegt.

## Revendications

1. Une méthode permettant d'identifier le statut Akt1 et Myc d'une tumeur de la prostate comprenant :
l'analyse, avec au moins un processeur d'ordinateur, d'un profil d'au moins 5 métabolites dans un échantillon de tumeur de la prostate prélevé sur un sujet afin d'attribuer un statut Akt1 et Myc à l'échantillon, dans laquelle :
au moins 5 métabolites sont produits de manière différentielle dans des tumeurs de la prostate présentant une expression élevée de l'Akt 1 par rapport à des tumeurs de la prostate présentant une expression élevée de Myc, dans laquelle les métabolites sont choisis dans le Tableau 1 ;
dans laquelle le profil d'expression des métabolites est comparé à un profil de référence approprié des métabolites, dans laquelle le profil de référence approprié des métabolites se compose de profils des métabolites dans une tumeur de la prostate présentant une expression élevée d'Akt1, dans une tumeur de la prostate présentant une expression faible d'Akt1, dans une tumeur de la prostate présentant une expression élevée de Myc, et dans une tumeur de la prostate présentant une expression faible de Myc ; et
dans laquelle le processeur attribue à l'échantillon un statut correspondant à Akt1 élevée/Myc élevé, Akt1 élevée/Myc faible, Atk 1 faible/Myc élevé, ou Akt1 faible/Myc faible.

2. La méthode de la revendication 1, comprenant en outre :
d'effectuer un dosage pour mesurer le profil d'au moins 5 métabolites dans l'échantillon de tumeur de la prostate prélevé sur un sujet.

3. La méthode de l'une quelconque des revendications 1 à 2, dans laquelle le profil métabolique est composé d'au moins 10, d'au moins 25, d'au moins 50, d'au moins 75, d'au moins 100, d'au moins 125, d'au moins 150, d'au moins 175, d'au moins 200, d'au moins 225, d'au moins 250, d'au moins 275, d'au moins 300, ou d'au moins 350 métabolites.

4. La méthode de l'une quelconque des revendications 1 à 3, dans laquelle le profil métabolique de l'échantillon de tumeur est mesuré à l'aide d'une ou plusieurs méthodes telles que la spectroscopie de masse, la résonance magnétique nucléaire (RMN), ou la chromatographie

5. La méthode de l'une quelconque des revendications 1 à 4, dans laquelle le profil d'au moins 5 métabolites de l'échantillon de tumeur est comparé à l'aide d'une analyse typologique, dans laquelle, éventuellement, l'analyse typologique est choisie dans le groupe constitué de : classification hiérarchique, le partitionnement en k-moyennes, le partitionnement basé sur la distribution, et le partitionnement basé sur la densité.

6. La méthode de l'une quelconque des revendications 1 à 5, dans laquelle des métabolites produits de manière différentielle sont sélectionnés à l'aide d'une valeur seuil p < 0,05.

7. La méthode de l'une quelconque des revendications 1 à 6, comprenant en outre :
de déterminer une valeur de confiance pour le statut Akt1 et Myc attribué à l'échantillon ; et
de fournir une indication de la valeur de confiance et du statut Akt1 et Myc attribué à l'échantillon à un utilisateur, la méthode comprenant éventuellement en outre : de déterminer si la valeur de confiance est inférieure à une valeur seuil ; et d'indiquer que la valeur de confiance est inférieure à la valeur seuil.

8. Un support de stockage informatique encodé doté d'une pluralité d'instructions qui, lorsqu'exécutées par au moins un processeur d'ordinateur, effectuent ce qui est indiqué aux revendications 1 à 7.

9. Le support de stockage informatique de la revendication 8, dans lequel la méthode comprend en outre :
de déterminer une valeur de confiance pour le statut Akt1 et Myc attribué à l'échantillon ;
Et
de fournir une indication de la valeur de confiance et du statut Akt1 et Myc attribué à l'échantillon à un utilisateur, la méthode comprenant éventuellement en outre :
de déterminer si la valeur de confiance est inférieure à une valeur seuil ; et
d'indiquer que la valeur de confiance est inférieure à la valeur seuil.
